# DEMANDE DE BREVET EUROPEEN

(11) **EP 3 257 867 A1**
(43) Date de publication de la demande: **20.12.2017**
(21) Numéro de dépôt: 16175041.9
(22) Date de dépôt: 17.06.2016
(51) Int. Cl.: C07K 16/26, G01N 33/53

(54) **PROCÉDÉ DE PRÉPARATION D'ANTICORPS ANTI-AMH ET LEURS UTILISATIONS**

(71) Demandeur: Biomérieux, 69280 Marcy L'etoile (FR)
(72) Inventeur: ATAMAN-ONAL, Yasemin, 01600 Reyrieux (FR); CHEUCLE, Sylvie, 69890 La Tour de Salvagny (FR); COMBE, Maxime, 69002 Lyon (FR); SOIZIC, Daniel, 01600 Trevoux (FR); OTTONE, Sophie, 69210 Sain-Bel (FR)
(74) Mandataire: Bitaud, Valérie Marie-Odile

(57) **Abrégé**

La présente invention concerne un procédé de préparation d'anticorps anti-AMH de mammifère comprenant les étapes de :
(i) immunisation d'un animal avec un polypeptide d'AMH ou un polynucléotide codant pour ce polypeptide d'AMH, ledit polypeptide d'AMH comprenant au moins les 99 acides aminés de séquence SEQ ID N°1 ou de séquence ayant au moins 75% d'identité avec la séquence SEQ ID N°1, et au plus les 560 acides aminés de séquence SEQ ID N°2 ou de séquence ayant au moins 75% d'identité avec la séquence SEQ ID N°2,
(ii) préparation d'hybridomes à partir des cellules d'un organe lymphoïde de l'animal ayant reçu l'immunogène,
(iii) sélection des hybridomes secrétant des anticorps reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQ ID N°13 ou de séquence ayant au moins 75% d'identité avec la séquence SEQ ID N°13 et au plus les 156 acides aminés de séquence SEQ ID N°1 ou de séquence ayant au moins 75% d'identité avec la séquence SEQ ID N°11, ni (b) aucun épitope linéaire situé dans la séquence SEQ ID N°1 ou une séquence ayant au moins 75% d'identité avec la séquence SEQ ID N°1, et
(iv) production des anticorps.

Elle concerne également les anticorps, fragments d'anticorps et leur utilisation pour le dosage d'AMH, notamment en fertilité.

## Description

La présente invention concerne le domaine de détection *in vitro* de l'hormone anti-Müllérienne, autrement appelée AMH. En particulier, l'invention concerne la préparation d'anticorps anti-AMH, les anticorps anti-AMH et leurs utilisations pour la détermination de la concentration d'AMH, notamment dans le cadre des explorations liées à la fertilité chez la femme ou animaux femelles en âge de procréer.

L'hormone anti-Müllérienne, (également appelée AMH pour anti-Müllerian hormone) est une glycoprotéine dimérique de 144 kDa de la famille du Transforming Growth Factor (TGF-β), famille comprenant de nombreux facteurs agissant sur la croissance et la différentiation. L'AMH est une prohormone dimérique : elle est constituée de deux sous-unités identiques qui sont reliées par des ponts disulfures. Cette prohormone subit un clivage protéolytique près de l'extrémité C-terminale afin d'acquérir une activité biologique et se transformer en hormone mature. Après clivage, le complexe moléculaire reste associé et est dosable dans le sang par immunoessai permettant des performances de sensibilité adaptées au suivi de l'évolution de la réserve ovarienne depuis la naissance jusqu'à la ménopause (KELSEY TW, et al., 2011). Cette protéine est également connue sous le nom de MIF (Müllerian-inhibiting factor), MIH (Müllerian-inhibiting hormone) et MIS (Müllerian-inhibiting substance).

L'AMH est présente chez tous les mammifères. Sa longueur et sa séquence en acides aminés sont dépendantes de l'espèce. Ainsi, l'AMH humaine possède 560 acides aminés et se compose comme suit : un peptide signal court (acides aminés 1-18), une partie précurseur (acides aminés 19-25), une partie N-terminale (acides aminés 26-451) et une partie C-terminale (acides aminés 452-560). L'AMH équine possède 573 acides aminés et se compose comme suit : un peptide signal court (acides aminés 1-22), pas de partie précurseur, une partie N-terminale (acides aminés 23-464) et une partie C-terminale (acides aminés 465-573). L'AMH canine possède 572 acides aminés et se compose comme suit : un peptide signal court (acides aminés 1-21), pas de partie précurseur, une partie N-terminale (acides aminés 22-463) et une partie C-terminale (acides aminés 464-572). L'AMH bovine quant à elle possède 575 acides aminés et se compose comme suit : un peptide signal court (acides aminés 1-17), une partie précurseur (acides aminés 18-24), une partie N-terminale (acides aminés 25-466) et une partie C-terminale (acides aminés 467-575). Quel que soit l'espèce, la partie N-terminale de l'AMH est appelée « région pro » et la partie C-terminale est appelée « région mature ».

L'AMH est synthétisée sous forme de précurseur polypeptidique comprenant le peptide signal, suivie de la pré-prohormone (chez l'Homme et le Bovin) ou de la prohormone (chez le Cheval ou le Chien). Le peptide signal, qui permet l'adressage vers le réticulum endoplasmique, est clivé suite à la translocation. Dans le réticulum endoplasmique, le précurseur polypeptidique subit des modifications post-traductionnelles, à savoir (i) l'homodimérisation et (ii) la glycosylation, afin d'atteindre sa conformation native. Chez l'Homme, il s'agit d'une glycoprotéine dimérique de 144 kDa correspondant aux acides aminés 26-560. Chaque monomère, de 72 kDa chez l'Homme, et contient la région pro (58 kDa chez l'homme) suivie de la région mature (12 kDa chez l'homme). Avant sécrétion vers le milieu extracellulaire et la circulation sanguine, la glycoprotéine AMH subit une ultime maturation post-traductionnelle par clivage. Ainsi, chez l'Homme et le Bovin, la pré-prohormone est transformée en pro-hormone par clivage de la partie précurseur. La pro-hormone est également clivée entre la partie N-terminale et la partie C-terminale afin d'obtenir l'hormone mature (ayant une activité biologique). Ces deux parties restant toutefois liées de façon non covalente (Zec I., et al., 2011). Chez l'Homme, des taux de clivage variables ont été rapportés selon les études : entre 5 à 20% par Zec et al. et de 67% chez les garçons ou encore de 81% chez la femme par Pankhurst et al. (2016). Ainsi, dans la circulation sanguine, on retrouve de l'AMH clivée et de l'AMH non clivée.

L'AMH est produite chez le mâle par les cellules de Sertoli et intervient au début de sa vie pour la différentiation du tractus masculin. Chez la femelle, l'AMH est produite par les cellules de la granulosa des petits follicules en croissance et intervient donc plutôt à partir de la puberté. Ainsi, chez la femme, l'AMH a montré son utilité dans différents domaines, notamment liés à la fertilité (DEWAILLY D, et al. 2014). Par exemple, le dosage de l'AMH dans la circulation permet d'estimer le nombre de follicules antraux et pré-antraux présents dans les ovaires, indépendamment des cycles (DEWAILLY D, et al., 2014). De plus, comme indicateur de la décroissance naturelle de la réserve ovarienne et donc des risques inhérents d'hypofertilité, le dosage de l'AMH est indiqué pour aider les femmes à gérer leurs projets de grossesse. Dans un contexte de procréation médicalement assistée, le dosage d'AMH contribue à sélectionner la meilleure stratégie pour la patiente, en optimisant notamment l'étape de stimulation contrôlée des ovaires tout en évitant le risque d'hyperstimulation (ARCE JC, et al., 2014). Le dosage d'AMH permet aussi de suivre l'évolution de la réserve ovarienne chez les jeunes filles ou femmes ayant reçu un traitement gonadotoxique en cas de cancer par exemple (CHAI J et HOWIE AF., 2014). Pour les femmes présentant des troubles de l'ovulation, la détermination des taux sériques d'AMH permet de mieux caractériser le type de dysfonctionnement ovarien, particulièrement l'anovulation hypergonadotropique associée à une insuffisance ovarienne comme le syndrome des ovaires polykystiques (FONG SL, et al., 2015). Chez le jeune garçon, l'AMH est produite de façon très importante par les testicules du foetus et du nouveau-né et intervient ainsi de façon précoce dans la différentiation du tractus masculin. Ainsi, le dosage de l'AMH a trouvé utilité chez le garçon avant la puberté, dans le cadre des troubles liés à la différentiation sexuelle.

Le dosage d'AMH est réalisé par immunoessai sandwich et nécessite la mise en oeuvre d'un test à la fois sensible, spécifique et reproductible. Or, malgré les diverses méthodes proposées dans la littérature (HUDSON, 1990 ; LONG, 2000) ou encore les différentes trousses disponibles sur le marché, un dosage alliant l'ensemble de ces caractéristiques n'est toujours pas disponible. Une des difficultés majeures à résoudre a été identifiée dès 1996 : il s'agit du manque de stabilité apparent de l'AMH dans l'échantillon biologique. Ainsi, les conditions de stockage (durée, température, cycles de congélation, ...) peuvent causer des variations dans les concentrations mesurées (LEE, 1996). Ces variations sont bien entendu un artéfact et peuvent résulter en une mauvaise interprétation du résultat biologique.

Différentes trousses ont été mises sur le marché. Les anticorps utilisés dans ces trousses reconnaissent différentes régions de l'AMH. La première génération de dosages d'AMH correspond aux trousses EIA AMH/MIS (Immunotech) et la trousse Active MIS/AMH ELISA (DSL). Les anticorps utilisés dans ces trousses ne sont pas identiques mais chacune utilise un premier anticorps reconnaissant la région pro de l'AMH, l'autre anticorps reconnaissant la région mature. En 2010, la Société Beckman Coulter qui les commercialisait, les a remplacés par une trousse de deuxième génération (AMH Gen II Assay) avec, selon eux, des performances améliorées. En effet, selon les auteurs, cette trousse est hautement spécifique et l'AMH mesurée ne serait pas affectée par la protéolyse car les deux anticorps monoclonaux utilisés reconnaissent chacun la région mature (KUMAR, 2015 ; US7897350). Les auteurs pensent que la région mature, qui contient plusieurs résidus cystéines, serait plus stable que la région pro en cas de protéolyse. Une fois déployée sur le terrain, en utilisation clinique de routine, il s'est vite avéré que la trousse AMH Gen II ne tenait pas ses promesses. Une large étude rétrospective a confirmé le manque de stabilité du dosage de l'AMH eu égard aux conditions de stockage des tubes d'échantillons (RUSTAMOV O, 2012) et a montré que la trousse AMH Gen II était extrêmement sensible à ces variations. Plusieurs hypothèses ont été émises pour expliquer les fluctuations observées. Une des interprétations possibles pourrait être liée au fait que la molécule d'AMH subit des changements conformationnels, après prélèvement d'un échantillon de sang, variables d'un individu à l'autre.

Ces résultats ont rapidement été répétés et confirmés par une équipe indépendante (XAN et al., 2014). De plus, cette équipe a décrit une solution pour favoriser la stabilité de l'AMH pendant la conservation de l'échantillon : ils ont effectué une pré-dilution avant dosage, ce qui permet une amélioration de la reproductibilité générale des dosages d'AMH par Gen II. Cette solution, certes facile à mettre en oeuvre, a cependant quelques inconvénients :
(i) Les concentrations d'AMH mesurées après pré-dilution sont augmentées. Cette augmentation varie d'un individu à l'autre (de 1,35 fois à 3,06 fois pour les données disponibles). Il est donc nécessaire de redéfinir les valeurs de référence de manière expérimentale, sur une large cohorte, ainsi que les règles d'interprétation clinique.
(ii) La dilution effectuée n'est pas négligeable (1/5, soit 60 µL d'échantillon dans 300 µL de tampon). Elle permet certes de s'affranchir des interférences observées, mais entraine de fait une diminution de la sensibilité du dosage, importante pour de nombreuses utilisations cliniques.
(iii) La cause de l'instabilité de l'AMH n'est toujours pas identifiée. Par conséquent, il n'est pas possible d'affirmer que la dilution pourra résoudre le problème de manque de stabilité pour tous les échantillons.

Par ailleurs, LUKASZUK et al. (2014) ont décrit une diminution de la concentration moyenne d'AMH mesurée par la trousse AMH Gen II Assay et une diminution de la sensibilité. Leur conclusion est qu'il est « très dangereux de mettre en oeuvre un protocole de stimulation basé sur les résultats de la trousse Gen II ».

Il est donc primordial de disposer d'une trousse robuste, pour laquelle la concentration d'AMH mesurée est juste et reproductible, quel que soit le moment entre le prélèvement et le dosage, et quel que soit les conditions de stockage (dose indépendant des conditions pré-analytiques).

La demande de brevet WO2014/074835 propose différents procédés de dosage de différentes isoformes d'AMH (AMH dimérique uniquement, AMH non clivée, AMH clivée puis réassociée, ...) afin de déterminer si une isoforme particulière ou le changement d'une isoforme vers une autre peut avoir un sens clinique. Ainsi, la demande décrit de nombreux anticorps dirigés contre un grand nombre d'épitopes linéaires, situés soit dans la région pro, soit dans la région mature de l'AMH. Les auteurs affirment que les compositions et méthodes divulguées répondent au besoin d'un dosage juste, reproductible et standardisé. Cette demande divulgue 18 épitopes, soit au total 324 (18x18) façons de construire des immunoessais sandwich en combinant les anticorps reconnaissant ces épitopes. Etant donné les nombreux problèmes pré-analytiques rencontrés avec les différents dosages de l'AMH, il est difficile de croire sans démonstration expérimentale que chacune de ces combinaisons d'épitope, par conséquent d'anticorps, permette d'obtenir un test robuste. Par ailleurs, aucune donnée dans cette demande de brevet ne montre que l'utilisation des anticorps décrits permet un dosage utilisable en pratique médicale courante, robuste, juste, reproductible et standardisé, et surtout dont le résultat est indépendant des conditions pré-analytiques. Les seules données présentées dans la demande concernent des données de stabilité à partir de dosages de type « single epitope sandwich ou SES », à savoir de dosages utilisant le même anticorps monoclonal en capture et détection. De plus, ces tests détectent exclusivement la forme dimérique de l'AMH dont l'intérêt clinique est pour le moment inconnu (car les concentrations mesurées sont très différentes des concentrations obtenues par les dosages d'AMH totale qui sont celles utilisées dans la pratique médicale courante).

La Demanderesse a trouvé de façon inattendue qu'il était possible de pallier les inconvénients de l'art antérieur en préparant de nouveaux anticorps ne reconnaissant que des épitopes non-linéaires, dans une zone particulière de l'AMH située dans la partie médiane de la région pro de l'AMH (entre les acides aminés 157-255 chez l'Homme), lesquels anticorps permettent d'effectuer un dosage d'AMH stable et fiable utilisable en pratique médicale courante, robuste, juste et reproductible, indépendamment des conditions pré-analytiques ou de stockage de l'échantillon. En choisissant cette zone particulière d'AMH, décrite comme possédant une zone de clivage (WO2014/074835), le dosage de l'AMH avec ces anticorps à reconnaissance d'épitope non-linéaire sur la zone définie détecte de façon surprenante et contre toute attente toute l'AMH d'intérêt biologique présente dans un échantillon biologique.

Ainsi, un premier objet de l'invention concerne un procédé de préparation d'anticorps anti-AMH de mammifère (hormone antimüllérienne de mammifère), caractérisé en ce qu'il comprend les étapes de :
(i) immunisation d'un animal avec un polypeptide d'AMH ou un polynucléotide codant pour ce polypeptide d'AMH, ledit polypeptide d'AMH comprenant au moins les 99 acides aminés de séquence SEQ ID N°1 ou de séquence ayant au moins 75% d'identité avec la séquence SEQIDN°1, et au plus les 560 acides aminés de séquence SEQ ID N°2 ou de séquence ayant au moins 75% d'identité avec la séquence SEQ ID N°2,
(ii) préparation d'hybridomes à partir des cellules d'un organe lymphoïde de l'animal ayant reçu l'immunogène,
(iii) sélection des hybridomes secrétant des anticorps reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQ ID N°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11, ni (b) aucun épitope linéaire situé dans la séquence SEQ ID N°1 ou une séquence ayant au moins 75% d'identité avec la séquence SEQ ID N°1, et
(iv) production des anticorps.

Un deuxième objet de l'invention concerne les anticorps monoclonaux ou fragments d'anticorps monoclonaux anti-AMH de mammifère reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQIDN°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11, ni aucun épitope linéaire situé dans la séquence SEQ ID N°1 ou une séquence ayant au moins 75% d'identité avec la séquence SEQ ID N°1.

Un troisième objet de l'invention concerne des conjugués comprenant (i) un anticorps monoclonal ou un fragment d'anticorps monoclonal anti-AMH tel que défini ci-dessus ou préparé selon le procédé tel que défini ci-dessus et (ii) un marqueur capable de générer l'émission d'un signal détectable pour la visualisation d'une réaction immunologique entre ce conjugué et l'AMH d'un échantillon biologique.

Un quatrième objet de l'invention concerne un procédé de quantification d'AMH de mammifère par immunoessai sandwich, dans un échantillon biologique susceptible de contenir de l'AMH, lequel comprend les étapes suivantes :
- mettre en contact ledit échantillon biologique avec deux partenaires de liaison à l'AMH dont au moins un desdits partenaires est un anticorps ou fragment d'anticorps tel que défini ci-dessus ou tel que préparé selon le procédé tel que défini ci-dessus, ou bien un conjugué tel que défini ci-dessus,
- détecter un signal émis par la liaison entre lesdits partenaires de liaison et l'AMH, si elle est présente, en utilisant un marqueur capable d'émettre un signal détectable, et
- transformer le signal détecté en une concentration en AMH.

Un cinquième objet de l'invention concerne l'utilisation d'un procédé de quantification d'AMH tel que défini ci-dessus comme une aide pour le diagnostic de troubles liés à un dysfonctionnement ovarien chez les femmes en âge de procréer ou comme une aide à l'évaluation de la réserve folliculaire ovarienne chez les jeunes filles de plus de 12 ans et les femmes, ou encore comme une aide à l'évaluation des troubles liés à la différentiation sexuelle chez le garçon avant la puberté.

Enfin, un dernier objet de l'invention concerne une trousse comprenant un anticorps ou fragment d'anticorps tel que défini ci-dessus ou tel que préparé selon le procédé tel que défini ci-dessus, ou bien un conjugué tel que défini ci-dessus.

La Demanderesse a donc trouvé de façon inattendue que l'utilisation de la partie médiane de la région pro de l'AMH pour sélectionner des anticorps monoclonaux anti-AMH lors de leur procédé de préparation, tout en écartant les anticorps à reconnaissance d'épitopes linéaires sur cette partie, permettait, lorsque ces anticorps ainsi sélectionnés sont utilisés pour le dosage d'AMH, d'avoir un dosage fiable et reproductible.

Un épitope, aussi appelé déterminant antigénique, est la plus petite partie d'un antigène qui peut être reconnue par un paratope qui est la partie variable d'un anticorps. La structure de l'épitope est complémentaire avec le paratope de l'anticorps. La structure mise en jeu peut être la structure primaire, dans le cas d'un épitope linéaire, également appelé épitope séquentiel, ou la structure tertiaire dans le cas d'un épitope discontinu, également appelé épitope non-linéaire. Lorsque l'antigène est de nature protéique, comme dans le cas présent, les épitopes linéaires correspondent à une séquence peptidique de longueur variable.

La séquence d'un épitope linéaire peut comporter des modifications dites conservatrices qui ne changent pas significativement la liaison entre l'épitope et l'anticorps d'un point de vue de la spécificité.

Les anticorps préparés selon le procédé de l'invention ne reconnaissent donc pas d'épitopes linéaires sur la partie médiane de la région pro de l'AMH mais reconnaissent cette partie médiane.

Par anticorps monoclonaux reconnaissant la partie médiane de la région pro de l'AMH, on entend que les anticorps sont capables de se lier à l'AMH quand cette partie est présente, comme par exemple à l'AMH entière, mais ne sont pas capables de se lier à un polypeptide d'AMH quand cette partie est absente.

Sans vouloir être lié à une théorie, une hypothèse a postériori consisterait à penser que la partie médiane de la région pro de l'AMH contre laquelle sont dirigés les anticorps pourrait correspondre à une zone où l'AMH ne subirait pas de protéolyse dans l'échantillon biologique dans laquelle elle est dosée, par exemple le plasma, de sorte que cette zone resterait bien présentée sous sa conformation d'origine, ou encore que sa conformation ne serait pas impactée par des modifications qui pourraient toucher des zones environnantes de la molécule.

Les mammifères pour qui on souhaite produire des anticorps dirigés contre leur partie médiane de la région pro de leur AMH sont tout mammifère pour qui le dosage d'AMH constitue une aide. A titre d'exemple, on peut citer l'Homme (femme et homme dont le garçon), le Cheval (en particulier la jument), le Chien (en particulier la chienne), les Bovins, les Ovins, les Félins.

Les AMH sécrétées par les mammifères ne sont pas identiques mais sont proches. Entre l'AMH humaine et les AMH des autres mammifères pour qui le dosage d'AMH constitue une aide, on observe au moins 75% d'identité de séquences. L'AMH pris en référence dans l'invention sera l'AMH humaine.

Les termes « identité» ou « pourcentage d'identité » dans le contexte de deux ou plusieurs séquences polypeptidiques signifient que les deux séquences comparées ont un pourcentage spécifié de résidus d'acides aminés qui sont les mêmes sur la longueur maximale qui a pu être alignée, lorsqu'on les aligne pour une correspondance maximale. De tels alignements et calculs du pourcentage d'identité peuvent être réalisés par des algorithmes et/ou des logiciels de comparaison de séquences (Emboss Needle, LAlign, Blast, Clustal, ...) ou par inspection visuelle dans des cas simples.

Comme indiqué précédemment, l'AMH est constituée d'une région pro et d'une région mature. Chez l'Homme, la région pro se termine en position 451 et la région mature commence en position 452. La partie médiane de la région pro de l'AMH reconnue par les anticorps préparés selon l'invention commence chez l'Homme en position 157 et se termine en position 255. Elle est constituée des 99 acides aminés de la séquence SEQ ID N°1. Chez le Cheval, la partie médiane de la région pro de l'AMH reconnue par les anticorps préparés selon l'invention commence en position 167, se termine en position 265 et correspond aux 99 acides de séquence SEQ ID N°14. La partie médiane de la région pro de l'AMH canine reconnue par les anticorps préparés selon l'invention commence quant à elle en position 166, se termine en position 264 et correspond aux 99 acides de séquence SEQ ID N°23. S'agissant des bovins, la partie médiane de la région pro de l'AMH reconnue par les anticorps préparés selon l'invention commence en position 171, se termine en position 270 et correspond aux 100 acides de séquence SEQ ID N°32.

Pour produire ces anticorps anti-AMH particuliers et avantageux, le procédé de l'invention comprend, comme première étape, l'immunisation d'un animal avec un immunogène. Cet immunogène est un polypeptide d'AMH, ou un polynucléotide codant pour ce polypeptide d'AMH, qui doit comprendre au moins la zone que les anticorps doivent reconnaître, à savoir au moins la partie médiane de la région pro de l'AMH. Ainsi l'immunogène utilisé comprend au moins les 99 acides aminés de séquence SEQ ID N°1 ou de séquence ayant au moins 75% d'identité avec la séquence SEQ ID N°1.

L'immunogène comprend également au plus l'AMH entière de l'espèce considérée ou le polynucléotide codant pour l'AMH entière de l'espèce considérée, à savoir, dans le cas de l'AMH humaine, les 560 acides aminés de séquence SEQ ID N°2 (N°accession Uniprot KB F2YMM5), les autres AMH comprenant au moins 75% d'identité avec la séquence SEQ ID N°2. Par exemple, le polypeptide d'AMH totale équine comprend les 573 acides aminés de séquence SEQ ID N°15 (N°accession Uniprot KB F2YMM5), le polypeptide d'AMH totale canine comprend les 572 acides aminés de séquence SEQ ID N°24 (N°accession Uniprot KB A0A0E3N0I3) et le polypeptide d'AMH totale bovine (N°accession Uniprot KB P03972) comprend les 575 acides aminés de séquence SEQ ID N°33.

Selon un mode de réalisation particulier, l'immunogène utilisé est un polypeptide d'AMH, ou polynucléotide codant pour ce polypeptide, dénué, par rapport à la séquence entière d'AMH, de tous les acides aminés situés après la partie médiane de la région pro de l'AMH, à savoir un polypeptide d'AMH, ou un polynucléotide codant pour ledit polypeptide d'AMH, comprenant au moins les 230 acides aminés de séquence SEQIDN°10 ou de séquence ayant au moins 75% d'identité avec la séquence SEQ ID N°10 et au plus les 255 acides aminés de séquence SEQ ID N°8 ou de séquence ayant au moins 75% d'identité avec la séquence SEQ ID N°8, de préférence choisi parmi les polypeptides d'AMH de séquences SEQ ID N°8, SEQ ID N°9 et SEQ ID N°10 ou de séquences ayant au moins 75% d'identité avec ces séquences. La séquence SEQ ID N°8 correspond aux acides aminés 1-255 de l'AMH humaine (appelée AMH-2), la séquence SEQ ID N°9 correspond aux acides aminés 19-255 de l'AMH humaine, à savoir la séquence SEQ ID N°8 sans le peptide signal (appelée AMH-2 sans peptide signal) et la séquence SEQ ID N°10 correspond aux acides aminés 26-255 de l'AMH humaine, à savoir la séquence SEQ ID N°8 sans le peptide signal, ni la partie précurseur (appelée AMH-2 sans peptide signal ni partie précurseur).

Selon un autre mode de réalisation, l'immunogène utilisé est un polypeptide d'AMH, ou polynucléotide codant pour ce polypeptide, qui correspond à l'AMH totale, avec toute ou partie du peptide signal et éventuellement toute ou partie de la partie précurseur. Ainsi, le polypeptide d'AMH comprend au moins les 535 acides aminés de séquence SEQ ID N°4 ou de séquence ayant au moins 75% d'identité avec la séquence SEQ ID N°4, de préférence choisi parmi les polypeptides d'AMH de séquences SEQ ID N°2, SEQ ID N°3 et SEQ ID N°4 ou de séquences ayant au moins 75% d'identité avec ces séquences. La séquence SEQ ID N°2 correspond aux acides aminés 1-560 de l'AMH humaine, la séquence SEQ ID N°3 correspond aux acides aminés 19-560 de l'AMH humaine, à savoir la séquence SEQ ID N°2 sans le peptide signal, et la séquence SEQ ID N°3 correspond aux acides aminés 26-560 de l'AMH humaine, à savoir la séquence SEQ ID N°2 sans le peptide signal, ni la partie précurseur.

Selon un autre mode de réalisation, l'immunogène utilisé est un polypeptide d'AMH, ou polynucléotide codant pour ce polypeptide, qui correspond à l'AMH totale, avec toute ou partie du peptide signal et éventuellement toute ou partie de la partie précurseur, mais sans la région mature. Ainsi, le polypeptide d'AMH comprend au moins les 426 acides aminés de séquence SEQ ID N°7 ou de séquence ayant au moins 75% d'identité avec la séquence SEQ ID N°7 et au plus les 451 acides aminés de séquence SEQ ID N°5 ou de séquence ayant au moins 75% d'identité avec la séquence SEQ ID N°5, de préférence choisi parmi les polypeptides d'AMH de séquences SEQ ID N°5, SEQ ID N°6 et SEQ ID N°7 ou de séquences ayant au moins 75% d'identité avec ces séquences. La séquence SEQ ID N°5 correspond aux acides aminés 1-451 de l'AMH humaine (appelée AMH-3), la séquence SEQ ID N°6 correspond aux acides aminés 19-451 de l'AMH humaine, à savoir la séquence SEQ ID N°5 sans le peptide signal (appelée AMH-3 sans peptide signal) et la séquence SEQ ID N°7 correspond aux acides aminés 26-451 de l'AMH humaine, à savoir la séquence SEQ ID N°5 sans le peptide signal, ni la partie précurseur (appelée AMH-3 sans peptide signal ni partie précurseur).

Le tableau ci-après (Tableau 1) présente différents polypeptides d'AMH utiles dans le cadre de l'invention, notamment en tant qu'immunogène pour certains, donnant le positionnement des acides aminés et les SEQ ID correspondantes (entre parenthèses) selon l'espèce de mammifère.

**Tableau 1**

| | Humaine | Equine | Canine | Bovine |
|---|---|---|---|---|
| Partie médiane de la région Pro de l'AMH | 157-255 (SEQ ID N°1) | 167-265 (SEQ ID N°14) | 166-264 (SEQ ID N°23) | 171-270 (SEQ ID N°32) |
| AMH totale | 1-560 (SEQ ID N°2) | 1-573 (SEQ ID N° 15) | 1-572 (SEQ ID N°24) | 1-575 (SEQ ID N°33) |
| AMH totale sans peptide signal | 19-560 (SEQ ID N°3) | 23-573 (SEQ ID N°16) | 22-572 (SEQ ID N°25) | 18-575 (SEQ ID N°34) |
| AMH totale sans peptide signal, ni partie précurseur | 26-560 (SEQ ID N°4) | NA | NA | 25-575 (SEQ ID N°35) |
| AMH-3 totale | 1-451 (SEQ ID N°5) | 1-464 (SEQ ID N° 17) | 1-463 (SEQ ID N°26) | 1-466 (SEQ ID N°36) |
| AMH-3 totale sans peptide signal | 19-451 (SEQ ID N°6) | 23-464 (SEQ ID N°18) | 22-463 (SEQ ID N°27) | 18-466 (SEQ ID N°37) |
| AMH-3 totale sans peptide signal, ni partie précurseur | 26-451 (SEQ ID N°7) | NA | NA | 25-466 (SEQ ID N°38) |
| AMH-2 totale | 1-255 (SEQ ID N°8) | 1-265 (SEQ ID N°19) | 1-264 (SEQ ID N°28) | 1-270 (SEQ ID N°39) |
| AMH-2 totale sans peptide signal | 19-255 (SEQ ID N°9) | 23-265 (SEQ ID N°20 | 22-264 (SEQ ID N°29) | 18-270 (SEQ ID N°40) |
| AMH-2 totale sans peptide signal, ni partie précurseur | 26-255 (SEQIDN°10) | NA | NA | 25-270 (SEQ ID N°41) |

| | | | | |
|---|---|---|---|---|
| NA = Non Applicable car pas de partie précurseur dans l'AMH concernée | | | | |

Bien entendu, outre les acides aminés décrits dans ces séquences, l'immunogène, lorsqu'il est sous forme de polypeptide, peut également comprendre d'autres acides aminés utilisés pour la production du polypeptide ou sa purification, comme par exemple une queue polyhistidine.

Les méthodes de production des polypeptides sont largement connues de l'homme du métier. Par exemple, les polypeptides d'AMH peuvent être obtenus par génie génétique en utilisant des étapes, classiquement connues de l'homme du métier, consistant à :
- disposer de l'ADN codant pour les polypeptides d'AMH, ledit ADN étant obtenu selon des méthodes classiques,
- insérer cet ADN par clonage dans un vecteur d'expression tel qu'un plasmide, un cosmide, un phage λ ou un vecteur viral (baculovirus (Autographa californica Nuclear Polyhedrosis Virus), virus de la vaccine, virus de la fôret de Semliki, adénovirus, lentivirus, ...), lequel vecteur comprend également une origine de réplication (pour plasmides ou cosmides) ou système de réplication permettant son amplification dans la cellule hôte et un ou des promoteurs permettant la transcription d'ARN messagers qui seront traduits en protéines,

- introduire le vecteur avec le gène d'intérêt pour expression dans une cellule hôte, tel qu'une cellule procaryote (par exemple bactérie comme Escherichia coli, Bacillus subtilis) par transformation ou infection ou une cellule eucaryote (par exemple levures (Saccharomyces cerevisiae, Pichia pastoris), cellules d'insectes (cellules Sf9, Sf21, High5), cellules de mammifères (CHO, 293, Per.C6, BHK-21, Vero, ...) par transfection transitoire ou permanente, ou encore infection virale,
- culture et éventuellement multiplication de la cellule hôte contenant le vecteur d'expression, avec éventuellement amplification du vecteur dans la cellule hôte,
- au besoin, induction de la transcription et de la synthèse protéique pour la production des polypeptides recombinants d'AMH, et
- purification pour extraire lesdits polypeptides, par exemple grâce à une queue polyHistidine. Les polypeptides sont alors dits recombinants.

Lorsque l'immunogène est un polynucléotide, on utilisera par exemple le même ADN que celui qu'on utiliserait pour préparer le polypeptide d'AMH par génie génétique.

L'animal utilisé pour l'immunisation est tout animal habituellement utilisé pour obtenir des anticorps monoclonaux, tels que par exemple une souris, un rat, un lapin, une chèvre ou un mouton.

Les conditions et paramètres pour l'immunisation, tels que concentration en immunogène, mode d'immunisation, etc. sont largement connus de l'Homme du Métier qui pourra se référer aux procédures décrites par Kôlher et Milstein, dès 1975, et notamment dans le manuel Current protocols in Cell Biology (Yokoyama WM, 2001).

La deuxième étape du procédé de l'invention est une étape classique et consiste à préparer les hybridomes à partir des cellules d'un organe lymphoïde de l'animal ayant reçu l'immunogène, tel que par exemple la rate, les ganglions lymphatiques et les amygdales. Une telle étape est décrite dans tout manuel de production d'anticorps monoclonaux. Toutefois, il faut utiliser le partenaire de fusion adapté à chaque espèce. Ainsi, par exemple, pour la souris, on peut se servir de la lignée cGPS CHO-Sa, pour le lapin de la lignée 240E-W (US7,429,487) et pour le mouton de la lignée SFP1 (WO92/15699).

La troisième étape du procédé consiste en la sélection particulière des hybridomes obtenus. Pour ce faire, les hybridomes sont tout d'abord mis en culture dans un milieu approprié, comme bien connu de l'Homme du Métier, comprenant notamment des nutriments pour leur multiplication, des stimulateurs de croissance. Puis la sélection des clones sécrétant les anticorps d'intérêt est effectuée en utilisant l'antigène reconnu par les anticorps selon des techniques connues de l'homme du métier telles que la détection directe sans marqueur (précipitation spontanée après réaction antigène-anticorps), la détection indirecte (par exemple ELISA ou autre type d'immunoessai), la résonnance plasmonique de surface (BIAcore®) et l'interférométrie (Octet). L'originalité de cette étape consiste en la sélection particulière des hybridomes qui peut être mise en oeuvre en 2 criblages successifs, dans n'importe quel ordre, avec des polypeptides et peptides particuliers.

Le premier criblage, appelé criblage 1, consiste à ne retenir que les hybridomes qui produisent des anticorps reconnaissant la partie médiane de la région pro, comme décrit précédemment, zone étant connue pour présenter pourtant un site de clivage et étant plutôt à exclure. Pour ce faire, deux méthodes sont possibles :
Méthode 1 : On sélectionne tout d'abord les hybridomes sécrétant des anticorps reconnaissant l'AMH-2, à savoir, pour l'AMH-2 humaine, les acides aminés 1-255 (SEQ ID N°8), le cas échéant sans toute ou partie du peptide signal et toute ou partie de la partie précurseur, ou toute séquence ayant au moins 75% d'identité avec cette séquence. Selon un mode de réalisation, le polypeptide AMH-2 reconnu par les anticorps est un polypeptide de séquences choisies parmi : SEQ ID N°8, SEQ ID N°9 et SEQ ID N°10 et de séquences ayant au moins 75% d'identité avec ces séquences. Puis on écarte les hybridomes qui produisent des anticorps reconnaissant la partie de l'AMH située avant la partie médiane de la région pro de l'AMH, à savoir, pour l'AMH humaine, les acides aminés 1-156 (SEQ ID N°11), le cas échéant sans toute ou partie du peptide signal et toute ou partie de la partie précurseur, et toute séquence ayant au moins 75% d'identité avec cette séquence. Ce polypeptide d'AMH est appelé polypeptide d'AMH (a) non reconnu par les anticorps préparés selon l'invention. Selon un mode de réalisation, le polypeptide d'AMH (a) non reconnu par les anticorps est un polypeptide de séquences choisies parmi: SEQ ID N°11, SEQIDN°12 et SEQ IDN°13 et de séquences ayant au moins 75% d'identité avec ces séquences. La séquence SEQIDN°11 correspond aux acides aminés 1-156 de l'AMH humaine (appelée AMH-1), la séquence SEQ ID N°12 correspond aux acides aminés 19-156 de l'AMH humaine, à savoir la séquence SEQIDN°11 sans le peptide signal (appelée AMH-1 sans peptide signal) et la séquence SEQ ID N°13 correspond aux acides aminés 26-156 de l'AMH humaine, à savoir la séquence SEQ IDN°11 sans le peptide signal, ni la partie précurseur (appelée AMH-1 sans peptide signal ni partie précurseur).
Méthode 2 : On sélectionne les hybridomes sécrétant des anticorps reconnaissant la partie médiane de la région pro de l'AMH, à savoir, pour l'AMH humaine, les acides aminés 157-255 (SEQ ID N°1) et toute séquence ayant au moins 75% d'identité avec cette séquence.

Le tableau ci-après (Tableau 2) présente différents polypeptides d'AMH (a), non reconnus, utiles dans le cadre de l'invention, donnant le positionnement des acides aminés et les SEQ ID correspondantes (entre parenthèses) selon l'espèce de mammifère.

**Tableau 2**

| | Humaine | Equine | Canine | Bovine |
|---|---|---|---|---|
| AMH-1 totale | 1-156 (SEQ ID N°11) | 1-166 (SEQ ID N°21) | 1-165 (SEQ ID N°30) | 1-170 (SEQ ID N°42) |
| AMH-1 totale sans peptide signal | 19-156 (SEQ ID N°12) | 23-166 (SEQ ID N°22) | 22-165 (SEQ ID N°31) | 18-170 (SEQ ID N°43) |
| AMH-1 totale sans peptide signal, ni partie précurseur | 26-156 (SEQ ID N°13) | NA | NA | 25-170 (SEQ ID N°44) |

| | | | | |
|---|---|---|---|---|
| NA = Non Applicable car pas de partie précurseur dans l'AMH concernée | | | | |

Ainsi, selon un mode de réalisation particulier, les séquences ayant au moins 75% d'identité avec les séquences SEQ ID N°11, SEQ ID N°12, et SEQ ID N°13 sont choisies parmi les séquences: SEQ ID N°21, SEQ ID N°22, SEQ ID N°30, SEQ ID N°31, SEQ ID N°42, SEQ ID N°43 et SEQ ID N°44.

Les polypeptides d'AMH (a) peuvent être préparés comme décrit précédemment et comprendre d'autres acides aminés n'appartenant pas à l'AMH.

Le deuxième criblage, appelé criblage 2 consiste à écarter les hybridomes qui produisent des anticorps qui reconnaissent des épitopes linéaires dans la partie médiane de la région pro de l'AMH, à savoir dans la séquence SEQ ID N°1 ou toute séquence présentant 75% d'identité avec la séquence SEQ ID N°1. Ces épitopes sont appelés épitopes linéaires (b).

La détermination d'épitopes linéaires sur une séquence d'acides aminés est bien connue de l'homme du métier. Elle consiste à découper la séquence d'intérêt en peptides chevauchant de longueur déterminée, par exemple de 10, 12 ou 15 acides aminés, puis d'identifier parmi ces peptides ceux qui sont reconnus par des anticorps monoclonaux ou polyclonaux.

Le tableau ci-après (Tableau 3) présente différents épitopes linéaires (b) déterminés dans la partie médiane de la région pro de l'AMH, utiles dans le cadre de l'invention, donnant les SEQ ID selon l'espèce de mammifère.

**Tableau 3**

| | Humaine | Equine | Canine | Bovine |
|---|---|---|---|---|
| Epitopes linéaires sur la partie médiane de la région pro | SEQ ID N°45 | SEQ ID N°48 | SEQ ID N°51 | SEQ ID N°54 |
| | SEQ ID N°46 | SEQ ID N°49 | SEQ ID N°52 | SEQ ID N°55 |
| | SEQ ID N°47 | SEQ ID N°50 | SEQ ID N°53 | SEQ ID N°56 |

Ainsi, selon un mode de réalisation, les épitopes linéaires (b) non reconnus par les anticorps ont les séquences SEQ ID N°45 à SEQ ID N°56.

Comme précédemment, les épitopes sont produits selon des méthodes classiques, comme décrit précédemment. Ils peuvent également être produits par synthèse peptidique, ce qui est souvent préféré.

Un mode de réalisation particulier comprend au moins des caractéristiques suivantes pour l'étape de sélection (iii) :
- Le criblage 1 met en oeuvre la méthode 1 ;
- Le criblage 1 est effectué avant le criblage 2.

Entre chaque étape de criblage, si besoin, les anticorps contenus dans les surnageants d'hybridomes sont purifiés selon les techniques classiques, comme par exemple la chromatographie d'affinité sur protéine A. Une fois la sélection effectuée et/ou si besoin, entre chaque étape de criblage, les hybridomes sont clonés afin de garantir une lignée stabilisée et homogène. Ceci est largement connu de l'homme du métier.

La dernière étape du procédé de l'invention consiste en la production des anticorps d'intérêt, étape également bien connue de l'homme du métier et décrite dans tous les manuels de production d'anticorps monoclonaux, comme par exemple le manuel Current Protocols in Cell Biology.

Outre les étapes ci-dessus, dont le mode opératoire générale a été largement décrit, le procédé de l'invention peut également comprendre d'autres étapes telles que de lavage, conservation et transformation des anticorps en fragments.

Dans le procédé de préparation d'anticorps anti-AMH de l'invention, l'immunogène (polypeptide d'AMH ou polynucléotide codant pour ce polypeptide comprenant au moins les 99 acides aminés de séquence SEQ ID N°1 ou de séquence ayant au moins 75% d'identité avec la séquence SEQ ID N°1, et au plus les 560 acides aminés de séquence SEQ ID N°2 ou de séquence ayant au moins 75% d'identité avec la séquence SEQ ID N°2), la partie médiane de la région pro de l'AMH (polypeptide d'AMH comprenant au moins les 99 acides aminés de séquence SEQ ID N°1, ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8), le polypeptide (a) non reconnu par les anticorps d'intérêt (polypeptide d'AMH comprenant au moins les 131 acides aminés de séquence SEQIDN°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11) et les épitope linéaires (situés dans la séquence SEQ ID N°1 ou une séquence ayant au moins 75% d'identité avec la séquence SEQ ID N°1) appartiennent de préférence à la même espèce.

Selon un mode de réalisation :
- le mammifère est l'humain, l'immunogène est un polypeptide d'AMH, ou un polynucléotide codant pour ce polypeptide d'AMH, lequel comprend au moins les 99 acides aminés de séquence SEQ ID N°1 et au plus les 560 acides aminés de séquence SEQ ID N°2, le polypeptide d'AMH reconnu par les anticorps sécrétés par les hybridomes est un polypeptide d'AMH comprenant au moins les 99 acides aminés de séquence SEQ ID N°1 et au plus les 255 acides aminés de séquence SEQ ID N°8, et le polypeptide d'AMH (a) non reconnu par les anticorps sécrétés par les hybridomes est un polypeptide d'AMH comprenant au moins les 131 acides aminés de séquence SEQ ID N°13 et au plus les 156 acides aminés de séquence SEQ ID N°11, ou
- le mammifère est le cheval, l'immunogène est un polypeptide d'AMH, ou un polynucléotide codant pour ce polypeptide d'AMH, lequel comprend au moins les 99 acides aminés de séquence SEQ ID N°14 et au plus les 573 acides aminés de séquence SEQ ID N° 15, le polypeptide d'AMH reconnu par les anticorps sécrétés par les hybridomes est un polypeptide d'AMH comprenant au moins les 99 acides aminés de séquence SEQ ID N°14 et au plus les 265 acides aminés de séquence SEQ ID N°19, et le polypeptide d'AMH (a) non reconnu par les anticorps sécrétés par les hybridomes est un polypeptide d'AMH comprenant au moins les 144 acides aminés de séquence SEQ ID N°22 et au plus les 166 acides aminés de séquence SEQ ID N°21, ou
- le mammifère est le chien, l'immunogène est un polypeptide d'AMH, ou un polynucléotide codant pour ce polypeptide d'AMH, lequel comprend au moins les 99 acides aminés de séquence SEQ ID N°23 et au plus les 572 acides aminés de séquence SEQ ID N°24, le polypeptide d'AMH reconnu par les anticorps sécrétés par les hybridomes est un polypeptide d'AMH comprenant au moins les 99 acides aminés de séquence SEQ ID N°23 et au plus les 264 acides aminés de séquence SEQ ID N°28, et le polypeptide d'AMH (a) non reconnu par les anticorps sécrétés par les hybridomes est un polypeptide d'AMH comprenant au moins les 144 acides aminés de séquence SEQ ID N°31 et au plus les 165 acides aminés de séquence SEQ ID N°30, ou
- le mammifère est un bovin, l'immunogène est un polypeptide d'AMH, ou un polynucléotide codant pour ce polypeptide d'AMH, lequel comprend au moins les 100 acides aminés de séquence SEQ ID N°32 et au plus les 575 acides aminés de séquence SEQ ID N°33, le polypeptide d'AMH reconnu par les anticorps sécrétés par les hybridomes est un polypeptide d'AMH comprenant au moins les 100 acides aminés de séquence SEQ ID N°32 et au plus les 270 acides aminés de séquence SEQ ID N°39, et le polypeptide d'AMH (a) non reconnu par les anticorps sécrétés par les hybridomes est un polypeptide d'AMH comprenant au moins les 146 acides aminés de séquence SEQ ID N°44 et au plus les 270 acides aminés de séquence SEQ ID N°42.

En particulier :
- le mammifère est l'humain, l'immunogène est un polypeptide d'AMH, ou un polynucléotide codant pour ce polypeptide d'AMH, choisi parmi les polypeptides de séquence SEQ ID N°2 à 10, le polypeptide d'AMH reconnu par les anticorps sécrétés par les hybridomes est un polypeptide d'AMH choisi parmi les polypeptides de séquence SEQ ID N°1 et SEQ ID N°8 à 10, et le polypeptide d'AMH (a) non reconnu par les anticorps sécrétés par les hybridomes est un polypeptide d'AMH choisi parmi les polypeptides de séquence SEQ ID N°11 à 13, ou
- le mammifère est le cheval, l'immunogène est un polypeptide d'AMH, ou un polynucléotide codant pour ce polypeptide d'AMH, choisi parmi les polypeptides de séquence SEQ ID N°15 à 20, le polypeptide d'AMH reconnu par les anticorps sécrétés par les hybridomes est un polypeptide d'AMH choisi parmi les polypeptides de séquence SEQ ID N°14 et SEQ ID N°19 et 20, et le polypeptide d'AMH (a) non reconnu par les anticorps sécrétés par les hybridomes est un polypeptide d'AMH choisi parmi les polypeptides de séquence SEQ ID N°21 et 22, ou
- le mammifère est le chien, l'immunogène est un polypeptide d'AMH, ou un polynucléotide codant pour ce polypeptide d'AMH, choisi parmi les polypeptides de séquence SEQ ID N°24 à 29, le polypeptide d'AMH reconnu par les anticorps sécrétés par les hybridomes est un polypeptide d'AMH choisi parmi les polypeptides de séquence SEQ ID N°23 et SEQ ID N°28 et 29, et le polypeptide d'AMH (a) non reconnu par les anticorps sécrétés par les hybridomes est un polypeptide d'AMH choisi parmi les polypeptides de séquence SEQ ID N°30 et 31, ou
- le mammifère est un bovin, l'immunogène est un polypeptide d'AMH, ou un polynucléotide codant pour ce polypeptide d'AMH, choisi parmi les polypeptides de séquence SEQ ID N°33 à 41, le polypeptide d'AMH reconnu par les anticorps sécrétés par les hybridomes est un polypeptide d'AMH choisi parmi les polypeptides de séquence SEQ ID N°32 et SEQ ID N°39 à 41, et le polypeptide d'AMH (a) non reconnu par les anticorps sécrétés par les hybridomes est un polypeptide d'AMH choisi parmi les polypeptides de séquence SEQ ID N°42 à 44.

Les anticorps, produits par le procédé de l'invention, sont nouveaux et peuvent être produits par un autre procédé, tandis qu'ils gardent les mêmes caractéristiques. Aussi, un autre objet de l'invention concerne les anticorps ou fragments d'anticorps monoclonaux anti-AMH de mammifère reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQ ID N°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11, ni aucun épitope linéaire situé dans la séquence SEQ ID N°1 ou une séquence ayant au moins 75% d'identité avec la séquence SEQ ID N°1.

Par fragment d'anticorps monoclonal, on entend toute partie de l'anticorps qui a les mêmes caractéristiques de reconnaissance immunologique que l'anticorps dont il est issu, dans le cas présent il est capable de reconnaître la partie médiane de la région pro de l'AMH et aussi l'AHM entière. A titre d'exemples de fragments, on peut citer les fragments Fab, Fab', F(ab')2 ainsi que les scFv (Single chain variable fragment), dsFv (Double-stranded variable fragment). Ces fragments fonctionnels peuvent notamment être obtenus par génie génétique ou encore par digestion protéolytique spécifique puis purification.

Les caractéristiques et définitions décrites précédemment pour le procédé de l'invention s'appliquent également aux anticorps de l'invention.

Ainsi, les anticorps de l'invention peuvent remplir une ou plusieurs des conditions suivantes :
- le polypeptide d'AMH (a) non reconnu est un polypeptide de séquences choisies parmi : SEQ ID N°11, SEQ ID N°12 et SEQ ID N°13 ou de séquences ayant au moins 75% d'identité avec ces séquences,
- les séquences ayant au moins 75% d'identité avec les séquences SEQ ID N°11, SEQ ID N°12, et SEQ ID N°13 sont choisies parmi les séquences: SEQ ID N°21, SEQ ID N°22, SEQ ID N°30, SEQ ID N°31, SEQ ID N°42, SEQ ID N°43 et SEQ ID N°44,
- les épitopes linéaires (b) non reconnus par les anticorps ont les séquences SEQ ID N°45 à SEQ ID N°56.

En particulier,
- le mammifère est l'humain, le polypeptide d'AMH reconnu est un polypeptide d'AMH comprenant au moins les 99 acides aminés de séquence SEQ ID N°1 et au plus les 255 acides aminés de séquence SEQ ID N°8, et le polypeptide d'AMH (a) non reconnu est un polypeptide d'AMH comprenant au moins les 131 acides aminés de séquence SEQ ID N°13 et au plus les 156 acides aminés de séquence SEQ ID N°11, ou
- le mammifère est le cheval, le polypeptide d'AMH reconnu est un polypeptide d'AMH comprenant au moins les 99 acides aminés de séquence SEQ ID N°14 et au plus les 265 acides aminés de séquence SEQ ID N°19, et le polypeptide d'AMH (a) non reconnu est un polypeptide d'AMH comprenant au moins les 144 acides aminés de séquence SEQ ID N°22 et au plus les 166 acides aminés de séquence SEQ ID N°21, ou
- le mammifère est le chien, le polypeptide d'AMH reconnu est un polypeptide d'AMH comprenant au moins les 99 acides aminés de séquence SEQ ID N°23 et au plus les 264 acides aminés de séquence SEQ ID N°28, et le polypeptide d'AMH (a) non reconnu est un polypeptide d'AMH comprenant au moins les 144 acides aminés de séquence SEQ ID N°31 et au plus les 165 acides aminés de séquence SEQ ID N°30, ou
- le mammifère est un bovin, le polypeptide d'AMH reconnu est un polypeptide d'AMH comprenant au moins les 100 acides aminés de séquence SEQ ID N°32 et au plus les 270 acides aminés de séquence SEQ ID N°39, et le polypeptide d'AMH (a) non reconnu est un polypeptide d'AMH comprenant au moins les 146 acides aminés de séquence SEQ ID N°44 et au plus les 270 acides aminés de séquence SEQ ID N°42.

Les anticorps anti-AMH de l'invention sont particulièrement utiles pour le dosage d'AMH dans un échantillon biologique susceptible de contenir de l'AMH. Ils peuvent être utilisés tels quels, sous forme entier ou de fragment, et/ou sous forme conjuguées avec un marqueur capable de générer l'émission d'un signal détectable pour la visualisation d'une réaction immunologique après liaison avec l'AMH de l'échantillon, ce qui constitue un autre objet de l'invention.

Par marqueur capable de générer l'émission d'un signal détectable pour la visualisation d'une réaction immunologique, on entend toute molécule contenant un groupement réactif avec un groupement de l'anticorps ou du fragment d'anticorps, directement sans modification chimique, ou après modification chimique pour inclure un tel groupement, laquelle molécule est capable de générer directement ou indirectement un signal détectable qui sera utilisé pour donner le dosage recherché.

La liaison entre le marqueur et l'anticorps ou fragment d'anticorps de l'invention peut être réalisée par toute méthode connue de l'homme du métier, et en particulier par couplage.

Des exemples de marqueurs comprennent notamment :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le 32P, le 35S ou le 1251,
- les molécules fluorescentes telles que les Alexa ou les phycocyanines, et
- les sels électrochimiluminescents tels que des dérivés organo-métalliques à base d'acridinium ou de ruthénium.

Des exemples de systèmes indirects de détection comprennent par exemple des ligands capables de réagir avec un anti-ligand. Le ligand correspond alors au marqueur pour constituer, avec l'anticorps ou fragment d'anticorps, le conjugué.

Les couples ligand/anti-ligand sont bien connus de l'homme du métier, et comprennent par exemple les couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Le dosage de l'AMH est effectué par immunoessai sandwich qui est un essai largement connu de l'homme du métier. En quelques mots, il consiste à déterminer un analyte, dans le cas présent l'AMH de mammifère, en mettant en oeuvre deux partenaires de liaison à l'analyte, dans le cas présent au moins un anticorps ou fragment d'anticorps de l'invention.

Bien entendu, le préfixe « immuno » dans le terme « immunoessai », par exemple, n'est pas à considérer dans la présente demande comme indiquant strictement que le partenaire de liaison, autre que l'anticorps ou fragment d'anticorps de l'invention, est nécessairement un partenaire d'origine immunologique, tel qu'un anticorps ou un fragment d'anticorps. En effet, comme cela est bien connu de l'homme du métier, ce terme est plus largement utilisé pour désigner aussi des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition essentielle est que le partenaire de liaison concerné soit capable de se lier à l'analyte recherché, dans le cas présent l'AMH, de préférence de manière spécifique. Ainsi, il est connu de parler de l'essai ELISA pour des essais qui utilisent des partenaires de liaison non immunologiques stricto sensu, appelés plus largement en anglais « ligand binding assay », que l'on pourrait traduire en langue française par « essai utilisant la liaison à un ligand », alors que le terme « immuno » est inclus dans l'intitulé in extenso correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et quantifier ce dernier, de préférence de manière spécifique, même quand le partenaire de liaison autre que l'anticorps ou fragment d'anticorps de l'invention n'est pas de nature ou d'origine immunologique au sens strict.

Un autre objet de l'invention concerne donc un procédé de quantification d'AMH de mammifère par immunoessai sandwich, dans un échantillon biologique susceptible de contenir de l'AMH, lequel comprend ou consiste en les étapes suivantes :
- mettre en contact ledit échantillon biologique avec deux partenaires de liaison à l'AMH dont au moins un desdits partenaires est un anticorps, un fragment d'anticorps ou un conjugué de l'invention,
- détecter un signal émis par la liaison entre lesdits partenaires de liaison et l'AMH, si elle est présente, en utilisant un marqueur capable de générer l'émission d'un signal détectable, et
- transformer le signal détecté en une concentration d'AMH.

La première étape de ce procédé de quantification comprend ou consiste en la mise en contact d'un échantillon biologique avec deux partenaires de liaison à l'AMH dont au moins un desdits partenaires est un anticorps, un fragment d'anticorps ou un conjugué de l'invention.

Les échantillons biologiques susceptibles de contenir de l'AMH de mammifère sont les échantillons de sang, de sérum, de plasma, de liquide folliculaire et de sperme. Selon un mode de réalisation, l'échantillon biologique est un échantillon de sang, de sérum ou de plasma.

Le partenaire de liaison autre que l'anticorps ou fragment d'anticorps de l'invention, éventuellement sous forme de conjugué, comprend toute molécule capable de se lier à l'AMH. A titre d'exemple d'un tel partenaire de liaison, on peut citer les anticorps polyclonaux anti-AMH, les anticorps monoclonaux anti-AMH, les fragments d'anticorps monoclonaux anti-AMH, les analogues d'anticorps (molécules capables de mimer les anticorps) tels que les nanofitines, les aptamères ou encore les « DARPins », ou toute autre molécule qui est connue pour avoir une interaction avec l'AMH.

Les analogues d'anticorps nanofitines sont de petites protéines qui, comme les anticorps, sont capables de se lier à une cible biologique permettant ainsi de la détecter, de la capturer ou tout simplement de la cibler au sein d'un organisme.

Les analogues d'anticorps aptamères sont des oligonucléotides, généralement ARN ou ADN, identifiés dans des banques contenant jusqu'à 1015 séquences différentes, par une méthode combinatoire de sélection in vitro appelée SELEX pour « Systematic Evolution of Ligands by Exponentiel Enrichment » (Ellington AD et Szostak JW., 1990). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celle des anticorps.

Les analogues d'anticorps « DARPins » pour Designed Ankyrin Repeat ProteINS (Boersma YL et Plütckthun A, 2011) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par screening de banques combinatoires.

Les anticorps polyclonaux, les anticorps monoclonaux et les fragments d'anticorps anti-AMH peuvent être préparés de façon classique largement connue de l'homme du métier. Certains anticorps sont disponibles sur le marché, comme par exemple chez AnshLabs (US).

La Demanderesse a également préparé d'autres anticorps monoclonaux particulièrement utiles dans le cadre du dosage de l'AMH en utilisant le même procédé de préparation d'anticorps monoclonaux que celui décrit précédemment pour l'invention, à ceci près que la zone de reconnaissance des anticorps est la partie C-Terminale de la région pro de l'AMH, à savoir, pour l'AMH humaine, les acides aminés 256 à 451 (196 acides aminés SEQ ID N°57), pour l'AMH équine les acides aminés 266 à 464 (199 acides aminés SEQ ID N°58), pour l'AMH canine les acides aminés 265 à 463 (199 acides aminés SEQ ID N°59) et pour l'AMH bovine les acides aminés 271 à 466 (199 acides aminés SEQ ID N°60). La partie C-Terminale de la région pro d'intérêt est donc un polypeptide d'AMH comprenant au moins les 196 acides aminés de séquence SEQ ID N°57 ou de séquence ayant au moins 75% d'identité avec la séquence SEQ ID N°57. De façon inattendue, les anticorps reconnaissant la partie C-Terminale de la région pro de l'AMH n'ont pas besoin d'être utilisés en grande quantité dans le dosage dans lequel ils sont mis en oeuvre, contrairement aux anticorps de l'art antérieur.

Pour préparer ces anticorps reconnaissant la partie C-Terminale de l'AMH, l'immunogène utilisé est un polypeptide d'AMH, ou un polynucléotide codant pour ce polypeptide d'AMH, qui doit comprendre au moins la partie que les anticorps doivent reconnaître, à savoir au moins la partie C-Terminale de la région pro de l'AMH, à savoir au moins les 196 acides aminés de séquence SEQ ID N°57 ou de séquence ayant au moins 75% d'identité avec la séquence SEQ ID N°57. L'immunogène comprend également au plus l'AMH entière de l'espèce considérée ou le polynucléotide codant pour l'AMH entière de l'espèce considérée, à savoir, dans le cas de l'AMH humaine, les 560 acides aminés de séquence SEQ ID N°2, les autres AMH comprenant au moins 75% d'identité avec la séquence SEQ ID N°2. Un immunogène particulier peut être choisi parmi les suivants :
- un polypeptide d'AMH, ou polynucléotide codant pour ce polypeptide, dénué, par rapport à la séquence entière d'AMH, de tous les acides aminés situés après la partie C-Terminale de la région pro de l'AMH, à savoir un polypeptide d'AMH, ou un polynucléotide codant pour ledit polypeptide d'AMH, comprenant au moins les 426 acides aminés de séquence SEQ ID N°7 ou de séquence ayant au moins 75% d'identité avec la séquence SEQ ID N°7 et au plus les 451 acides aminés de séquence SEQ ID N°5 ou de séquence ayant au moins 75% d'identité avec la séquence SEQ ID N°5, de préférence choisis parmi les polypeptides d'AMH de séquences SEQ ID N°5, SEQ ID N°6 et SEQ ID N°7 ou de séquences ayant au moins 75% d'identité avec ces séquences, par exemple de séquences SEQ ID N°17, 18, 26, 27, 36, 37 ou 38. Ceci correspond à l'AMH-3, le cas échéant sans peptide signal et éventuellement sans partie précurseur ;
- un polypeptide d'AMH, ou polynucléotide codant pour ce polypeptide, qui correspond à l'AMH totale, avec toute ou partie du peptide signal et éventuellement toute ou partie de la partie précurseur, à savoir qui comprend au moins les 535 acides aminés de séquence SEQ ID N°4 ou de séquence ayant au moins 75% d'identité avec la séquence SEQ ID N°4, de préférence choisis parmi les polypeptides d'AMH de séquences SEQ ID N°2, SEQ ID N°3 et SEQ ID N°4 ou de séquences ayant au moins 75% d'identité avec ces séquences, par exemple de séquences SEQ ID N°15, 16, 24, 25, 33, 34 ou 35.

La sélection des hybridomes se fait également en utilisant 2 étapes de criblages, dans n'importe quel ordre, avec des polypeptides et peptides particuliers.

Le premier criblage, appelé criblage 1, consiste à ne retenir que les hybridomes qui produisent des anticorps reconnaissant la partie C-Terminale de la région pro, comme décrit précédemment. Pour ce faire, deux méthodes sont possibles :
Méthode 1 : On sélectionne tout d'abord les hybridomes sécrétant des anticorps reconnaissant l'AMH-3, à savoir, pour l'AMH-3 humaine, les acides aminés 1-451 (SEQ ID N°5), le cas échéant sans toute ou partie du peptide signal et toute ou partie de la partie précurseur, ou toute séquence ayant au moins 75% d'identité avec cette séquence. Selon un mode de réalisation, le polypeptide AMH-3 reconnu par les anticorps est un polypeptide de séquences choisies parmi : SEQ ID N°5, SEQ ID N°6 et SEQ ID N°7 et de séquences ayant au moins 75% d'identité avec ces séquences. Puis on écarte les hybridomes qui produisent des anticorps reconnaissant la partie de l'AMH située avant la partie C-Terminale de la région pro de l'AMH, à savoir, pour l'AMH humaine, les acides aminés 1-255 (SEQ ID N°8 - AMH-2), le cas échéant sans toute ou partie du peptide signal et toute ou partie de la partie précurseur, et toute séquence ayant au moins 75% d'identité avec cette séquence. Ce polypeptide d'AMH est appelé polypeptide d'AMH (a) non reconnu par les anticorps préparés selon l'invention. Selon un mode de réalisation, le polypeptide d'AMH (a) non reconnu par les anticorps est un polypeptide de séquences choisies parmi : SEQ ID N°8, SEQ ID N°9 et SEQ ID N°10 et de séquences ayant au moins 75% d'identité avec ces séquences.
Méthode 2 : On sélectionne les hybridomes sécrétant des anticorps reconnaissant la partie C-Terminale de la région pro de l'AMH, à savoir, pour l'AMH humaine, les acides aminés 256-451 (SEQ ID N°57), ou toute séquence ayant au moins 75% d'identité avec cette séquence.

Le deuxième criblage, appelé criblage 2, consiste à écarter les hybridomes qui produisent des anticorps qui reconnaissent des épitopes linéaires (b) dans la partie C-Terminale de la région pro de l'AMH, à savoir dans la séquence SEQ ID N°57 ou toute séquence présentant 75% avec la séquence SEQ ID N°57.

Le tableau ci-après (Tableau 4) présente différents épitopes linéaires (b) déterminés dans la partie C-Terminale de la région pro de l'AMH, utiles pour préparer ces anticorps, donnant les SEQ ID selon l'espèce de mammifère.

**Tableau 4**

| | Humaine | Equine | Canine | Bovine |
|---|---|---|---|---|
| Epitopes linéaires sur la partie C-Terminale de la région pro | SEQ ID N°61 à 68 | SEQ ID N°69 à 74 SEQ ID N°62 et 63 | SEQ ID N°75 à 81 SEQ ID N°73 | SEQ ID N°82 à 85 SEQ ID N°62, 63, 70 et 73 |

Les anticorps anti-AMH ainsi préparés ont les caractéristiques suivantes : ils reconnaissent un polypeptide d'AMH comprenant au moins les 196 acides aminés de séquence SEQ ID N°57 ou de séquence ayant au moins 75% d'identité avec la séquence SEQ ID N°57, ils ne reconnaissent ni (a) un polypeptide d'AMH comprenant au moins les 230 acides aminés de séquence SEQ ID N°10, ou de séquence ayant au moins 75% d'identité avec la séquence SEQ IDN°10 et au plus les 255 acides aminés de séquence SEQ ID N°8, ou de séquence ayant au moins 75% d'identité avec la séquence SEQ ID N°8, ni (b) un épitope linéaire situé dans la séquence SEQ ID N°57 ou une séquence ayant au moins 75% d'identité avec la séquence SEQ ID N°57, par exemple les séquences SEQ ID N°61 à 85.

Toutes les caractéristiques et définitions décrites précédemment pour le procédé de préparation d'anticorps de l'invention et pour les anticorps de l'invention s'appliquent également ici.

L'un des deux partenaires peut être couplé à un marqueur pour former un conjugué ou un traceur. L'autre partenaire de liaison peut être capturé sur un support solide, et ce directement ou indirectement. On parle alors de partenaire de capture pour ce dernier et partenaire de détection pour le premier.

Les couples de partenaires de liaison à l'AMH utilisés dans le procédé de quantification d'AMH de l'invention peuvent reconnaître soit la région pro et la région mature de l'AMH, soit uniquement la région pro, notamment lorsqu'on utilise, avec les anticorps de l'invention, un anticorps anti-AMH reconnaissant la partie C-Terminale de la région pro. Ainsi, selon un mode de réalisation particulier, le deuxième partenaire de liaison à l'AMH utilisé dans le procédé de quantification est un anticorps reconnaissant un polypeptide d'AMH comprenant au moins les 196 acides aminés de séquence SEQ ID N°57 ou de séquence ayant au moins 75% d'identité avec la séquence SEQ ID N°57, mais ne reconnaissant ni (a) un polypeptide d'AMH comprenant au moins les 230 acides aminés de séquence SEQIDN°10, ou de séquence ayant au moins 75% d'identité avec la séquence SEQ IDN°10 et au plus les 255 acides aminés de séquence SEQ ID N°8, ou de séquence ayant au moins 75% d'identité avec la séquence SEQ ID N°8, ni (b) un épitope linéaire situé dans la séquence SEQ ID N°57 ou une séquence ayant au moins 75% d'identité avec la séquence SEQ ID N°57, ce dernier pouvant être sous forme de fragment et/ou de conjugué avec un marqueur capable de générer l'émission un signal détectable pour la visualisation d'une réaction immunologique entre ce conjugué et l'AMH d'un échantillon biologique.

La mise en contact de l'échantillon biologique avec les deux partenaires de liaison à l'AMH peut être en une étape ou en deux étapes, comme cela est largement connu de l'homme du métier. En quelques mots, un immunoessai en une étape comprend la mise en présence de l'échantillon à tester simultanément avec les deux partenaires de liaison, dont les anticorps, fragments d'anticorps ou conjugués de l'invention tels que définis précédemment, alors qu'un immunoessai en deux étapes comprend la mise en présence de l'échantillon à tester d'une part avec le premier partenaire de liaison, puis le complexe analyte-premier partenaire de liaison ainsi formé est mis en présence du deuxième partenaire de liaison, l'un des deux partenaires de liaison étant bien entendu un anticorps, fragment d'anticorps ou conjugué de l'invention tel que défini précédemment.

Le procédé peut également comprendre d'autres étapes connues de l'homme du métier, telles que des étapes de lavage et des étapes d'incubation.

La deuxième étape du procédé de quantification de l'invention comprend ou consiste en la détection d'un signal émis par la liaison entre lesdits partenaires de liaison et l'AMH, si elle est présente, en utilisant un marqueur capable de générer l'émission d'un signal détectable tel que défini précédemment. Ce marqueur peut être conjugué à l'un desdits partenaires de liaison à l'AMH.

Selon le type de marquage utilisé, l'Homme du Métier ajoutera des réactifs permettant la visualisation du marquage, ou l'émission du signal détectable, par tout type d'appareil de mesure approprié, comme par exemple un spectrophotomètre, un spectrofluorimètre, un densitomètre, un luminomètre ou encore une caméra haute définition.

La dernière étape du procédé de quantification d'AMH comprend ou consiste en la transformation du signal détecté en concentration d'AMH. On parle aussi de taux ou quantités d'AMH. Le principe général est que le signal mesuré émis lors de l'immunoessai est proportionnel à la quantité d'AMH de l'échantillon biologique.

Cette étape de transformation du signal détecté en concentration d'AMH est largement connue de l'homme du métier. Elle consiste à utiliser un modèle mathématique préétabli à partir d'une gamme étalon. Cette gamme étalon sera obtenue préalablement de façon connue. En quelques mots, l'obtention d'une gamme étalon consiste à mesurer le signal généré par des quantités ou concentrations croissantes et connues de l'analyte cible (AMH), à tracer la courbe donnant le signal en fonction du taux d'AMH et à trouver un modèle mathématique qui représente de la manière la plus fidèle possible cette relation. Le modèle mathématique sera utilisé pour déterminer les quantités, titres ou concentrations d'AMH inconnues, contenues dans l'échantillon biologique à tester.

La détermination de la concentration d'AMH peut être utilisée à plusieurs niveaux et notamment dans le cadre de la fertilité chez la femme, pour estimer la réserve ovarienne chez la femme, par exemple chez celles qui devraient recevoir un traitement lourd qui risquerait de détruire des follicules, mais également chez le garçon avant la puberté, par exemple en cas de troubles liés à la différentiation sexuelle.

Ainsi, un autre objet de l'invention concerne l'utilisation des anticorps, fragments d'anticorps ou conjugués de l'invention, qu'ils soient préparés ou non à partir du procédé de l'invention, ou bien du procédé de quantification de l'invention comme une aide :
- pour le diagnostic de troubles liés à un dysfonctionnement ovarien chez les femmes en âge de procréer, ou
- à l'évaluation de la réserve folliculaire ovarienne chez les jeunes filles de plus de 12 ans et les femmes, ou
- à l'évaluation des troubles liés à la différentiation sexuelle chez le garçon avant la puberté.

Pour mettre en oeuvre les procédés de l'invention, utilisés notamment selon les utilisations décrites ci-dessus, les anticorps, fragments ou conjugués de l'invention peuvent être contenus dans des trousses.

Aussi, un autre objet de l'invention concerne les trousses comprenant un anticorps ou fragment d'anticorps tel que défini ou préparé précédemment et/ou un conjugué tel que défini précédemment.

Là encore, les caractéristiques et définitions décrites précédemment dans le cadre des anticorps et procédés de l'invention s'appliquent aux trousses de l'invention.

Selon un mode de réalisation particulier, les trousses comprennent ou contiennent également au moins un contrôle positif. Ce contrôle positif comprend un composé capable de se lier aux partenaires de liaison mis en oeuvre lors de l'utilisation de la trousse, le composé étant présent en un taux prédéterminé.

A titre d'exemples non limitatifs de tels composés, on peut citer l'AMH totale de séquence SEQ ID N°2 ou de séquence ayant au moins 75% d'identité avec la séquence SEQ ID N°2, le cas échéant avec toute ou partie du peptide signal et éventuellement toute ou partie de la partie précurseur.

Les trousses peuvent également contenir tous les composés nécessaires pour la mise en évidence de la réaction entre les partenaires de liaison et l'AMH, tels que des tampons de lavage ou des réactifs permettant la visualisation d'un marquage ou l'émission d'un signal détectable.

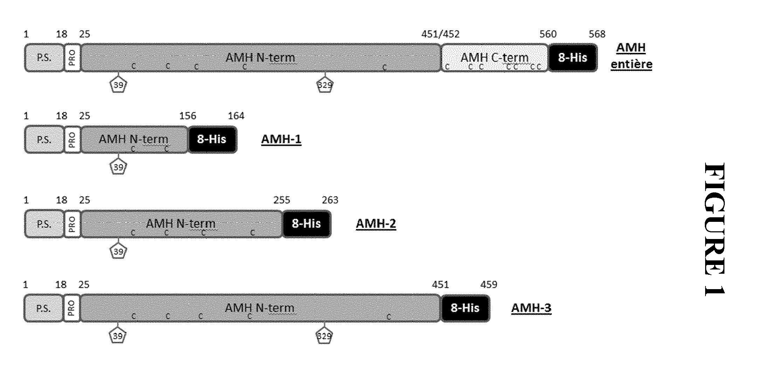

L'invention sera mieux comprise à l'aide des exemples suivants qui sont donnés à titre illustratif et non limitatif, ainsi qu'à l'aide des figures 1 à 4, dans lesquelles :
- La Figure 1 est une représentation schématique de la structure de la protéine AMH humaine et des constructions protéiques AMH-1, AMH-2 et AMH-3 humaines qui en dérivent.

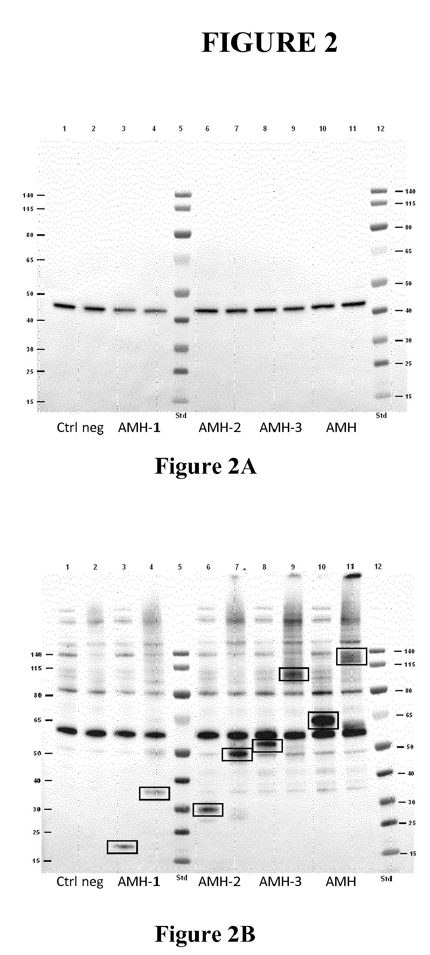

- La Figure 2 est une analyse par Western blot des lysats cellulaires obtenus par transfections des constructions géniques, AMH-1, AMH-2, AMH-3 et AMH entière dans les cellules HEK293T, après séparation par électrophorèse sur un gel Bis-Tris 4-12%. Puits 1, 2 : lysat contrôle négatif (cellules transfectées par un plasmide vide) ; puits 3, 4 : lysat AMH-1 ; puits 5, 12 : standard de poids moléculaire ; puits 6, 7 : lysat AMH-2 ; puits 8, 9 : lysat AMH-3 ; puits 10, 11 : lysat AMH entière. Les puits 1, 3, 6, 8 et 10 correspondent à la condition A : les échantillons déposés ont été chauffés et réduits. Les puits 2, 4, 7, 9 et 11 correspondent à la condition B : les échantillons déposés ont été chauffés mais pas réduits. La figure 2A est une photographie de la membrane testée avec un anticorps anti-β-actine, qui sert à contrôler que des quantités équivalentes de protéines totales ont été déposées dans chaque puits. La figure 2B est une photographie de la membrane testée avec un anticorps anti-histidines (Qiagen). Les bandes qui correspondent aux produits d'expression des constructions AMH ont été encadrées.

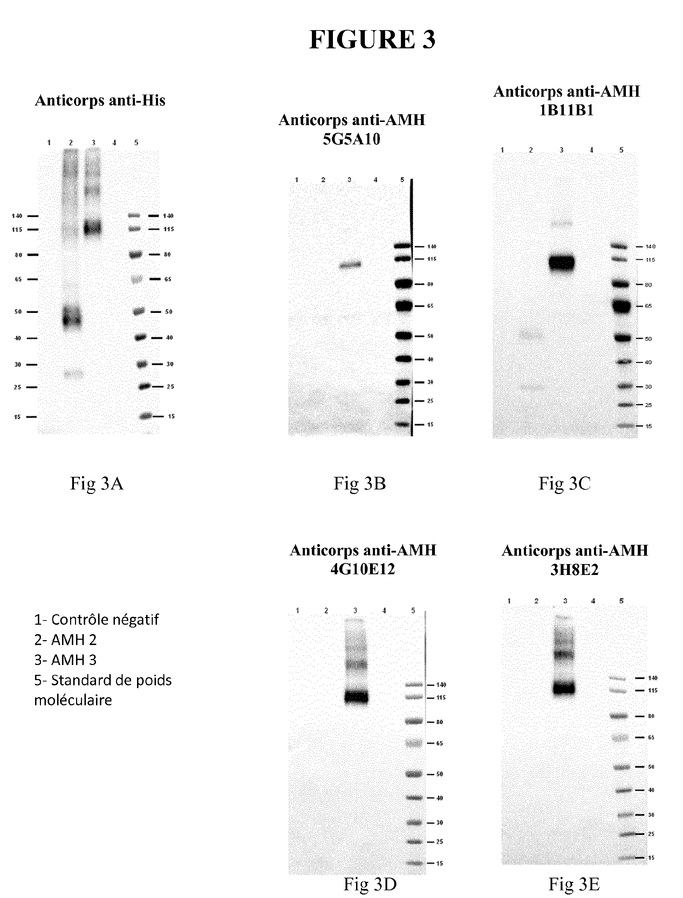

- La Figure 3 est une analyse par Western blot des protéines AMH-2 et AMH-3 purifiées à partir des surnageants de transfections des constructions géniques dans les cellules HEK293T, après séparation par électrophorèse sur un gel Bis-Tris 4-12%. Puits 1 : contrôle négatif (cellules transfectées par un plasmide vide) ; puits 2 : AMH-2 ; puits 3 : AMH-3 ; puits 5 : standard de poids moléculaire. Les échantillons déposés ont été chauffés mais pas réduits. Cinq membranes identiques (figures 3A à 3E) ont été préparées et chacune testée soit par un anticorps anti-AMH, soit un anticorps anti-histidines (Qiagen).

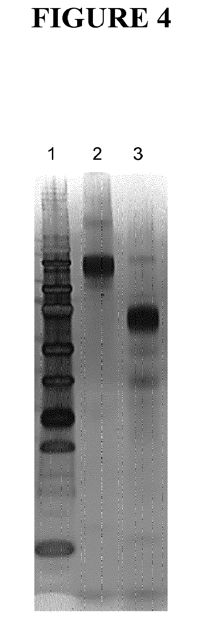

- La Figure 4 est une analyse en SDS-PAGE de la protéine AMH recombinante CHO-AMH5 3F1 purifiée par chromatographie d'affinité. Le gel SDS-PAGE a été coloré au nitrate d'argent afin de visualiser les protéines totales. Puits 1 : standard de poids moléculaire ; puits 2 : AMH recombinante chauffée mais pas réduite ; puits 3 : AMH recombinante chauffée et réduite.

### EXEMPLES

### Exemple 1 : Clonage de fragments d'ADN correspondant à la séquence entière de l'AMH humaine ou à des séquences tronquées, et transfection transitoire en cellule HEK 293T

### 1.1. Constructions géniques

La séquence d'AMH entière exprimée est celle de l'AMH humaine qui comprend 560 acides aminés (SEQ ID N°2 correspondant au n° accession P03971 de la base de donnée Uniprot KB), cette construction est dénommée AMH-560. Elle comprend le peptide signal (acides aminés 1-18) et une partie précurseur (acides aminés 19-25) qui sont clivés lors de la maturation post-traductionnelle de la protéine.

Trois autres constructions protéiques appelées AMH-1, AMH-2 et AMH-3 correspondant respectivement aux séquences en acides aminés 1-156 (SEQ ID N°11), 1-255 (SEQ ID N°8) et 1-451 (SEQ ID N°5) de l'AMH ont été réalisées. Un tag poly histidines (8-His) a été ajouté du côté C-terminal pour ces 4 constructions afin de faciliter la purification (Tag non ajouté dans le listage de séquences).

Les fragments d'ADN correspondant aux constructions AMH-1, AMH-2, AMH-3 et AMH entière, dont les séquences sont données dans le Tableau 5 ci-dessous, ont été obtenus sous forme de gènes synthétiques auprès de la société GeneArt® (Life Technologies). Chaque fragment d'ADN (AMH-1, AMH-2, AMH-3 et AMH entière) a été cloné entre les sites Eco RI et Not I dans le vecteur pCMV6-XL5 sous le contrôle du promoteur CMV. Les plasmides obtenus ont été vérifiés par séquençage au niveau des inserts afin de s'assurer qu'ils ne comportaient pas d'erreurs.

**Tableau 5**

| **Nom** | **Polynucléotide d'AMH** | **Fragment d'ADN correspondant SEQ ID N°X-8HIS-codon stop** |
|---|---|---|
| AMH-1 | SEQ ID N°86 | SEQ ID N°86-CACCACCATCATCACCATCACCAC-TGA |
| AMH-2 | SEQ ID N°87 | SEQ ID N°87-CACCACCATCATCACCATCACCAC-TGA |
| AMH-3 | SEQ ID N°88 | SEQ ID N°88-CACCACCATCATCACCATCACCAC-TGA |
| AMH entière | SEQ ID N°89 | SEQ ID N°89-CACCACCATCATCACCATCACCAC-TGA |

### 1.2. Transfection transitoire en cellules HEK293T

*Culture*. Les cellules HEK-293T/17 SF (ATCC ACS-4500™) ont été cultivées sans sérum dans du milieu HEK Plus SFM (ATCC # 8006386597) enrichi avec de la glutamine (GIBCO # 250030.24), selon les instructions du fournisseur. Les cellules ont été mises en culture dans des flasques de culture F75 et maintenues dans une étuve à 37°C avec 5% de CO2 avant transfection.

*Transfection.* Les cellules HEK293T SF (10⁶ cellules) ont été transfectées par nucléofection à l'aide de l'appareil Amaxa nucleofector (Lonza), en appliquant le protocole fourni avec la trousse Amaxa cell line nucleofector kit V (#VCA-1003) et en utilisant 5 µg d'ADN pour 1 million de cellules par transfection. Brièvement, 1 million de cellules sont centrifugées à 200 g pendant 10 minutes afin de les récolter. Le culot cellulaire est ensuite resuspendu dans 100 µl de solution V (fournie dans le kit). 5 µg de plasmide est ajouté à la suspension cellulaire. Le tout est doucement mélangé et transféré dans une cuvette Amaxa (aussi fournie dans le kit). La cuvette est insérée dans l'appareil nucleofector réglé au programme AMAXA Q-001 et la nucléofection est alors activée. L'échantillon est ensuite immédiatement transféré à un milieu tiède dans une plaque de culture cellulaire de 6 puits qui sera incubée à 37°C, 5% CO₂. Les cellules HEK293T SF sont cultivées pendant 48 heures.

*Lyse et récolte.* 48 heures post-transfection les surnageants sont collectés puis congelés à -80°C après avoir ajouté des inhibiteurs de protéases (cOmplete™, EDTA-free protease inhibitor cocktail tablets de Roche). Les culots cellulaires transfectés (6 X 10⁶ cellules/culot) sont repris par 1,8 mL de tampon de lyse phosphate 5,5 mM, NaCl 130 mM, Triton X-100 0,5%, Benzonase Nuclease 5U/mL (Novagen), MgCl₂ 0,48 g/L et inhibiteurs de protéases (cOmplete™, EDTA-free protease inhibitor cocktail tablets, Roche Cat. No. 045-6642, 1 pastille/50 mL, pH 7,4). Le lysat cellulaire est ensuite placé dans la glace 30 minutes, puis centrifugé 15 minutes à 13000 g, 4°C. Les surnageants obtenus contiennent les protéines AMH-1, AMH-2, AMH-3 et AMH entière, et sont stockés à -80°C.

### 1.3. Analyse de l'expression des protéines par Western-blot

Une première caractérisation des produits d'expression obtenus lors de l'étape 1.2. a été réalisée par analyse SDS-PAGE sur un gel NuPAGE® Bis-Tris 4-12% en tampon NuPAGE® MES SDS (Life Technologies). Avant chargement sur le gel (14 µL/puits), les lysats et surnageants de transfection ont été dilués dans le tampon NuPAGE® LDS Sample Buffer 4X (Life Technologies) (3/1, volume/volume) et ont subi différents traitements. La réduction se fait par ajout de 55 mM final de dithiothreitol (DTT). Le chauffage est de 5 min à 75°C.
Condition A : CHAUFFEE et REDUITE (avec DTT)
Condition B : CHAUFFEE et NON REDUITE (sans DTT)

Après la migration du gel, le transfert des protéines séparées par électrophorèse est réalisé sur une membrane de nitrocellulose 0.45 µm à 350 mA constant pendant 50 minutes dans un tampon Tris-glycine 1X contenant 20% de méthanol. La passivation de la membrane est réalisée en présence de BSA 3% (albumine sérique bovine) dans du tampon phosphate 5,5 mM, NaCl 130 mM pendant une nuit à +2/8°C. Après passivation, un anticorps monoclonal de souris anti-histidines (Qiagen, Cat. No. 34660) est dilué en au 1/2000 dans du tampon phosphate 5,5 mM, NaCl 130 mM contenant 0,05% de Tween 20, puis 10 mL de cette dilution sont incubés avec la membrane pendant 1h à +18/25°C. Une seconde membrane préparée exactement de la même manière est incubée au même moment et dans les mêmes conditions avec un anticorps monoclonal de souris anti-β-actine (clone AC-15, Life Technologies, Cat. No. AM4302) à la place de l'anticorps anti-histidines. Cette membrane sert à vérifier que dans chaque puits, des quantités de protéine totale comparables ont été soumises à analyse (témoin de charge équivalente).

Après rinçage des membranes afin d'enlever les anticorps non liés (5 lavages de 5 min en tampon phosphate 5,5 mM, NaCl 130 mM, Tween 20 0,05%), elles sont incubées pendant 1h avec un anticorps secondaire anti-souris conjugué à la peroxydase de raifort (Jackson Immunoresearch, Cat. No. 115-036-003) dilué au 1/20 000 en PBS 1X, Tween 20 0,2%. Après 5 lavages de 5 minutes en PBS 1X, Tween 20 0,2%, la révélation est réalisée en incubant les membranes dans une solution Clarity Western-blot Substrate (Biorad, Cat. No. 170-5061), 5 minutes sous agitation avant une acquisition en chimiluminescence (Chemidoc XRS, Biorad).

Les résultats sont présentés en Figure 2. Sur la membrane révélée avec l'anticorps anti-β-actine (Figure 2A), on observe une seule bande par puits et les intensités de ces bandes sont équivalentes, à l'exception des puits AMH-1 (3 et 4), légèrement moins intenses. Ce témoin est donc validé. Sur la membrane révélée avec l'anticorps anti-histidines (Figure 2B), on observe des bandes allumées dans les puits contrôle négatif. Or, ces derniers contiennent un lysat des cellules transfectées à blanc, sans plasmide. Il s'agit d'une réactivité non spécifique de l'anticorps anti-histidines avec certaines protéines du lysat qui se retrouvent dans l'ensemble des puits. En plus de ces bandes non-spécifiques, les puits AMH-1, AMH-2, AMH-3 et AMH entière contiennent des bandes spécifiques, c'est-à-dire non présentes dans les puits contrôle négatif. Ces bandes spécifiques sont encadrées sur la figure 2B. Leurs masses moléculaires apparentes, pour la condition chauffée et réduite, sont d'environ 20, 30, 55 et 65 kDa respectivement pour les constructions AMH-1, AMH-2, AMH-3 et AMH entière (puits 3, 6, 8, 10). Les masses moléculaires apparentes pour la condition chauffée mais pas réduite, sont d'environ 36, 50, 100 et 120 kDa respectivement, pour les constructions AMH-1, AMH-2, AMH-3 et AMH entière (puits 4, 7, 9, 11). Ces estimations de masses moléculaires sont compatibles avec une dimérisation des constructions AMH en condition non réduite. Il est important de noter que la dimérisation a également lieu en l'absence de la région mature et malgré les importantes troncations C-terminales aléatoires effectuées dans les constructions AMH-1 et AMH-2.

En conclusion, cette analyse en Western blot montre que les constructions plasmidiques AMH-1, AMH-2, AMH-3 et AMH entière permettent bien d'exprimer des protéines contenant un tag histidines et dont les masses moléculaires apparentes correspondent aux masses moléculaires attendues de par les séquences nucléotidiques.

### Exemple 2 : Expression stable la protéine AMH en cellules CHO et purification

L'obtention d'un clone stable de cellules CHO (Chinese Hamster Ovary) recombinantes exprimant l'AMH entière a été réalisée en utilisant le kit cGPS CHO-Sa CEMAX de Cellectis (Cat. No. CHOSa-0011-05 et CHOSa-0011-10) et en suivant le protocole associé. Ce kit permet l'intégration intra-chromosomique ciblée d'un gène exogène en cellule CHO. Il est composé de la lignée cellulaire cGPS CHO-Sa (cellules adhérentes), un vecteur d'intégration dans lequel le gène d'intérêt est cloné, un vecteur exprimant de manière constitutive la méganuclease I-Sce I et le kit «TransMessenger™ Transfection Reagent» (Qiagen Cat. No. 301525). La lignée cellulaire cGPS CHO-Sa génétiquement modifiée a la particularité de contenir dans son génome un site particulier, de grande taille et unique, reconnu par la méganucléase I-Sce I qui a une activité endonucléasique.

### 2.1. Clonage

Un gène synthétique codant pour la protéine AMH humaine entière (aa 1-560) associée à un tag de 8 histidines en C-terminal a été commandé auprès de la société Geneart. Le gène a été optimisé pour l'expression chez l'hôte CHO. Ce gène AMH a été cloné dans le vecteur d'intégration pIM.LP2.Zeo commercialisé par Cellectis entre les sites Eco R1 et Not 1 sous le contrôle du promoteur CMV. Le vecteur d'intégration pIM.LP2.Zeo a la particularité de contenir 2 régions, homologues respectivement à la région en amont et en aval du site unique reconnu par la méganucléase I-Sce I dans le génome CHO. Ces 2 régions particulières encadrent le site de multi-insertion dans lequel le gène codant pour l'AMH a été inséré. De plus, ce plasmide d'intégration apporte 2 avantages sélectifs aux cellules CHO transfectées. Le premier est le gène de résistance à la zéocine, contrôlé par le promoteur CMV. Le second est le gène de résistance à la néomycine contrôlé par le promoteur SV40. Ce gène permet aussi la résistance à la généticine (G418), antibiotique proche de la néomycine.

### 2.2. Transfection

Un jour avant la transfection (J-1), 2 x 10⁵ cellules adhérentes cGPS CHO-Sa CEMAX sont ensemencées par boîte de Pétri 10 cm dans un milieu F-12K supplémenté avec 2 mM L-glutamine, pénicilline (100 UI/ml), streptomycine (100 µg/ml), amphotéricine B (Fongizone) (0,25 µg/ml) et 10% de sérum de veau foetal.

Le jour de la transfection (J), 1 µg du vecteur d'intégration (pIM.LP2.Zeo) contenant le gène AMH et 1 µg de mRNA méganuclease sont dilués dans le tampon EC-R (disponible dans le kit « TransMessenger™ Transfection reagent »). 4 µl du réactif Enhancer sont ensuite ajoutés [ratio acide nucléique (mg) / Enhancer (ml) = ½]. Le volume total de réaction doit être de 100 µl. La solution est alors incubée 5 minutes à température ambiante. Ensuite, 16 µl du réactif TransMessenger™ sont ajoutés puis l'ensemble est incubé 10 minutes à +18/25°C avant d'être déposé sur les 2 x 10⁵ cellules dont le milieu de culture a été préalablement remplacé par 900 µl de milieu F-12k sans sérum et sans antibiotique.

### 2.3 Sélection clonale et caractérisation de clones

Les clones recombinants AMH ont été sélectionnés selon leurs résistances à la zéocine et à la généticine (G418).

24h après la transfection (J+1), le milieu de culture est remplacé par 10 ml de milieu complet supplémenté avec 0,6 mg/ml de G418 puis à partir du 6^{ème} jour (J+6), le milieu de culture est régulièrement remplacé par du milieu frais supplémenté avec 0,6 mg/ml de G418 et 0,4 mg/ml de zéocine. 15 jours après la transfection (J+15), les cellules sont isolées par dilution limite en plaque de 96 puits. Après ce clonage cellulaire, les clones ont été amplifiés par culture puis testés :
- par PCR à partir de l'ADN génomique extrait des cellules CHO en utilisant des amorces spécifiques des extrémités 5' et 3' du gène AMH,
- par Western blot pour vérifier l'expression de la protéine d'AMH.

Parmi les clones positifs à la fois en PCR et en Western blot, le clone AMH5 3F1 a été sélectionné. Pour ce clone, le produit de PCR obtenu par amplification du gène AMH a été séquencé dans sa totalité afin de vérifier l'intégrité du gène et s'assurer qu'il n'y a pas eu de mutation introduite lors de la sélection.

### 2.4. Expression de l'AMH recombinante à partir du clone stable CHO-AMH5 3F1

Le clone AMH5 3F1 est cultivé en boite de culture de 225 cm² à raison de 9 x 10⁶ cellules dans 60 ml de milieu de culture Excell 302 (Sigma Cat. No. 4324C) complémenté avec des antibiotiques et antifongiques 1% v/v (Gibco Cat. No. 15240), L-glutamine 12 mM (Gibco Cat. No. 25030), glucose 6 g/L (Sigma Cat. No. G8769), citrate de fer 0,4 mM (Sigma Cat. No. F6129), hypoxanthine-thymidine 2% (Gibco Cat. No. 41065), glycérol 1%, pepstatine A 1 mg/L (Sigma Cat. No. P4265). Les boites de culture sont incubées en étuve à 37°C sous atmosphère de 7,5% CO₂. Après 4 jours de culture (amplification), la suspension cellulaire obtenue (environ 66 x 10⁶ cellules) est réensemencée dans 7 boites de culture F225 selon le même protocole. Les surnageants de ces cultures sont récoltées, puis centrifugés à 5000 g et congelés à -25°C jusqu'à purification.

### 2.5. Purification de la protéine AMH recombinante CHO-AMH5 3F1

Les surnageants de culture précédemment récoltés sont décongelés et poolés. Un volume de 5 litres de surnageant est alors filtré sur membrane 0,8 µm (Nalgène, VWR Cat. No. 7345084) puis sur membrane 0,22 µm (Nalgène aPES Rapid Flow). L'échantillon est ensuite concentré 20 fois sur fibre creuse ayant un seuil de coupure de 30 kDa (GE Cat. No. 564110-18). Le retentât d'environ 100 ml est ensuite diafiltré contre 5 fois son volume par un tampon phosphate de potassium et de sodium 50 mM pH7,8, NaCl 100 mM, EDTA 1 mM, azide 0,9 g/L. Deux pastilles d'inhibiteurs de protéases Complete EDTA free (Roche Cat. No. 11 873 580 001) sont ajoutés. Ce retentât est purifié par chromatographie d'affinité sur colonne Hi-trap NHS Sépharose couplé à un anticorps anti-AMH (polyclonal ou monoclonal) à raison de 10 mg d'anticorps par ml de gel selon le protocole fournisseur (GE Cat. No. 17-0716-01). La colonne d'affinité est équilibrée en tampon de diafiltration, puis 50 ml de retentât sont injectés sur la colonne à une vitesse de 0,5 ml/min. On effectue ensuite un lavage en tampon Tris HCl 50 mM pH 7,9, NaCl 100 mM, EDTA ImM. L'élution de la protéine AMH est ensuite réalisée par un tampon Glycine-HCl 0,1 M pH 2,9 à une vitesse de 0,5 ml/min. Les fractions d'élution collectées sont immédiatement neutralisées à pH 7-8, additionnées d'inhibiteurs de protéases, poolées et conservées à -80°C dans un tampon de stockage NaHCO₃ 0,2M, NaCl 0,5M pH 7,5 et éthanol 20%. La figure 4 donne la photographie de la membrane suite à une analyse en SDS-PAGE de la protéine AMH recombinante CHO-AMH5 3F1 selon le protocole de l'exemple 1.3. A la place du Western blot, une coloration au nitrate d'argent des protéines totales a été effectuée. La protéine purifiée est très pure.

### Exemple 3 : Préparation d'anticorps anti-AMH de l'invention par immunisation de souris

### 3.1. Les immunogènes

Les plasmides AMH entière et AMH-3 obtenus dans l'exemple 1 ont été amplifiés par culture dans les bactéries *E. coli,* puis purifiés en utilisant le kit EndoFree Plasmid Mega Kit de Qiagen (Cat. No. 12381) ou kit équivalent. Pour la préparation des cartouches Gene Gun (Bio-Rad), 2 µg d'ADN plasmidique ont été précipités sur 0,22 mg de billes d'or de 1 µm de diamètre en présence de CaCl₂ et de spermidine, selon les instructions du fabricant. Les billes ainsi préparées peuvent être conservées à +2/8°C dans le noir, en présence d'un absorbeur d'humidité (sachet déssicant).

La protéine AMH recombinante CHO-AMH5 3F1 obtenue dans l'exemple 2 a été mélangée volume pour volume avec l'adjuvant de Freund (Sigma), préparé sous forme d'émulsion eau-dans-huile et dont il est connu qu'il présente un bon pouvoir adjuvant. Cette préparation a été effectuée extemporanément avant chaque injection.

### 3.2. Les immunisations

Les expériences d'immunisations ont été réalisées chez des souris BALB/c (H-2^{d}) femelles âgées de six à huit semaines au moment de la première immunisation. Différents protocoles sont mis en oeuvre :
- 4 doses de 4 µg par injection d'ADN AMH entière à 0, 2, 4 et 6 semaines,
- 4 doses de 4 µg par injection d'ADN AMH-2 à 0, 2, 4 et 6 semaines,
- 4 doses de 4 µg par injection d'ADN AMH-3 à 0, 2, 4 et 6 semaines,
- 3 doses de 10 µg par injection de protéine AMH à 0, 2, et 4 semaines.

Pour les immunisations ADN, les souris ont été rasées au niveau de l'abdomen. Le système Helios Gene Gun Delivery System (Bio-Rad) a été utilisé à une pression de 2750 kPa pour injecter les billes d'or enrobées d'ADN dans la peau des souris. Pour les immunisations protéiques, l'injection a été réalisée par voie sous-cutanée.

Afin de suivre l'apparition des anticorps, on effectue régulièrement sur les souris des prélèvements de sang. La présence des anticorps anti-AMH dans ces sérums est testée en réalisant un ELISA en microplaque 96 puits. La protéine AMH recombinante CHO-AMH5 3F1 est utilisée en capture (1 µg/puits) ; après saturation on fait réagir avec cet antigène différentes dilutions de sérums à tester (incubation à 37°C, pendant 1h). Les anticorps anti-AMH présents dans le sérum sont révélés par un anticorps de chèvre anti-IgG de souris AffiniPure conjugué à la phosphatase alcaline (H+L, Jacskon Immunoresearch, Cat no. 115-055-146), qui se lie aux anticorps recherchés (0,1 µg/puits). On identifie ainsi parmi les souris immunisées, celles qui ont développé des anticorps anti-AMH.

Entre 50 à 70 jours après les premières injections, les souris ayant développé une réponse humorale anti-AMH ont été restimulées par une injection intraveineuse de 100 µg de protéine AMH.

### 3.3. Préparation des hybridomes

Trois jours après cette dernière injection, les souris répondantes ont été sacrifiées ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par Kohler et Milstein (Kohler et Milstein 1975, Kohler et al., 1976). Après une période de culture de 12-14 jours les surnageants d'hybridomes obtenus ont été criblés pour déterminer la présence d'anticorps anti-AMH en utilisant le test ELISA décrit dans le paragraphe précédent.

### 3.4. Sélection des surnageants d'hybridomes reconnaissant la région 157-255 ou la région 256-451 de la protéine AMH humaine

Un anticops anti-6 histidines de lapin (Sigma Aldrich, Cat. No. SAB4301134 ou équivalent) a été dilué en PBS 1X et adsorbé sur une plaque de microtitration 96 puits à raison de 1 µg/puits, par incubation sur la nuit à +18/25°C. La plaque est ensuite lavée 3 fois dans un tampon PBS-Tween 20 0,05% (PBS-T) puis passivée par incubation dans un tampon PBS-T contenant 10 g/L de BSA et à nouveau lavée 3 fois en tampon PBS-T.

Les lysats obtenus dans l'exemple 1 et conservés à -80°C sont décongelés et dilués entre 1/2 et 1/10 en tampon PBS 1X. 100 µl de chacun des lysats (contrôle négatif, AMH-1, AMH-2, AMH-3 et AMH entière) sont distribués dans plusieurs puits de la plaque et incubés 2h à 37°C. Ensuite, la plaque est vidée et lavée 4 fois en tampon PBS-T contenant 300 mM de NaCl. Les surnageants d'hybridomes à tester (100 µl) sont ensuite ajoutés sur chacun des 5 différents lysats et incubés 1h à 37°C. Après une nouvelle étape de 4 lavages en PBS-T NaCl 300 mM, on ajoute l'anticorps secondaire qui est un anti-IgG de souris AffiniPure fait chez la chèvre, conjugué à la peroxidase (H+L, Jacskon Immunoresearch, Cat no. 115-035-166). Après une nouvelle étape de 3 lavages en PBS-T NaCl 300 mM, la réaction est révélée en incubant la plaque 10 min à +18/25°C en présence du substrat SureBlue™ TMB Microwell Peroxidase (KLP, Cat. No. 52-00-01). La lecture de la plaque est faite par mesure de la DO à 450 et 630 nm.

*Sélection des surnageants d'hybridomes reconnaissent la région 157-255.* Les hybridomes sélectionnés à partir des résultats de l'ELISA sont ceux dont les surnageants contiennent des anticorps qui reconnaissent le lysat AMH-2 et ne reconnaissent pas le lysat AMH-1. Bien entendu, les lysats AMH-3 et AMH entière sont également reconnus par les surnageants des hybridomes choisis. Les hybridomes 5G5 et 1B11 ont ainsi été sélectionnés.

*Sélection des surnageants d'hybridomes reconnaissent la régions 256-451.* Les hybridomes sélectionnés à partir des résultats de l'ELISA sont ceux dont les surnageants contiennent des anticorps qui reconnaissent le lysat AMH-3 et ne reconnaissent pas le lysat AMH-2, ni le lysat AMH-1. Bien entendu, le lysat AMH entière est également reconnu par les surnageants des hybridomes choisis. Les hybridomes 3H8, 4C7 et 4G10 ont ainsi été sélectionnés.

Après la sélection, les hybridomes choisis ont été clonés selon la technique de la dilution limite, bien connue par l'homme du métier, afin d'assurer la clonalité. On a ainsi pu obtenir les hybridomes monoclonaux sécrétant les anticorps anti-AMH suivants :
- 5G5A10 et 1B11B1 qui reconnaissant la région 157-255 de l'AMH humaine,
- 3H8E2, 4C7E12 et 4G10E12 qui reconnaissant la région 256-451 de l'AMH humaine.

La production à grande échelle des anticorps monoclonaux a été réalisée par culture des hybridomes dans le bioréacteur miniPERM™, selon un protocole dérivé de la publication de Falkenberg (1998). Les anticorps monoclonaux ont ensuite été purifiés à partir du surnageant de culture par chromatographie d'affinité sur protéine A.

### Exemple 4 : Sélection des anticorps monoclonaux anti-AMH reconnaissant les épitopes non-linéaires

Parmi les anticorps monoclonaux obtenus dans l'exemple 3, nous avons déterminé ceux qui étaient dirigés contre des épitopes linéaires afin de sélectionner ceux reconnaissant des épitopes non-linéaires. Pour ce faire, 80 peptides synthétiques couvrant l'ensemble de la séquence en acides aminés de l'AMH humaine ont été synthétisés. La fixation des anticorps à chacun de ces peptides a été testée en ELISA.

### 4.1. Synthèse peptidique

80 peptides de 16 acides aminés ont été synthétisés. Parmi les 16 acides aminés, 12 correspondent à la séquence de l'AMH et se chevauchent de 5 acides aminés recouvrant ainsi l'ensemble de la séquence de l'AMH humaine (acides aminés 1-560). A l'extrémité N-terminale, une biotine puis une séquence SGSG (bras espaceur) ont été ajoutées, afin de faciliter l'analyse de ces peptides en ELISA. Ces peptides sont non purifiés et en solution (eau/acétonitrile). Ils ont été vérifiés par spectrométrie de masse LC/MS. Les séquences synthétisées sont présentées dans le Tableau 6.

**Tableau 6. Séquences des peptides AMH humaine.**

| **n° peptide** | **Peptide d'AMH (SEQ ID N)** | **Positions** | **Séquence totale** |
|---|---|---|---|
| 1 | MRDLPLTSLALV (SEQ ID N°90) | 1-12 | Bio-SGSG-MRDLPLTSLALV-amide |
| 2 | SLALVLSALGAL (SEQ ID N°91) | 8-19 | Bio-SGSG-SLALVLSALGAL-amide |
| 3 | ALGALLGTEALR (SEQ ID N°92) | 15-26 | Bio-SGSG-ALGALLGTEALR-amide |
| 4 | TEALRAEEPAVG (SEQ ID N°93) | 22-33 | Bio-SGSG-TEALRAEEPAVG-amide |
| 5 | EPAVGTSGLIFR (SEQ ID N°94) | 29-40 | Bio-SGSG-EPAVGTSGLIFR-amide |
| 6 | GLIFREDLDWPP (SEQ ID N°95) | 36-47 | Bio-SGSG-GLIFREDLDWPP-amide |
| 7 | LDWPPGSPQEPL (SEQ ID N°96) | 43-54 | Bio-SGSG-LDWPPGSPQEPL-amide |
| 8 | PQEPLCLVALGG (SEQ ID N°97) | 50-61 | Bio-SGSG-PQEPLCLVALGG-amide |
| 9 | VALGGDSNGSSS (SEQ ID N°98) | 57-68 | Bio-SGSG-VALGGDSNGSSS-amide |
| 10 | NGSSSPLRVVGA (SEQ ID N°99) | 64-75 | Bio-SGSG-NGSSSPLRVVGA-amide |
| 11 | RVVGALSAYEQA (SEQ ID N°100) | 71-82 | Bio-SGSG-RVVGALSAYEQA-amide |
| 12 | AYEQAFLGAVQR (SEQ ID N°101) | 78-89 | Bio-SGSG-AYEQAFLGAVQR-amide |
| 13 | GAVQRARWGPRD (SEQ ID N°102) | 85-96 | Bio-SGSG-GAVQRARWGPRD-amide |
| 14 | WGPRDLATFGVC (SEQ ID N°103) | 92-103 | Bio-SGSG-WGPRDLATFGVC-amide |
| 15 | TFGVCNTGDRQA (SEQ ID N°104) | 99-110 | Bio-SGSG-TFGVCNTGDRQA-amide |
| 16 | GDRQAALPSLRR (SEQ ID N°105) | 106-117 | Bio-SGSG-GDRQAALPSLRR-amide |
| 17 | PSLRRLGAWLRD (SEQ ID N°106) | 113-124 | Bio-SGSG-PSLRRLGAWLRD-amide |
| 18 | AWLRDPGGQRLV (SEQ ID N°107) | 120-131 | Bio-SGSG-AWLRDPGGQRLV-amide |
| 19 | GQRLVVLHLEEV (SEQ ID N°108) | 127-138 | Bio-SGSG-GQRLVVLHLEEV-amide |
| 20 | HLEEVTWEPTPS (SEQ ID N°109) | 134-145 | Bio-SGSG-HLEEVTWEPTPS-amide |
| 21 | EPTPSLRFQEPP (SEQ ID N°110) | 141-152 | Bio-SGSG-EPTPSLRFQEPP-amide |
| 22 | FQEPPPGGAGPP (SEQ ID N°111) | 148-159 | Bio-SGSG-FQEPPPGGAGPP-amide |
| 23 | GAGPPELALLVL (SEQ ID N°112) | 155-166 | Bio-SGSG-GAGPPELALLVL-amide |
| 24 | ALLVLYPGPGPE (SEQ ID N°113) | 162-173 | Bio-SGSG-ALLVLYPGPGPE-amide |
| 25 | GPGPEVTVTRAG (SEQ ID N°114) | 169-180 | Bio-SGSG-GPGPEVTVTRAG-amide |
| 26 | VTRAGLPGAQSL (SEQ ID N°115) | 176-187 | Bio-SGSG-VTRAGLPGAQSL-amide |
| 27 | GAQSLCPSRDTR (SEQ ID N°116) | 183-194 | Bio-SGSG-GAQSLCPSRDTR-amide |
| 28 | SRDTRYLVLAVD (SEQ ID N°117) | 190-201 | Bio-SGSG-SRDTRYLVLAVD-amide |
| 29 | VLAVDRPAGAWR (SEQ ID N°45) | 197-208 | Bio-SGSG-VLAVDRPAGAWR-amide |
| 30 | AGAWRGSGLALT (SEQ ID N°118) | 204-215 | Bio-SGSG-AGAWRGSGLALT-amide |
| 31 | GLALTLQPRGED (SEQ ID N°119) | 211-222 | Bio-SGSG-GLALTLQPRGED-amide |
| 32 | PRGEDSRLSTAR (SEQ ID N°46) | 218-229 | Bio-SGSG-PRGEDSRLSTAR-amide |
| 33 | LSTARLQALLFG (SEQ ID N°120) | 225-236 | Bio-SGSG-LSTARLQALLFG-amide |
| 34 | ALLFGDDHRCFT (SEQ ID N°121) | 232-243 | Bio-SGSG-ALLFGDDHRCFT-amide |
| 35 | HRCFTRMTPALL (SEQ ID N°47) | 239-250 | Bio-SGSG-HRCFTRMTPALL-amide |
| 36 | TPALLLLPRSEP (SEQ ID N°122) | 246-257 | Bio-SGSG-TPALLLLPRSEP-amide |
| 37 | PRSEPAPLPAHG (SEQ ID N°123) | 253-264 | Bio-SGSG-PRSEPAPLPAHG-amide |
| 38 | LPAHGQLDTVPF (SEQ ID N°61) | 260-271 | Bio-SGSG-LPAHGQLDTVPF-amide |
| 39 | DTVPFPPPRPSA (SEQ ID N°124) | 267-278 | Bio-SGSG-DTVPFPPPRPSA-amide |
| 40 | PRPSAELEESPP (SEQ ID N°125) | 274-285 | Bio-SGSG-PRPSAELEESPP-amide |
| 41 | EESPPSADPFLE (SEQ ID N°126) | 281-292 | Bio-SGSG-EESPPSADPFLE-amide |
| 42 | DPFLETLTRLVR (SEQ ID N°127) | 288-299 | Bio-SGSG-DPFLETLTRLVR-amide |
| 43 | TRLVRALRVPPA (SEQ ID N°128) | 295-306 | Bio-SGSG-TRLVRALRVPPA-amide |
| 44 | RVPPARASAPRL (SEQ ID N°129) | 302-313 | Bio-SGSG-RVPPARASAPRL-amide |
| 45 | SAPRLALDPDAL (SEQ ID N°130) | 309-320 | Bio-SGSG-SAPRLALDPDAL-amide |
| 46 | DPDALAGFPQGL (SEQ ID N°131) | 316-327 | Bio-SGSG-DPDALAGFPQGL-amide |
| 47 | FPQGLVNLSDPA (SEQ ID N°132) | 323-334 | Bio-SGSG-FPQGLVNLSDPA-amide |
| 48 | LSDPAALERLLD (SEQ ID N°62) | 330-341 | Bio-SGSG-LSDPAALERLLD-amide |
| 49 | ERLLDGEEPLLL (SEQ ID N°63) | 337-348 | Bio-SGSG-ERLLDGEEPLLL-amide |
| 50 | EPLLLLLRPTAA (SEQ ID N°64) | 344-355 | Bio-SGSG-EPLLLLLRPTAA-amide |
| 51 | RPTAATTGDPAP (SEQ ID N°133) | 351-362 | Bio-SGSG-RPTAATTGDPAP-amide |
| 52 | GDPAPLHDPTSA (SEQ ID N°65) | 358-369 | Bio-SGSG-GDPAPLHDPTSA-amide |
| 53 | DPTSAPWATALA (SEQ ID N°66) | 365-376 | Bio-SGSG-DPTSAPWATALA-amide |
| 54 | ATALARRVAAEL (SEQ ID N°134) | 372-383 | Bio-SGSG-ATALARRVAAEL-amide |
| 55 | VAAELQAAAAEL (SEQ ID N°135) | 379-390 | Bio-SGSG-VAAELQAAAAEL-amide |
| 56 | AAAELRSLPGLP (SEQ ID N°136) | 386-397 | Bio-SGSG-AAAELRSLPGLP-amide |
| 57 | LPGLPPATAPLL (SEQ ID N°137) | 393-404 | Bio-SGSG-LPGLPPATAPLL-amide |
| 58 | TAPLLARLLALC (SEQ ID N°138) | 400-411 | Bio-SGSG-TAPLLARLLALC-amide |
| 59 | LLALCPGGPGGL (SEQ ID N°139) | 407-418 | Bio-SGSG-LLALCPGGPGGL-amide |
| 60 | GPGGLGDPLRAL (SEQ ID N°140) | 414-425 | Bio-SGSG-GPGGLGDPLRAL-amide |
| 61 | PLRALLLLKALQ (SEQ ID N°141) | 421-432 | Bio-SGSG-PLRALLLLKALQ-amide |
| 62 | LKALQGLRVEWR (SEQ ID N°67) | 428-439 | Bio-SGSG-LKALQGLRVEWR-amide |
| 63 | RVEWRGRDPRGP (SEQ ID N°68) | 435-446 | Bio-SGSG-RVEWRGRDPRGP-amide |
| 64 | DPRGPGRAQRSA (SEQ ID N°142) | 442-453 | Bio-SGSG-DPRGPGRAQRSA-amide |
| 65 | AQRSAGATAADG (SEQ ID N°143) | 449-460 | Bio-SGSG-AQRSAGATAADG-amide |
| 66 | TAADGPCALREL (SEQ ID N°144) | 456-467 | Bio-SGSG-TAADGPCALREL-amide |
| 67 | ALRELSVDLRAE (SEQ ID N°145) | 463-474 | Bio-SGSG-ALRELSVDLRAE-amide |
| 68 | DLRAERSVLIPE (SEQ ID N°146) | 470-481 | Bio-SGSG-DLRAERSVLIPE-amide |
| 69 | VLIPETYQANNC (SEQ ID N°147) | 477-488 | Bio-SGSG-VLIPETYQANNC-amide |
| 70 | QANNCQGVCGWP (SEQ ID N°148) | 484-495 | Bio-SGSG-QANNCQGVCGWP-amide |
| 71 | VCGWPQSDRNPR (SEQ ID N°149) | 491-502 | Bio-SGSG-VCGWPQSDRNPR-amide |
| 72 | DRNPRYGNHWL (SEQ ID N°150) | 498-509 | Bio-SGSG-DRNPRYGNHVVL-amide |
| 73 | NHVVLLLKMQVR (SEQ ID N°151) | 505-516 | Bio-SGSG-NHVVLLLKMQVR-amide |
| 74 | KMQVRGAALARP (SEQ ID N°152) | 512-523 | Bio-SGSG-KMQVRGAALARP-amide |
| 75 | ALARPPCCVPTA (SEQ ID N°153) | 519-530 | Bio-SGSG-ALARPPCCVPTA-amide |
| 76 | CVPTAYAGKLLI (SEQ ID N°154) | 526-537 | Bio-SGSG-CVPTAYAGKLLI-amide |
| 77 | GKLLISLSEERI (SEQ ID N°155) | 533-544 | Bio-SGSG-GKLLISLSEERI-amide |
| 78 | SEERISAHHVPN (SEQ ID N°156) | 540-551 | Bio-SGSG-SEERISAHHVPN-amide |
| 79 | HHVPNMVATECG (SEQ ID N°157) | 547-558 | Bio-SGSG-HHVPNMVATECG-amide |
| 80 | VPNMVATECGCR (SEQ ID N°158) | 549-560 | Bio-SGSG-VPNMVATECGCR-amide |

### 4.2. ELISA

Les microplaques 96 puits sont coatées en streptavidine (10 µg/ml, 1 µg/puits) dans un tampon PBS 1X pendant 1h à 37°C, puis passivées dans un tampon PBS-Tween 20 0,05% (PBS-T) contenant 10 g/l de BSA durant une nuit à température ambiante. La solution de passivation est eliminée, puis les peptides biotinylés sont distribués (10 µg/ml en tampon PBS 1X, 1 µg par puits) et incubés 1h à 37°C. Après 4 lavages en PBS-T, on ajoute l'anticorps monoclonal à tester à une concentration de 1 µg/ml. Après une incubation de 1h30 à 37°C et 4 lavages PBS-T, on ajoute un anticorps anti-IgG de souris conjugué à la peroxidase. La révélation est faite avec le substrat TMB avec mesure de la densité optique (DO) à 450 nm.

Afin de valider le format de l'ELISA un anticorps anti-AMH commercial a été utilisé en tant que témoin positif. Il s'agit du clone AA011 de la Société AnhsLab. Cet anticorps reconnaît spécifiquement le peptide 52 (SEQ ID N°137), le signal de DO obtenue est supérieure à 1. Parmi les 5 anticorps monoclonaux obtenus dans l'exemple 3, le clone 4C7E12 reconnaît également le peptide 52. Le clone 4C7E12 reconnaît donc le même épitope que le clone AA011. Il n'est pas retenu. Les anticorps monoclonaux 5G5A10, 1B11B1, 3H8E2 et 4G10E12 ne reconnaissent aucun des 80 peptides AMH testés. Leurs épitopes ne sont donc pas linéaires. C'est ces 4 anticorps non-linéaires 5G5A10, 1B11B1, 3H8E2 et 4G10E12 qui ont été sélectionnés pour mettre au point une méthode de quantification de la protéine AMH dans les échantillons biologiques.

### Exemple 5 : Caractérisation des anticorps anti-AMH 5G5A10, 1B11B1, 3H8E2 et 4G10E12

### 5.1. Comparaison de la capacité de capture des anticorps anti-AMH 5G5A10, 1B11B1, 3H8E2 et 4G10E12 en ELISA

Les anticorps de capture (5G5A10, 1B11B1, 3H8E2 et 4G10E12) dilués à 5 µg/ml en Tris 200 mM pH6,2 sont distribués en plaque de microtitration 96 puits à raison de 0,5 µg/puits et incubés 1h30 à 37°C. La plaque est ensuite lavée 3 fois dans un tampon PBS-Tween 20 0,05% (PBS-T) puis passivée 2h par incubation dans un tampon PBS-T contenant 10 g/L de BSA et à nouveau lavée 3 fois en tampon PBS-T.

Les lysats obtenus dans l'exemple 1 et conservés à -80°C sont décongelés, dilués au 1/20^{ème} en PBS 1X. 100 µl de chacun des lysats (contrôle négatif, AMH-1, AMH-2, AMH-3 et AMH entière) sont distribués et incubés toute la nuit à 37°C. Ensuite, la plaque est vidée et lavée 4 fois en tampon PBS-T contenant 300 mM de NaCl. Un anticorps anti-histidines biotinylé (Qiagen Cat. No. 34440) dilué au 1/1000^{ème} en tampon de passivation, est distribué puis incubé 1h30 à 37°C. La plaque est à nouveau lavée 3 fois, avant incubation avec le conjugué streptavidine-peroxidase (Jackson Immunoresearch Cat. No. 016-030-034) dilué au 1/2000^{ème} en tampon de passivation pendant 1h30 à 37°C. Après une nouvelle étape de 3 lavages en PBS-T la réaction est révélée en incubant la plaque 10 min à +18/25°C en présence du substrat SureBlue™ TMB Microwell Peroxidase (KLP, Cat. No. 52-00-01). La lecture de la plaque est faite par mesure de la DO à 450 et 630 nm.

Les résultats obtenus sont présentés dans le Tableau 7. Les valeurs de DO mesurées pour les témoins négatifs PBS et lysat transfecté sans plasmide sont au maximum de 0,28, ce qui correspond au signal non spécifique.

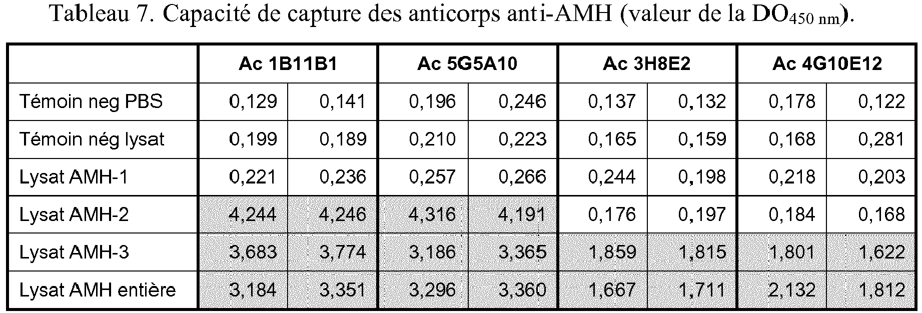

Les résultats dans le Tableau 7 montrent que les anticorps 1B11B1 et 5G5A10 capturent les lysats AMH-2, AMH-3 et AMH entière (DO > 3). Les anticorps 3H8E2 et 4G10E12 capturent les lysats AMH-3 et AMH entière (DO > 1,6). Le lysat AMH-1 (acides aminés 1-156) n'est capturé par aucun des anticorps monoclonaux testés.

De plus, les anticorps 1B11B1 et 5G5A10 qui reconnaissent la région 157-255 de la protéine AMH humaine ont des capacités de capture supérieures à celles des anticorps 3H8E2 et 4G10E12 reconnaissant la région 256-451. En effet, en cas de reconnaissance (valeurs grisées dans le Tableau 2), pour un lysat donné, les valeurs de DO obtenues avec les anticorps 1B11B1 et 5G5A10 sont supérieures à celles obtenues par les anticorps 3H8E2 et 4G10E12. Il sera donc judicieux d'utiliser préférentiellement en capture l'anticorps 1B11B1 ou l'anticorps 5G5A10.

### 5.2. Caractérisation en Western blot

Afin de mieux caractériser les réactivités des anticorps monoclonaux anti-AMH, des analyses en Western blot ont été réalisées.

*Purification des protéines.* Des transfections ont été réalisées selon le protocole de l'exemple 1 avec les plasmides codant pour les constructions AMH-2 et AMH-3, ainsi qu'un témoin négatif (sans plasmide). Les surnageants de culture sont récupérés et les protéines AMH qui s'y trouvent purifiées par chromatographie d'affinité métal-chélate en batch, grâce à leurs tags poly-histidines. Pour ce faire, les surnageants de transfection sont incubés une nuit à +2/8°C sous agitation avec une résine Ni-NTA (Roche, Cat. No. 115-26-70) équilibrée en tampon phosphate 11 mM, NaCl 260 mM, pH 7,4. La résine est ensuite récupérée par centrifugation 3 min à 3000 g et le surnageant contenant le matériel non adsorbé est éliminé. Après 3 lavages avec le tampon d'équilibration, la protéine est éluée par un tampon phosphate 5,5 mM, NaCl 130 mM, pH 7,4 contenant 500 mM d'imidazole, des inhibiteurs de protéases et dont le pH a été ajusté à 7.6. L'élution est effectuée par incubation à +2/8°C sous agitation, pendant une nuit. Le surnageant contenant l'AMH est alors récolté après centrifugation de la résine 3 minutes à 3000 g. Cette étape de purification est nécessaire pour concentrer les protéines AMH et surtout éliminer les grandes quantités de BSA présentes dans les surnageants, qui perturbent la migration des gels de SDS-PAGE.

*Western blot.* Cette analyse a été réalisée selon le protocole décrit dans l'exemple 1, paragraphe 1.3, soit en utilisant l'anticorps anti-histidines, soit en le remplaçant par un des anticorps anti-AMH (1 µg par membrane).

*Résultats.* Les résultats de cette analyse sont présentés en Figure 3. Les échantillons ont été analysés après chauffage mais sans réduction. Sur la membrane révélée par l'anticorps anti-histidines (Figure 3A) sont mises en évidence les deux protéines purifiées AMH-2 et AMH-3 et aucune réactivité n'est observée dans le puits contrôle négatif, comme attendu. Parmi les anticorps anti-AMH testés, tous reconnaissent la protéine AMH-3 mais c'est uniquement le clone 1B11B1 qui reconnaît aussi la protéine AMH-2, avec une réactivité bien moindre que pour AMH-3. Pour les clones 4G10E12 et 3H8E2, ce résultat est en accord avec les résultats obtenus en ELISA. En effet la zone de fixation de ces anticorps est dans la région 256-451 d'AMH qui n'est pas présente dans la construction AMH-2. En ELISA, les clones 1B11B1 et 5C5A10 présentent des réactivités similaires et reconnaissent bien AMH-2 et AMH-3. En Western blot l'anticorps 1B11B10 donne un signal intense avec AMH-3, le signal obtenu avec AMH-2 est beaucoup moins intense alors que la protéine est présente à des quantités comparables comme on peut le voir sur la membrane révélée avec l'anticorps anti-His. On peut en conclure que le Western blot, qui est une technique partiellement dénaturante, n'est pas adapté à l'étude de l'antigénicité de la protéine AMH-2. L'anticorps 5G5A10 qui donne une très bonne réactivité en ELISA, ne donne pas ou peu de signal en Western blot. Etant donnée sa faible fixation sur AMH-3 dans les conditions de l'expérience, il est normal de ne pas observer de réactivité avec l'AMH-2. Globalement cette expérience montre que le Western blot n'est pas adapté à l'étude de la reconnaissance des anticorps non-linéaires qui sont sensibles à la dénaturation par la chaleur comme le 5G5A10 ou le 1B11B1 dans une certaine mesure. Il convient donc d'interpréter avec beaucoup de précautions les analyses menées en Western blot sur les anticorps anti-AMH dans la littérature et privilégier si possible des analyses en ELISA plutôt qu'en Western blot.

### Exemple 6 : Détection de l'AMH par un immunoessai sandwich

Il a été observé sur des kits commerciaux d'AMH un écart significatif entre les concentrations obtenues pour un échantillon en fonction de l'heure du dosage après le prélèvement. Ce genre de variabilité est inacceptable en pratique clinique, il est donc primordial de disposer d'une trousse robuste, pour laquelle la concentration d'AMH mesurée est juste et reproductible et qui permette le stockage des échantillons à +2/8°C ou à -19/-31°C avant dosage. Un tel dosage a été réalisé en choisissant un anticorps dirigé contre les acides aminés 157-255 de l'AMH humaine.

### 6.1. Mode opératoire de l'immunoessai automatisé

La détection de l'AMH dans les échantillons biologiques a été réalisée par immunoessai sandwich en une étape en utilisant l'automate d'immunoanalyse VIDAS® (bioMérieux). Le cône à usage unique sert à la fois de phase solide pour la réaction et de système de pipetage. La cartouche de l'automate est composée de 10 puits (X0 à X9) recouverts d'une feuille d'aluminium scellée et étiquetée. Le premier puits (X0) comporte une partie prédécoupée pour faciliter l'introduction de l'échantillon. Le dernier puits (X9) est une cuvette optique dans laquelle la fluorescence du substrat est mesurée. Les différents réactifs nécessaires à l'analyse sont contenus dans les puits intermédiaires. Toutes les étapes du test sont ainsi réalisées automatiquement par l'instrument. Elles sont constituées d'une succession de cycles d'aspiration/refoulement du milieu réactionnel.

*Sensibilisation et passivation des cônes.* Les cônes ont été sensibilisés avec 270 µL d'une solution d'anticorps monoclonal 5G5A10 pour le couple C1 ou d'anticorps monoclonal 8C5B10H5 pour le couple C2, chacun à 7 µg/ml dans un tampon Tris pH 6,2. Après environ 20h d'incubation à +18/25°C avec la solution de sensibilisation, les cônes ont été vidés. Ensuite, 300 µL de cette même solution contenant 5 g/L d'albumine bovine sont ajoutés pour la passivation des cônes à +18/25°C pendant environ 20h. Les cônes sont ensuite vidés, séchés, puis conservés à +4°C jusqu'à utilisation, à l'abri de l'humidité.

*Préparation des solutions d'anticorps conjugués*. Pour le couple C1 la solution de conjugué contient l'anticorps monoclonal 4G10E12, sous forme de fragment Fab' couplé à la phosphatase alcaline. Pour le couple C2, la solution de conjugué contient l'anticorps monoclonal 5G5A10, sous forme de fragment Fab' couplé à la phosphatase alcaline. Les anticorps conjugués ont été dilués à environ 0,1 µg/ml dans un tampon Tris/ NaCl BSA pH6,5.

*Immunoessai.* Dès que le cône VIDAS® est en contact avec l'échantillon, la réaction immunologique commence car les anticorps de capture sont immobilisés sur ce cône. L'automate mélange l'échantillon à tester (89,6 µL) avec 226 µL de la solution de conjugué. L'incubation dure environ 10 minutes à 37°C et permet la liaison spécifique de l'AMH à l'anticorps adsorbé sur le cône d'une part et à l'anticorps conjugué (anticorps de détection) d'autre part. Ensuite, les composants non liés sont éliminés par 3 lavages avec un tampon Tris 54 mM pH 7,3, NaCl 154 mM, Tween 20 à 0,55%. Lors de l'étape finale de révélation, le substrat 4-méthylombelliferyl phosphate est aspiré puis refoulé dans le cône ; l'enzyme des anticorps conjugués catalyse la réaction d'hydrolyse de ce substrat en 4-méthylombelliferone dont la fluorescence émise est mesurée à 450 nm. La valeur du signal de fluorescence (RFV=relative fluorescence value) est proportionnelle à la concentration de l'antigène présent dans l'échantillon.

### 6.2. Dosage AMH des échantillons ayant eu différentes conditions de conservation

Huit pools d'échantillons naturels de femmes âgées entre 19 et 52 ans ont été dosés en parallèle avec le kit commercial AMH Gen II assay (Beckman Coulter) et avec deux couples d'anticorps (C1 et C2) sur l'automate VIDAS®. Chaque pool d'échantillons est constitué de 3 sérums de femmes issus d'un Etablissement Français du Sang (EFS) de la région Rhône Alpes.

Ces échantillons ont été testés moins de 4 heures après le prélèvement correspondant au temps T0, puis après 24 heures de conservation à +2/8°C, après 7 jours de conservation à +2/8°C et 7 jours de conservation à -19/-31°C correspondant aux différentes conditions de conservation de ces échantillons.

Pour le kit en microplaque, les doses respectives ont été calculées en fonction de la gamme préconisée par le fabricant. Ensuite les rapports des doses obtenues pour chaque condition de conservation par rapport à la dose obtenue à T0 ont été calculés. Pour l'automate VIDAS®, les signaux de fluorescence (RFV = Relative fluorescence value) obtenus avec chacun des 2 couples d'anticorps, pour chaque condition de conservation, ont été utilisés pour calculer les rapports sur les signaux de fluorescence obtenus à T0. Les résultats obtenus sont présentés dans le Tableau 8.

**Tableau 8. Rapport des doses ou signaux AMH obtenus après conservation dans différentes conditions et à T0 (ratio T/T0).**

| | **24h à +2/8°C** | | | **7 jours à +2/8°C** | | | **7 jours à -19/-31°C** | | |
|---|---|---|---|---|---|---|---|---|---|
| | **C1** | **C2** | **REF** | **C1** | **C2** | **REF** | **C1** | **C2** | **REF** |
| Pool 001 | 0,99 | 1,00 | 1,10 | 0,95 | 1,06 | 1,14 | 0,98 | 0,99 | 1,20 |
| Pool 002 | 0,98 | 1,04 | 1,27 | 0,96 | 1,13 | 1,20 | 0,99 | 1,03 | 1,30 |
| Pool 003 | 0,98 | 1,00 | 1,14 | 0,96 | 1,04 | 1,05 | 1,01 | 1,02 | 1,07 |
| Pool 004 | 1,01 | 0,99 | 1,58 | 0,97 | 1,08 | 1,57 | 0,99 | 1,02 | 1,73 |
| Pool 005 | 0,96 | 1,02 | 1,46 | 0,95 | 1,09 | 1,33 | 0,97 | 1,01 | 1,24 |
| Pool 006 | 1,00 | 1,01 | 1,35 | 0,97 | 1,10 | 1,40 | 0,97 | 0,99 | 1,35 |
| Pool 007 | 0,97 | 1,03 | 1,43 | 0,96 | 1,11 | 1,66 | 0,95 | 1,01 | 1,51 |
| Pool 008 | 0,99 | 1,06 | 1,34 | 0,95 | 1,07 | 1,34 | 1,00 | 1,03 | 1,35 |
| Moyenne | 0,99 | 1,02 | 1,33 | 0,96 | 1,09 | 1,34 | 0,98 | 1,01 | 1,34 |
| Min | 0,96 | 0,99 | 1,10 | 0,95 | 1,04 | 1,05 | 0,95 | 0,99 | 1,07 |
| Max | 1,01 | 1,06 | 1,58 | 0,97 | 1,13 | 1,66 | 1,01 | 1,03 | 1,73 |

| | | | | | | | | | |
|---|---|---|---|---|---|---|---|---|---|
| C1 : Dosage VIDAS® avec 5G5A10 comme anticorps de capture et 4G10E12 comme anticorps de détection; C2: Dosage VIDAS® avec 8C5B10H5 comme anticorps de capture et 5G5A10 comme anticorps de détection; REF: dosage microplaque AMH Gen II Assay. | | | | | | | | | |

Plus le ratio T/T0 est proche de 1, moins il y a de variabilité dans le temps ou selon les conditions de conservation dans les quantités d'AMH détectés. Les dosages d'AMH utilisant les couples C1 ou C2 rendent toujours le même résultat, quelque soient les conditions de conservation préalable de l'échantillon : le ratio T/T0 est de 0,99 (C1) et 1,02 (C2) en moyenne lorsque l'échantillon a été conservé 24h à +2/8°C. Ces valeurs sont très proches de 1, alors que le kit de référence affiche un ratio moyen de 1,33 indiquant que la dose mesurée est multipliée en moyenne par 1,33. Le même type d'observation est fait pour les autres conditions de conservation testées.

Pour rappel, l'anticorps 5G5A10 reconnaît un épitope non-linéaire de la zone 157-255 de l'AMH humaine, l'anticorps 4G10E12 reconnaît un épitope non-linéaire de la zone 256-451 de l'AMH humaine, l'anticorps 8C5B10H5 reconnaît un épitope linéaire (aa 508-519 de l'AMH humaine), dans la région mature de l'AMH, les anticorps du kit AMH Gen II Assay sont tous les deux dirigés contre des épitopes linéaires de la région mature.

Les couples d'anticorps C1 et C2 permettent de mettre au point des immunodosages d'AMH qui sont robustes et dont le résultat ne varie pas en fonction des conditions de conservation préalable de l'échantillon.

### Exemple 7 : Sensibilité analytique du VIDAS® AMH

La sensibilité analytique de l'immunodosage VIDAS® AMH (Couple C1) décrit dans l'exemple 6 a été comparée à celle de dosages selon l'art antérieur. Afin de pouvoir comparer les couples d'anticorps dans des conditions similaires toute l'expérience a été réalisée sur l'automate VIDAS®. Les cônes ont été coatés soit par l'anticorps 5G5A10 (anticorps reconnaissant la partie C-Terminale de la région pro de l'AMH), soit par l'anticorps AA012 (anticorps commercial - AnshLabs) selon le mode opératoire expliqué dans l'exemple 6. Pour l'étape de détection, deux conjugués ont été comparés: l'anticorps 4G10E12 et l'anticorps AA011 (anticorps commercial - AnshLabs). Tous les deux ont été conjugués à la phosphatase alcaline selon le mode opératoire de l'exemple 6. Le conjugué 4G10E12 a été utilisé à 180 ng/ml, le conjugué AA011 à 500 ng/ml. Le conjugué 4G10E12, reconnaissant la partie C-Terminale de la région pro de l'AMH, est donc utilisé en nettement plus faible quantité par rapport à l'anticorps commercial, ce qui est tout à fait inattendu.

Une gamme étalon allant de 0,055 à 11 ng/ml d'AMH a été préparée par en diluant des sérums pour lesquels les concentrations d'AMH étaient connues dans du sérum de femme ménopausée (concentration AMH négligeable), puis mesurée par les 3 formats de dosages suivants :
Couple C1 : capture 5G5A10 + détection 4G10E12
Couple AnshLab : capture AA012 + détection AA011
Couple mixte : capture 5G5A10 + détection AA011

Les résultats sont présentés dans le Tableau 9.

**Tableau 9. Comparaison des signaux RFV et des ratios S/N obtenus avec différents couples d'anticorps en VIDAS® pour la gamme étalon.**

| | **VIDAS RFV Signal** | | | | **VIDAS RFV/RFV0** | | |
|---|---|---|---|---|---|---|---|
| **Capture** | **5G5A10** | **AA012** | **5G5A10** | | **5G5A10** | **AA012** | **5G5A10** |
| **Détection** (conc ng/ml) | **4G10E12** (180) | **AA011** (500) | **AA011** (500) | | **4G10E12** (180) | **AA011** (500) | **AA011** (500) |
| AMH ng/ml | RFV | RFV | RFV | | S/N | S/N | S/N |
| 0 | 1 | 45 | 7 | | | | |
| 0,055 | 51 | 81 | 53 | | 51 | 2 | 8 |
| 0,11 | 92 | 113 | 91 | | 92 | 3 | 13 |
| 0,275 | 224 | 209 | 221 | | 224 | 5 | 32 |
| 0,9 | 916 | 735 | 910 | | 916 | 16 | 130 |
| 2,1 | 2113 | 1602 | 2097 | | 2113 | 36 | 300 |
| 4 | 3817 | 2938 | 3816 | | 3817 | 65 | 545 |
| 6,3 | 4839 | 3915 | 4891 | | 4839 | 87 | 699 |
| 8,7 | 5672 | 4810 | 5764 | | 5672 | 107 | 823 |
| 11 | 6335 | 5392 | 6404 | | 6335 | 120 | 915 |

| | | | | | | | |
|---|---|---|---|---|---|---|---|
| S/N = ration « signal/noise » ou signal/bruit de fond. Pour un format de dosage donné, ration du signal RFV sur le signal RFV du point 0 de la gamme (sans AMH). | | | | | | | |

Le couple C1 (5G5A10 & 4G10E12) présente une excellente dynamique du signal et un bruit de fond extrêmement faible (1 RFV au point 0). Le couple selon l'art antérieur AA012 & AA011 présente une dynamique du signal moins bonne (5392 RFV pour 11 ng/ml, au lieu de 6335 RFV pour le couple C1) et surtout un bruit de fond considérable de 45 RFV. Le couple mixte (5G5A10 & AA011) a une dynamique du signal comparable au couple C1 (même anticorps de capture). Le bruit de fond (7 RFV) est considérablement plus bas que pour le couple AA012 & AA011, mais reste plus élevé que pour le couple C1. Comme on peut le voir au niveau des ratios signal/bruit (S/N), le dosage le plus sensible analytiquement est celui qui utilise le couple C1, puis celui avec le couple mixte, et en dernier celui avec le couple AnshLabs.

### Exemple 8 : Spécificité analytique du dosage VIDAS AMH

La spécificité analytique du test VIDAS AMH (Couple C1) décrit dans l'exemple 6 a été établie en analysant des composés à réactivité croisée. Ces composés ont été ajoutés en surcharge à des échantillons de sérum contenant 1 ng/ml et 4 ng/ml d'AMH. Les résultats de cette étude sont résumés dans le Tableau 10 :

**Tableau 10. Réactivités croisées du dosage VIDAS® AMH**

| **Composé testé** | **Concentration testée** | **% Réactivité croisée** |
|---|---|---|
| Activine A | 100 ng/ml | ≤ 0,10% |
| Inhibine A | 100 ng/ml | ≤ 0,12% |
| LH | 500 IU/I | ≤ 0,21% |
| FSH | 500 IU/I | ≤ 0,23% |

Aucune réactivité croisée significative n'a été détectée aux concentrations testées. Le dosage VIDAS AMH présente une excellente spécificité analytique.

### Exemple 9: Précision du dosage VIDAS AMH

L'étude de précision du test VIDAS AMH (Couple C1) a été réalisée en utilisant un panel d'échantillons humains représentatifs de 5 niveaux de concentration de l'intervalle de mesure. Pour chaque niveau de concentration, la répétabilité (précision intra-série), la précision intra-lot et la précision intra-laboratoire (inter-lots intra-instrument) ont été estimées. Les valeurs obtenues lors de cette étude sont reportées dans le Tableau 11 :

**Tableau 11. Précision du dosage VIDAS AMH**

| **N (nombre de répétitions)** | **Niveau de concentration (ng/ml)** | **%CV Répétabilité** | **%CV Intra-lot** | **%CV Intra-laboratoire** |
|---|---|---|---|---|
| 519 | 0,22 | 4,1 | 6,6 | 8,3 |
| 520 | 1,08 | 4,4 | 8,0 | 9,9 |
| 520 | 2,99 | 4,4 | 7,4 | 9,8 |
| 520 | 5,45 | 4,8 | 7,6 | 8,9 |
| 520 | 7,37 | 4,4 | 8,2 | 10,6 |

Comme le montre cette très large étude, le dosage VIDAS AMH présente une excellente reproductibilité : le coefficient de variation (CV) entre différents lots ne dépasse pas 11%.

### Références Bibliographiques

- ARCE JC, et al., 2014, Fertility and Sterility, 102(6): 1633-40
- BOERSMA YL, PLÜCKTHUN A, 2011, Curr. Opin. Biotechnol, 22 : 849-857
- CHAI J et HOWIE AF, 2014, European Journal of Cancer, 50(14): 2367-74
- DEWAILLY D, et al., 2014, The physiology and clinical utility of anti-Müllerian hormone in women, Human Reproduction Update, 20(3): 370-85
- ELLINGTON AD et Szostak JW., 1990, Nature, 346 : 818-822
- FALKENBERG FW, 1998, Res Immunol 149(6): 560-570.
- FONG SL, et al., 2015, European Journal of Obstetrics & Gynecology and Reproductive Biology, 186: 75-9
- HAN X et al., 2014, Hum Reprod, 29(5): 1042-1048.
- HUDSON PL et al., 1990, J Clin Endocrinol Metab, 70: 16-22.
- KELSEY TW, et al., 2011, PLoS ONE, 6(7):e22024
- KOHLER G et Milstein C, 1975, Nature, 256: 495-497.
- KOHLER G et al., 1976, Eur J Immunol, 6: 292-295.
- KUMAR A et al., 2010, J Immunol Methods, 362: 51-59.
- LEE M et al., 1996, J Clin Endocrinol Metab, 81: 571-576.
- LONG WQ et al., 2000, J Clin Endocrinol Metab, 85(2): 540-544.
- LUKASZUK K et al., 2014, Hum Reprod, 27(10): 3085-3091.
- PANHURST M.W. et al, 2016, Physiological Reports, 4(9): 1-10
- ZEC I et al., 2011, Biochemia Medica, 21(3):219-30.

## Revendications

1. Procédé de préparation d'anticorps anti-AMH de mammifère (hormone antimüllérienne de mammifère), **caractérisé en ce qu'**il comprend les étapes de :
(i) immunisation d'un animal avec un polypeptide d'AMH ou un polynucléotide codant pour ce polypeptide d'AMH, ledit polypeptide d'AMH comprenant au moins les 99 acides aminés de séquence SEQ ID N°1 ou de séquence ayant au moins 75% d'identité avec la séquence SEQIDN°1, et au plus les 560 acides aminés de séquence SEQ ID N°2 ou de séquence ayant au moins 75% d'identité avec la séquence SEQ ID N°2,
(ii) préparation d'hybridomes à partir des cellules d'un organe lymphoïde de l'animal ayant reçu l'immunogène,
(iii) sélection des hybridomes secrétant des anticorps reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQ ID N°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11, ni (b) aucun épitope linéaire situé dans la séquence SEQ ID N°1 ou une séquence ayant au moins 75% d'identité avec la séquence SEQ ID N°1, et
(iv) production des anticorps.

2. Procédé de préparation d'anticorps anti-AMH selon la revendication 1, dans lequel l'immunogène utilisé est un polypeptide d'AMH, ou un polynucléotide codant pour ledit polypeptide d'AMH, lequel comprend au moins les 230 acides aminés de séquence SEQ ID N°10 ou de séquence ayant au moins 75% d'identité avec la séquence SEQ ID N°10 et au plus les 255 acides aminés de séquence SEQ ID N°8 ou de séquence ayant au moins 75% d'identité avec la séquence SEQ ID N°8, de préférence choisis parmi les polypeptides d'AMH de séquences SEQ ID N°8, SEQ ID N°9 et SEQ ID N°10 ou de séquences ayant au moins 75% d'identité avec ces séquences.

3. Procédé de préparation d'anticorps anti-AMH selon la revendication 1, dans lequel l'immunogène utilisé est un polypeptide d'AMH, ou un polynucléotide codant pour ledit polypeptide d'AMH, lequel comprend au moins les 535 acides aminés de séquence SEQ ID N°4 ou de séquence ayant au moins 75% d'identité avec la séquence SEQ ID N°4, de préférence choisis parmi les polypeptides d'AMH de séquences SEQ ID N°2, SEQ ID N°3 et SEQ ID N°4 ou de séquences ayant au moins 75% d'identité avec ces séquences.

4. Procédé de préparation d'anticorps anti-AMH selon l'une quelconque des revendications 1 à 3, dans lequel le polypeptide d'AMH (a) non reconnu par les anticorps est un polypeptide de séquences choisies parmi: SEQ ID N°11, SEQ ID N°12 et SEQ ID N°13 et de séquences ayant au moins 75% d'identité avec ces séquences.

5. Procédé de préparation d'anticorps anti-AMH selon la revendication 4, dans lequel les séquences ayant au moins 75% d'identité avec les séquences SEQ ID N°11, SEQ ID N°12, et SEQ ID N°13 sont choisies parmi les séquences: SEQ ID N°21, SEQ ID N°22, SEQ ID N°30, SEQ ID N°31, SEQ ID N°42, SEQ ID N°43 et SEQ ID N°44.

6. Procédé de préparation d'anticorps anti-AMH selon l'une quelconque des revendications 1 à 4, dans lequel les épitopes linéaires (b) non reconnus par les anticorps ont les séquences SEQ ID N°45 à SEQ ID N°56.

7. Procédé de préparation d'anticorps anti-AMH selon l'une quelconque des revendications 1 à 5, dans lequel
- le mammifère est l'humain, l'immunogène est un polypeptide d'AMH, ou un polynucléotide codant pour ce polypeptide d'AMH, lequel comprend au moins les 99 acides aminés de séquence SEQ ID N°1 et au plus les 560 acides aminés de séquence SEQ ID N°2, le polypeptide d'AMH reconnu par les anticorps sécrétés par les hybridomes est un polypeptide d'AMH comprenant au moins les 99 acides aminés de séquence SEQ ID N°1 et au plus les 255 acides aminés de séquence SEQ ID N°8, et le polypeptide d'AMH (a) non reconnu par les anticorps sécrétés par les hybridomes est un polypeptide d'AMH comprenant au moins les 131 acides aminés de séquence SEQ ID N°13 et au plus les 156 acides aminés de séquence SEQ ID N°11, ou
- le mammifère est le cheval, l'immunogène est un polypeptide d'AMH, ou un polynucléotide codant pour ce polypeptide d'AMH, lequel comprend au moins les 99 acides aminés de séquence SEQ ID N°14 et au plus les 573 acides aminés de séquence SEQIDN°15, le polypeptide d'AMH reconnu par les anticorps sécrétés par les hybridomes est un polypeptide d'AMH comprenant au moins les 99 acides aminés de séquence SEQ ID N°14 et au plus les 265 acides aminés de séquence SEQ ID N°19, et le polypeptide d'AMH (a) non reconnu par les anticorps sécrétés par les hybridomes est un polypeptide d'AMH comprenant au moins les 144 acides aminés de séquence SEQ ID N°22 et au plus les 166 acides aminés de séquence SEQ ID N°21, ou
- le mammifère est le chien, l'immunogène est un polypeptide d'AMH, ou un polynucléotide codant pour ce polypeptide d'AMH, lequel comprend au moins les 99 acides aminés de séquence SEQ ID N°23 et au plus les 572 acides aminés de séquence SEQ ID N°24, le polypeptide d'AMH reconnu par les anticorps sécrétés par les hybridomes est un polypeptide d'AMH comprenant au moins les 99 acides aminés de séquence SEQ ID N°23 et au plus les 264 acides aminés de séquence SEQ ID N°28, et le polypeptide d'AMH (a) non reconnu par les anticorps sécrétés par les hybridomes est un polypeptide d'AMH comprenant au moins les 144 acides aminés de séquence SEQ ID N°31 et au plus les 165 acides aminés de séquence SEQ ID N°30, ou
- le mammifère est un bovin, l'immunogène est un polypeptide d'AMH, ou un polynucléotide codant pour ce polypeptide d'AMH, lequel comprend au moins les 100 acides aminés de séquence SEQ ID N°32 et au plus les 575 acides aminés de séquence SEQ ID N°33, le polypeptide d'AMH reconnu par les anticorps sécrétés par les hybridomes est un polypeptide d'AMH comprenant au moins les 100 acides aminés de séquence SEQ ID N°32 et au plus les 270 acides aminés de séquence SEQ ID N°39, et le polypeptide d'AMH (a) non reconnu par les anticorps sécrétés par les hybridomes est un polypeptide d'AMH comprenant au moins les 146 acides aminés de séquence SEQ ID N°44 et au plus les 270 acides aminés de séquence SEQ ID N°42.

8. Anticorps monoclonal ou fragment d'anticorps monoclonal anti-AMH de mammifère reconnaissant un polypeptide d'AMH comprenant au moins les 99 acides aminés de séquence SEQ ID N°1 ou de séquence ayant au moins 75% d'identité avec la séquence SEQ ID N°1, et au plus les 255 acides aminés de séquence SEQ ID N°8 ou de séquence ayant au moins 75% d'identité avec la séquence SEQ ID N°8, mais ne reconnaissant ni (a) un polypeptide d'AMH comprenant au moins les 131 acides aminés de séquence SEQ ID N°13 ou de séquence ayant au moins 75% d'identité avec la séquence SEQ ID N°13 et au plus les 156 acides aminés de séquence SEQ ID N°11 ou de séquence ayant au moins 75% d'identité avec la séquence SEQ ID N°11, ni aucun épitope linéaire situé dans la séquence SEQ ID N°1 ou une séquence ayant au moins 75% d'identité avec la séquence SEQ ID N°1.

9. Anticorps monoclonal ou fragment d'anticorps monoclonal anti-AMH de mammifère selon la revendication 8, pour lequel le polypeptide d'AMH (a) non reconnu est un polypeptide de séquences choisies parmi : SEQ ID N°11, SEQ ID N°12 et SEQ ID N°13 ou de séquences ayant au moins 75% d'identité avec ces séquences.

10. Anticorps monoclonal ou fragment d'anticorps monoclonal anti-AMH de mammifère selon la revendication 9, pour lesquels les séquences ayant au moins 75% d'identité avec les séquences SEQ ID N°11, SEQ ID N°12, et SEQ ID N°13 sont choisies parmi les séquences: SEQ ID N°21, SEQ ID N°22, SEQ ID N°30, SEQ ID N°31, SEQ ID N°42, SEQ ID N°43 et SEQ ID N°44.

11. Anticorps monoclonal ou fragment d'anticorps monoclonal anti-AMH de mammifère selon l'une quelconque des revendications 8 à 10, pour les épitopes linéaires (b) non reconnus par les anticorps ont les séquences SEQ ID N°45 à SEQ ID N°56.

12. Anticorps monoclonal ou fragment d'anticorps monoclonal anti-AMH de mammifère selon l'une quelconque des revendications 9 à 11, pour lequel
- le mammifère est l'humain, le polypeptide d'AMH reconnu est un polypeptide d'AMH comprenant au moins les 99 acides aminés de séquence SEQ ID N°1 et au plus les 255 acides aminés de séquence SEQ ID N°8, et le polypeptide d'AMH (a) non reconnu est un polypeptide d'AMH comprenant au moins les 131 acides aminés de séquence SEQ ID N°13 et au plus les 156 acides aminés de séquence SEQ ID N°11, ou
- le mammifère est le cheval, le polypeptide d'AMH reconnu est un polypeptide d'AMH comprenant au moins les 99 acides aminés de séquence SEQ ID N°14 et au plus les 265 acides aminés de séquence SEQ ID N°19, et le polypeptide d'AMH (a) non reconnu est un polypeptide d'AMH comprenant au moins les 144 acides aminés de séquence SEQ ID N°22 et au plus les 166 acides aminés de séquence SEQ ID N°21, ou
- le mammifère est le chien, le polypeptide d'AMH reconnu est un polypeptide d'AMH comprenant au moins les 99 acides aminés de séquence SEQ ID N°23 et au plus les 264 acides aminés de séquence SEQ ID N°28, et le polypeptide d'AMH (a) non reconnu est un polypeptide d'AMH comprenant au moins les 144 acides aminés de séquence SEQ ID N°31 et au plus les 165 acides aminés de séquence SEQ ID N°30, ou
- le mammifère est un bovin, le polypeptide d'AMH reconnu est un polypeptide d'AMH comprenant au moins les 100 acides aminés de séquence SEQ ID N°32 et au plus les 270 acides aminés de séquence SEQ ID N°39, et le polypeptide d'AMH (a) non reconnu est un polypeptide d'AMH comprenant au moins les 146 acides aminés de séquence SEQ ID N°44 et au plus les 270 acides aminés de séquence SEQ ID N°42.

13. Conjugué comprenant (i) un anticorps monoclonal ou un fragment d'anticorps monoclonal anti-AMH tel que défini dans l'une quelconque des revendications 8 à 12 ou préparé selon le procédé tel que défini dans l'une quelconque des revendications 1 à 7 et (ii) un marqueur capable de générer l'émission un signal détectable pour la visualisation d'une réaction immunologique entre ce conjugué et l'AMH d'un échantillon biologique.

14. Procédé de quantification d'AMH de mammifère par immunoessai sandwich, dans un échantillon biologique susceptible de contenir de l'AMH, lequel comprend les étapes suivantes :
- mettre en contact ledit échantillon biologique avec deux partenaires de liaison à l'AMH dont au moins un desdits partenaires est un anticorps ou fragment d'anticorps tel que défini dans l'une quelconque des revendications 8 à 12 ou tel que préparé selon le procédé tel que défini dans l'une quelconque des revendications 1 à 7, ou bien un conjugué tel que défini dans la revendication 13,
- détecter un signal émis par la liaison entre lesdits partenaires de liaison et l'AMH, si elle est présente, en utilisant un marqueur capable de générer l'émission d'un signal détectable, et
- transformer le signal détecté en une concentration d'AMH.

15. Procédé de quantification d'AMH selon la revendication 14, dans lequel l'échantillon biologique est un échantillon de sang, de sérum ou de plasma.

16. Procédé de quantification d'AMH selon l'une des revendications 14 ou 15, dans lequel le deuxième partenaire de liaison à l'AMH est un anticorps reconnaissant un polypeptide d'AMH comprenant au moins les 196 acides aminés de séquence SEQ ID N°57 ou de séquence ayant au moins 75% d'identité avec la séquence SEQ ID N°57, mais ne reconnaissant ni (a) un polypeptide d'AMH comprenant au moins les 230 acides aminés de séquence SEQIDN°10, ou de séquence ayant au moins 75% d'identité avec la séquence SEQ IDN°10 et au plus les 255 acides aminés de séquence SEQ ID N°8, ou de séquence ayant au moins 75% d'identité avec la séquence SEQ ID N°8, ni (b) un épitope linéaire situé dans la séquence SEQ ID N°57 ou une séquence ayant au moins 75% d'identité avec la séquence SEQ ID N°57.

17. Utilisation d'un procédé de quantification d'AMH tel que défini dans l'une quelconque des revendications 14 à 16 comme une aide pour le diagnostic de troubles liés à un dysfonctionnement ovarien chez les femmes en âge de procréer.

18. Utilisation d'un procédé de quantification d'AMH tel que défini dans l'une quelconque des revendications 14 à 16 comme une aide à l'évaluation de la réserve folliculaire ovarienne chez les jeunes filles de plus de 12 ans et les femmes.

19. Utilisation d'un procédé de quantification d'AMH tel que défini dans l'une quelconque des revendications 14 à 16 comme une aide à l'évaluation des troubles liés à la différentiation sexuelle chez le garçon avant la puberté.

20. Trousse comprenant un anticorps ou fragment d'anticorps tel que défini dans l'une quelconque des revendications 8 à 12 ou tel que préparé selon le procédé tel que défini dans l'une quelconque des revendications 1 à 7, et/ou un conjugué tel que défini dans la revendication 13.


SEQUENCE LISTING

<110>  BIOMERIEUX

<120>  Procédé de préparation d'anticorps anti-AMH et leurs utilisations

<130>  ANTIAMH-DK1778

<160>  158

<170>  PatentIn version 3.5

<210>  1
<211>  99
<212>  PRT
<213>  artificial sequence

<220>
<223>  polypeptide

<400>  1

Gly Pro Pro Glu Leu Ala Leu Leu Val Leu Tyr Pro Gly Pro Gly Pro
1               5                   10                  15

Glu Val Thr Val Thr Arg Ala Gly Leu Pro Gly Ala Gln Ser Leu Cys
            20                  25                  30

Pro Ser Arg Asp Thr Arg Tyr Leu Val Leu Ala Val Asp Arg Pro Ala
        35                  40                  45

Gly Ala Trp Arg Gly Ser Gly Leu Ala Leu Thr Leu Gln Pro Arg Gly
    50                  55                  60

Glu Asp Ser Arg Leu Ser Thr Ala Arg Leu Gln Ala Leu Leu Phe Gly
65                  70                  75                  80

Asp Asp His Arg Cys Phe Thr Arg Met Thr Pro Ala Leu Leu Leu Leu
                85                  90                  95

Pro Arg Ser

<210>  2
<211>  560
<212>  PRT
<213>  human

<400>  2

<210>  3
<211>  542
<212>  PRT
<213>  artificial sequence

<220>
<223>  poplypeptide

<400>  3

Leu Leu Gly Thr Glu Ala Leu Arg Ala Glu Glu Pro Ala Val Gly Thr
1               5                   10                  15

Ser Gly Leu Ile Phe Arg Glu Asp Leu Asp Trp Pro Pro Gly Ser Pro
            20                  25                  30

Gln Glu Pro Leu Cys Leu Val Ala Leu Gly Gly Asp Ser Asn Gly Ser
        35                  40                  45

Ser Ser Pro Leu Arg Val Val Gly Ala Leu Ser Ala Tyr Glu Gln Ala
    50                  55                  60

Phe Leu Gly Ala Val Gln Arg Ala Arg Trp Gly Pro Arg Asp Leu Ala
65                  70                  75                  80

Thr Phe Gly Val Cys Asn Thr Gly Asp Arg Gln Ala Ala Leu Pro Ser
                85                  90                  95

Leu Arg Arg Leu Gly Ala Trp Leu Arg Asp Pro Gly Gly Gln Arg Leu
            100                 105                 110

Val Val Leu His Leu Glu Glu Val Thr Trp Glu Pro Thr Pro Ser Leu
        115                 120                 125

Arg Phe Gln Glu Pro Pro Pro Gly Gly Ala Gly Pro Pro Glu Leu Ala
    130                 135                 140

Leu Leu Val Leu Tyr Pro Gly Pro Gly Pro Glu Val Thr Val Thr Arg
145                 150                 155                 160

Ala Gly Leu Pro Gly Ala Gln Ser Leu Cys Pro Ser Arg Asp Thr Arg
                165                 170                 175

Tyr Leu Val Leu Ala Val Asp Arg Pro Ala Gly Ala Trp Arg Gly Ser

180                 185                 190

Gly Leu Ala Leu Thr Leu Gln Pro Arg Gly Glu Asp Ser Arg Leu Ser
        195                 200                 205

Thr Ala Arg Leu Gln Ala Leu Leu Phe Gly Asp Asp His Arg Cys Phe
    210                 215                 220

Thr Arg Met Thr Pro Ala Leu Leu Leu Leu Pro Arg Ser Glu Pro Ala
225                 230                 235                 240

Pro Leu Pro Ala His Gly Gln Leu Asp Thr Val Pro Phe Pro Pro Pro
                245                 250                 255

Arg Pro Ser Ala Glu Leu Glu Glu Ser Pro Pro Ser Ala Asp Pro Phe
            260                 265                 270

Leu Glu Thr Leu Thr Arg Leu Val Arg Ala Leu Arg Val Pro Pro Ala
        275                 280                 285

Arg Ala Ser Ala Pro Arg Leu Ala Leu Asp Pro Asp Ala Leu Ala Gly
    290                 295                 300

Phe Pro Gln Gly Leu Val Asn Leu Ser Asp Pro Ala Ala Leu Glu Arg
305                 310                 315                 320

Leu Leu Asp Gly Glu Glu Pro Leu Leu Leu Leu Leu Arg Pro Thr Ala
                325                 330                 335

Ala Thr Thr Gly Asp Pro Ala Pro Leu His Asp Pro Thr Ser Ala Pro
            340                 345                 350

Trp Ala Thr Ala Leu Ala Arg Arg Val Ala Ala Glu Leu Gln Ala Ala
        355                 360                 365

Ala Ala Glu Leu Arg Ser Leu Pro Gly Leu Pro Pro Ala Thr Ala Pro
    370                 375                 380

Leu Leu Ala Arg Leu Leu Ala Leu Cys Pro Gly Gly Pro Gly Gly Leu
385                 390                 395                 400

Gly Asp Pro Leu Arg Ala Leu Leu Leu Leu Lys Ala Leu Gln Gly Leu
                405                 410                 415

Arg Val Glu Trp Arg Gly Arg Asp Pro Arg Gly Pro Gly Arg Ala Gln
            420                 425                 430

Arg Ser Ala Gly Ala Thr Ala Ala Asp Gly Pro Cys Ala Leu Arg Glu
        435                 440                 445

Leu Ser Val Asp Leu Arg Ala Glu Arg Ser Val Leu Ile Pro Glu Thr
    450                 455                 460

Tyr Gln Ala Asn Asn Cys Gln Gly Val Cys Gly Trp Pro Gln Ser Asp
465                 470                 475                 480

Arg Asn Pro Arg Tyr Gly Asn His Val Val Leu Leu Leu Lys Met Gln
                485                 490                 495

Val Arg Gly Ala Ala Leu Ala Arg Pro Pro Cys Cys Val Pro Thr Ala
            500                 505                 510

Tyr Ala Gly Lys Leu Leu Ile Ser Leu Ser Glu Glu Arg Ile Ser Ala
        515                 520                 525

His His Val Pro Asn Met Val Ala Thr Glu Cys Gly Cys Arg
    530                 535                 540

<210>  4
<211>  535
<212>  PRT
<213>  artificial sequence

<220>
<223>  polypeptide

<400>  4

Arg Ala Glu Glu Pro Ala Val Gly Thr Ser Gly Leu Ile Phe Arg Glu
1               5                   10                  15

Asp Leu Asp Trp Pro Pro Gly Ser Pro Gln Glu Pro Leu Cys Leu Val
            20                  25                  30

Ala Leu Gly Gly Asp Ser Asn Gly Ser Ser Ser Pro Leu Arg Val Val
        35                  40                  45

Gly Ala Leu Ser Ala Tyr Glu Gln Ala Phe Leu Gly Ala Val Gln Arg
    50                  55                  60

Ala Arg Trp Gly Pro Arg Asp Leu Ala Thr Phe Gly Val Cys Asn Thr
65                  70                  75                  80

Gly Asp Arg Gln Ala Ala Leu Pro Ser Leu Arg Arg Leu Gly Ala Trp
                85                  90                  95

Leu Arg Asp Pro Gly Gly Gln Arg Leu Val Val Leu His Leu Glu Glu

            100                 105                 110

Val Thr Trp Glu Pro Thr Pro Ser Leu Arg Phe Gln Glu Pro Pro Pro
        115                 120                 125

Gly Gly Ala Gly Pro Pro Glu Leu Ala Leu Leu Val Leu Tyr Pro Gly
    130                 135                 140

Pro Gly Pro Glu Val Thr Val Thr Arg Ala Gly Leu Pro Gly Ala Gln
145                 150                 155                 160

Ser Leu Cys Pro Ser Arg Asp Thr Arg Tyr Leu Val Leu Ala Val Asp
                165                 170                 175

Arg Pro Ala Gly Ala Trp Arg Gly Ser Gly Leu Ala Leu Thr Leu Gln
            180                 185                 190

Pro Arg Gly Glu Asp Ser Arg Leu Ser Thr Ala Arg Leu Gln Ala Leu
        195                 200                 205

Leu Phe Gly Asp Asp His Arg Cys Phe Thr Arg Met Thr Pro Ala Leu
    210                 215                 220

Leu Leu Leu Pro Arg Ser Glu Pro Ala Pro Leu Pro Ala His Gly Gln
225                 230                 235                 240

Leu Asp Thr Val Pro Phe Pro Pro Pro Arg Pro Ser Ala Glu Leu Glu
                245                 250                 255

Glu Ser Pro Pro Ser Ala Asp Pro Phe Leu Glu Thr Leu Thr Arg Leu
            260                 265                 270

Val Arg Ala Leu Arg Val Pro Pro Ala Arg Ala Ser Ala Pro Arg Leu
        275                 280                 285

Ala Leu Asp Pro Asp Ala Leu Ala Gly Phe Pro Gln Gly Leu Val Asn
    290                 295                 300

Leu Ser Asp Pro Ala Ala Leu Glu Arg Leu Leu Asp Gly Glu Glu Pro
305                 310                 315                 320

Leu Leu Leu Leu Leu Arg Pro Thr Ala Ala Thr Thr Gly Asp Pro Ala
                325                 330                 335

Pro Leu His Asp Pro Thr Ser Ala Pro Trp Ala Thr Ala Leu Ala Arg
            340                 345                 350

Arg Val Ala Ala Glu Leu Gln Ala Ala Ala Ala Glu Leu Arg Ser Leu
        355                 360                 365

Pro Gly Leu Pro Pro Ala Thr Ala Pro Leu Leu Ala Arg Leu Leu Ala
    370                 375                 380

Leu Cys Pro Gly Gly Pro Gly Gly Leu Gly Asp Pro Leu Arg Ala Leu
385                 390                 395                 400

Leu Leu Leu Lys Ala Leu Gln Gly Leu Arg Val Glu Trp Arg Gly Arg
                405                 410                 415

Asp Pro Arg Gly Pro Gly Arg Ala Gln Arg Ser Ala Gly Ala Thr Ala
            420                 425                 430

Ala Asp Gly Pro Cys Ala Leu Arg Glu Leu Ser Val Asp Leu Arg Ala
        435                 440                 445

Glu Arg Ser Val Leu Ile Pro Glu Thr Tyr Gln Ala Asn Asn Cys Gln
    450                 455                 460

Gly Val Cys Gly Trp Pro Gln Ser Asp Arg Asn Pro Arg Tyr Gly Asn
465                 470                 475                 480

His Val Val Leu Leu Leu Lys Met Gln Val Arg Gly Ala Ala Leu Ala
                485                 490                 495

Arg Pro Pro Cys Cys Val Pro Thr Ala Tyr Ala Gly Lys Leu Leu Ile
            500                 505                 510

Ser Leu Ser Glu Glu Arg Ile Ser Ala His His Val Pro Asn Met Val
        515                 520                 525

Ala Thr Glu Cys Gly Cys Arg
    530                 535

<210>  5
<211>  451
<212>  PRT
<213>  artificial sequence

<220>
<223>  polypeptide

<400>  5

Met Arg Asp Leu Pro Leu Thr Ser Leu Ala Leu Val Leu Ser Ala Leu
1               5                   10                  15

Gly Ala Leu Leu Gly Thr Glu Ala Leu Arg Ala Glu Glu Pro Ala Val

20                  25                  30

Gly Thr Ser Gly Leu Ile Phe Arg Glu Asp Leu Asp Trp Pro Pro Gly
        35                  40                  45

Ser Pro Gln Glu Pro Leu Cys Leu Val Ala Leu Gly Gly Asp Ser Asn
    50                  55                  60

Gly Ser Ser Ser Pro Leu Arg Val Val Gly Ala Leu Ser Ala Tyr Glu
65                  70                  75                  80

Gln Ala Phe Leu Gly Ala Val Gln Arg Ala Arg Trp Gly Pro Arg Asp
                85                  90                  95

Leu Ala Thr Phe Gly Val Cys Asn Thr Gly Asp Arg Gln Ala Ala Leu
            100                 105                 110

Pro Ser Leu Arg Arg Leu Gly Ala Trp Leu Arg Asp Pro Gly Gly Gln
        115                 120                 125

Arg Leu Val Val Leu His Leu Glu Glu Val Thr Trp Glu Pro Thr Pro
    130                 135                 140

Ser Leu Arg Phe Gln Glu Pro Pro Pro Gly Gly Ala Gly Pro Pro Glu
145                 150                 155                 160

Leu Ala Leu Leu Val Leu Tyr Pro Gly Pro Gly Pro Glu Val Thr Val
                165                 170                 175

Thr Arg Ala Gly Leu Pro Gly Ala Gln Ser Leu Cys Pro Ser Arg Asp
            180                 185                 190

Thr Arg Tyr Leu Val Leu Ala Val Asp Arg Pro Ala Gly Ala Trp Arg
        195                 200                 205

Gly Ser Gly Leu Ala Leu Thr Leu Gln Pro Arg Gly Glu Asp Ser Arg
    210                 215                 220

Leu Ser Thr Ala Arg Leu Gln Ala Leu Leu Phe Gly Asp Asp His Arg
225                 230                 235                 240

Cys Phe Thr Arg Met Thr Pro Ala Leu Leu Leu Leu Pro Arg Ser Glu
                245                 250                 255

Pro Ala Pro Leu Pro Ala His Gly Gln Leu Asp Thr Val Pro Phe Pro
            260                 265                 270

Pro Pro Arg Pro Ser Ala Glu Leu Glu Glu Ser Pro Pro Ser Ala Asp
        275                 280                 285

Pro Phe Leu Glu Thr Leu Thr Arg Leu Val Arg Ala Leu Arg Val Pro
    290                 295                 300

Pro Ala Arg Ala Ser Ala Pro Arg Leu Ala Leu Asp Pro Asp Ala Leu
305                 310                 315                 320

Ala Gly Phe Pro Gln Gly Leu Val Asn Leu Ser Asp Pro Ala Ala Leu
                325                 330                 335

Glu Arg Leu Leu Asp Gly Glu Glu Pro Leu Leu Leu Leu Leu Arg Pro
            340                 345                 350

Thr Ala Ala Thr Thr Gly Asp Pro Ala Pro Leu His Asp Pro Thr Ser
        355                 360                 365

Ala Pro Trp Ala Thr Ala Leu Ala Arg Arg Val Ala Ala Glu Leu Gln
    370                 375                 380

Ala Ala Ala Ala Glu Leu Arg Ser Leu Pro Gly Leu Pro Pro Ala Thr
385                 390                 395                 400

Ala Pro Leu Leu Ala Arg Leu Leu Ala Leu Cys Pro Gly Gly Pro Gly
                405                 410                 415

Gly Leu Gly Asp Pro Leu Arg Ala Leu Leu Leu Leu Lys Ala Leu Gln
            420                 425                 430

Gly Leu Arg Val Glu Trp Arg Gly Arg Asp Pro Arg Gly Pro Gly Arg
        435                 440                 445

Ala Gln Arg
    450

<210>  6
<211>  433
<212>  PRT
<213>  artificial sequence

<220>
<223>  polypeptide

<400>  6

Leu Leu Gly Thr Glu Ala Leu Arg Ala Glu Glu Pro Ala Val Gly Thr
1               5                   10                  15

Ser Gly Leu Ile Phe Arg Glu Asp Leu Asp Trp Pro Pro Gly Ser Pro

20                  25                  30

Gln Glu Pro Leu Cys Leu Val Ala Leu Gly Gly Asp Ser Asn Gly Ser
        35                  40                  45

Ser Ser Pro Leu Arg Val Val Gly Ala Leu Ser Ala Tyr Glu Gln Ala
    50                  55                  60

Phe Leu Gly Ala Val Gln Arg Ala Arg Trp Gly Pro Arg Asp Leu Ala
65                  70                  75                  80

Thr Phe Gly Val Cys Asn Thr Gly Asp Arg Gln Ala Ala Leu Pro Ser
                85                  90                  95

Leu Arg Arg Leu Gly Ala Trp Leu Arg Asp Pro Gly Gly Gln Arg Leu
            100                 105                 110

Val Val Leu His Leu Glu Glu Val Thr Trp Glu Pro Thr Pro Ser Leu
        115                 120                 125

Arg Phe Gln Glu Pro Pro Pro Gly Gly Ala Gly Pro Pro Glu Leu Ala
    130                 135                 140

Leu Leu Val Leu Tyr Pro Gly Pro Gly Pro Glu Val Thr Val Thr Arg
145                 150                 155                 160

Ala Gly Leu Pro Gly Ala Gln Ser Leu Cys Pro Ser Arg Asp Thr Arg
                165                 170                 175

Tyr Leu Val Leu Ala Val Asp Arg Pro Ala Gly Ala Trp Arg Gly Ser
            180                 185                 190

Gly Leu Ala Leu Thr Leu Gln Pro Arg Gly Glu Asp Ser Arg Leu Ser
        195                 200                 205

Thr Ala Arg Leu Gln Ala Leu Leu Phe Gly Asp Asp His Arg Cys Phe
    210                 215                 220

Thr Arg Met Thr Pro Ala Leu Leu Leu Leu Pro Arg Ser Glu Pro Ala
225                 230                 235                 240

Pro Leu Pro Ala His Gly Gln Leu Asp Thr Val Pro Phe Pro Pro Pro
                245                 250                 255

Arg Pro Ser Ala Glu Leu Glu Glu Ser Pro Pro Ser Ala Asp Pro Phe
            260                 265                 270

Leu Glu Thr Leu Thr Arg Leu Val Arg Ala Leu Arg Val Pro Pro Ala
        275                 280                 285

Arg Ala Ser Ala Pro Arg Leu Ala Leu Asp Pro Asp Ala Leu Ala Gly
    290                 295                 300

Phe Pro Gln Gly Leu Val Asn Leu Ser Asp Pro Ala Ala Leu Glu Arg
305                 310                 315                 320

Leu Leu Asp Gly Glu Glu Pro Leu Leu Leu Leu Leu Arg Pro Thr Ala
                325                 330                 335

Ala Thr Thr Gly Asp Pro Ala Pro Leu His Asp Pro Thr Ser Ala Pro
            340                 345                 350

Trp Ala Thr Ala Leu Ala Arg Arg Val Ala Ala Glu Leu Gln Ala Ala
        355                 360                 365

Ala Ala Glu Leu Arg Ser Leu Pro Gly Leu Pro Pro Ala Thr Ala Pro
    370                 375                 380

Leu Leu Ala Arg Leu Leu Ala Leu Cys Pro Gly Gly Pro Gly Gly Leu
385                 390                 395                 400

Gly Asp Pro Leu Arg Ala Leu Leu Leu Leu Lys Ala Leu Gln Gly Leu
                405                 410                 415

Arg Val Glu Trp Arg Gly Arg Asp Pro Arg Gly Pro Gly Arg Ala Gln
            420                 425                 430

Arg

<210>  7
<211>  426
<212>  PRT
<213>  artificial sequence

<220>
<223>  polypeptide

<400>  7

Arg Ala Glu Glu Pro Ala Val Gly Thr Ser Gly Leu Ile Phe Arg Glu
1               5                   10                  15

Asp Leu Asp Trp Pro Pro Gly Ser Pro Gln Glu Pro Leu Cys Leu Val
            20                  25                  30

Ala Leu Gly Gly Asp Ser Asn Gly Ser Ser Ser Pro Leu Arg Val Val

35                  40                  45

Gly Ala Leu Ser Ala Tyr Glu Gln Ala Phe Leu Gly Ala Val Gln Arg
    50                  55                  60

Ala Arg Trp Gly Pro Arg Asp Leu Ala Thr Phe Gly Val Cys Asn Thr
65                  70                  75                  80

Gly Asp Arg Gln Ala Ala Leu Pro Ser Leu Arg Arg Leu Gly Ala Trp
                85                  90                  95

Leu Arg Asp Pro Gly Gly Gln Arg Leu Val Val Leu His Leu Glu Glu
            100                 105                 110

Val Thr Trp Glu Pro Thr Pro Ser Leu Arg Phe Gln Glu Pro Pro Pro
        115                 120                 125

Gly Gly Ala Gly Pro Pro Glu Leu Ala Leu Leu Val Leu Tyr Pro Gly
    130                 135                 140

Pro Gly Pro Glu Val Thr Val Thr Arg Ala Gly Leu Pro Gly Ala Gln
145                 150                 155                 160

Ser Leu Cys Pro Ser Arg Asp Thr Arg Tyr Leu Val Leu Ala Val Asp
                165                 170                 175

Arg Pro Ala Gly Ala Trp Arg Gly Ser Gly Leu Ala Leu Thr Leu Gln
            180                 185                 190

Pro Arg Gly Glu Asp Ser Arg Leu Ser Thr Ala Arg Leu Gln Ala Leu
        195                 200                 205

Leu Phe Gly Asp Asp His Arg Cys Phe Thr Arg Met Thr Pro Ala Leu
    210                 215                 220

Leu Leu Leu Pro Arg Ser Glu Pro Ala Pro Leu Pro Ala His Gly Gln
225                 230                 235                 240

Leu Asp Thr Val Pro Phe Pro Pro Pro Arg Pro Ser Ala Glu Leu Glu
                245                 250                 255

Glu Ser Pro Pro Ser Ala Asp Pro Phe Leu Glu Thr Leu Thr Arg Leu
            260                 265                 270

Val Arg Ala Leu Arg Val Pro Pro Ala Arg Ala Ser Ala Pro Arg Leu
        275                 280                 285

Ala Leu Asp Pro Asp Ala Leu Ala Gly Phe Pro Gln Gly Leu Val Asn
    290                 295                 300

Leu Ser Asp Pro Ala Ala Leu Glu Arg Leu Leu Asp Gly Glu Glu Pro
305                 310                 315                 320

Leu Leu Leu Leu Leu Arg Pro Thr Ala Ala Thr Thr Gly Asp Pro Ala
                325                 330                 335

Pro Leu His Asp Pro Thr Ser Ala Pro Trp Ala Thr Ala Leu Ala Arg
            340                 345                 350

Arg Val Ala Ala Glu Leu Gln Ala Ala Ala Ala Glu Leu Arg Ser Leu
        355                 360                 365

Pro Gly Leu Pro Pro Ala Thr Ala Pro Leu Leu Ala Arg Leu Leu Ala
    370                 375                 380

Leu Cys Pro Gly Gly Pro Gly Gly Leu Gly Asp Pro Leu Arg Ala Leu
385                 390                 395                 400

Leu Leu Leu Lys Ala Leu Gln Gly Leu Arg Val Glu Trp Arg Gly Arg
                405                 410                 415

Asp Pro Arg Gly Pro Gly Arg Ala Gln Arg
            420                 425


<210>  8
<211>  255
<212>  PRT
<213>  artificial sequence

<220>
<223>  polypeptide

<400>  8

Met Arg Asp Leu Pro Leu Thr Ser Leu Ala Leu Val Leu Ser Ala Leu
1               5                   10                  15

Gly Ala Leu Leu Gly Thr Glu Ala Leu Arg Ala Glu Glu Pro Ala Val
            20                  25                  30

Gly Thr Ser Gly Leu Ile Phe Arg Glu Asp Leu Asp Trp Pro Pro Gly
        35                  40                  45

Ser Pro Gln Glu Pro Leu Cys Leu Val Ala Leu Gly Gly Asp Ser Asn
    50                  55                  60

Gly Ser Ser Ser Pro Leu Arg Val Val Gly Ala Leu Ser Ala Tyr Glu

65                  70                  75                  80

Gln Ala Phe Leu Gly Ala Val Gln Arg Ala Arg Trp Gly Pro Arg Asp
                85                  90                  95

Leu Ala Thr Phe Gly Val Cys Asn Thr Gly Asp Arg Gln Ala Ala Leu
            100                 105                 110

Pro Ser Leu Arg Arg Leu Gly Ala Trp Leu Arg Asp Pro Gly Gly Gln
        115                 120                 125

Arg Leu Val Val Leu His Leu Glu Glu Val Thr Trp Glu Pro Thr Pro
    130                 135                 140

Ser Leu Arg Phe Gln Glu Pro Pro Pro Gly Gly Ala Gly Pro Pro Glu
145                 150                 155                 160

Leu Ala Leu Leu Val Leu Tyr Pro Gly Pro Gly Pro Glu Val Thr Val
                165                 170                 175

Thr Arg Ala Gly Leu Pro Gly Ala Gln Ser Leu Cys Pro Ser Arg Asp
            180                 185                 190

Thr Arg Tyr Leu Val Leu Ala Val Asp Arg Pro Ala Gly Ala Trp Arg
        195                 200                 205

Gly Ser Gly Leu Ala Leu Thr Leu Gln Pro Arg Gly Glu Asp Ser Arg
    210                 215                 220

Leu Ser Thr Ala Arg Leu Gln Ala Leu Leu Phe Gly Asp Asp His Arg
225                 230                 235                 240

Cys Phe Thr Arg Met Thr Pro Ala Leu Leu Leu Leu Pro Arg Ser
                245                 250                 255

<210>  9
<211>  237
<212>  PRT
<213>  artificial sequence

<220>
<223>  polypeptide

<400>  9

Leu Leu Gly Thr Glu Ala Leu Arg Ala Glu Glu Pro Ala Val Gly Thr
1               5                   10                  15

Ser Gly Leu Ile Phe Arg Glu Asp Leu Asp Trp Pro Pro Gly Ser Pro
            20                  25                  30

Gln Glu Pro Leu Cys Leu Val Ala Leu Gly Gly Asp Ser Asn Gly Ser
        35                  40                  45

Ser Ser Pro Leu Arg Val Val Gly Ala Leu Ser Ala Tyr Glu Gln Ala
    50                  55                  60

Phe Leu Gly Ala Val Gln Arg Ala Arg Trp Gly Pro Arg Asp Leu Ala
65                  70                  75                  80

Thr Phe Gly Val Cys Asn Thr Gly Asp Arg Gln Ala Ala Leu Pro Ser
                85                  90                  95

Leu Arg Arg Leu Gly Ala Trp Leu Arg Asp Pro Gly Gly Gln Arg Leu
            100                 105                 110

Val Val Leu His Leu Glu Glu Val Thr Trp Glu Pro Thr Pro Ser Leu
        115                 120                 125

Arg Phe Gln Glu Pro Pro Pro Gly Gly Ala Gly Pro Pro Glu Leu Ala
    130                 135                 140

Leu Leu Val Leu Tyr Pro Gly Pro Gly Pro Glu Val Thr Val Thr Arg
145                 150                 155                 160

Ala Gly Leu Pro Gly Ala Gln Ser Leu Cys Pro Ser Arg Asp Thr Arg
                165                 170                 175

Tyr Leu Val Leu Ala Val Asp Arg Pro Ala Gly Ala Trp Arg Gly Ser
            180                 185                 190

Gly Leu Ala Leu Thr Leu Gln Pro Arg Gly Glu Asp Ser Arg Leu Ser
        195                 200                 205

Thr Ala Arg Leu Gln Ala Leu Leu Phe Gly Asp Asp His Arg Cys Phe
    210                 215                 220

Thr Arg Met Thr Pro Ala Leu Leu Leu Leu Pro Arg Ser
225                 230                 235

<210>  10
<211>  230
<212>  PRT
<213>  artificial sequence

<220>
<223>  polypeptide

<400>  10

Arg Ala Glu Glu Pro Ala Val Gly Thr Ser Gly Leu Ile Phe Arg Glu
1               5                   10                  15

Asp Leu Asp Trp Pro Pro Gly Ser Pro Gln Glu Pro Leu Cys Leu Val
            20                  25                  30

Ala Leu Gly Gly Asp Ser Asn Gly Ser Ser Ser Pro Leu Arg Val Val
        35                  40                  45

Gly Ala Leu Ser Ala Tyr Glu Gln Ala Phe Leu Gly Ala Val Gln Arg
    50                  55                  60

Ala Arg Trp Gly Pro Arg Asp Leu Ala Thr Phe Gly Val Cys Asn Thr
65                  70                  75                  80

Gly Asp Arg Gln Ala Ala Leu Pro Ser Leu Arg Arg Leu Gly Ala Trp
                85                  90                  95

Leu Arg Asp Pro Gly Gly Gln Arg Leu Val Val Leu His Leu Glu Glu
            100                 105                 110

Val Thr Trp Glu Pro Thr Pro Ser Leu Arg Phe Gln Glu Pro Pro Pro
        115                 120                 125

Gly Gly Ala Gly Pro Pro Glu Leu Ala Leu Leu Val Leu Tyr Pro Gly
    130                 135                 140

Pro Gly Pro Glu Val Thr Val Thr Arg Ala Gly Leu Pro Gly Ala Gln
145                 150                 155                 160

Ser Leu Cys Pro Ser Arg Asp Thr Arg Tyr Leu Val Leu Ala Val Asp
                165                 170                 175

Arg Pro Ala Gly Ala Trp Arg Gly Ser Gly Leu Ala Leu Thr Leu Gln
            180                 185                 190

Pro Arg Gly Glu Asp Ser Arg Leu Ser Thr Ala Arg Leu Gln Ala Leu
        195                 200                 205

Leu Phe Gly Asp Asp His Arg Cys Phe Thr Arg Met Thr Pro Ala Leu
    210                 215                 220

Leu Leu Leu Pro Arg Ser
225                 230

<210>  11
<211>  156

<212>  PRT
<213>  artificial sequence

<220>
<223>  polypeptide

<400>  11

Met Arg Asp Leu Pro Leu Thr Ser Leu Ala Leu Val Leu Ser Ala Leu
1               5                   10                  15

Gly Ala Leu Leu Gly Thr Glu Ala Leu Arg Ala Glu Glu Pro Ala Val
            20                  25                  30

Gly Thr Ser Gly Leu Ile Phe Arg Glu Asp Leu Asp Trp Pro Pro Gly
        35                  40                  45

Ser Pro Gln Glu Pro Leu Cys Leu Val Ala Leu Gly Gly Asp Ser Asn
    50                  55                  60

Gly Ser Ser Ser Pro Leu Arg Val Val Gly Ala Leu Ser Ala Tyr Glu
65                  70                  75                  80

Gln Ala Phe Leu Gly Ala Val Gln Arg Ala Arg Trp Gly Pro Arg Asp
                85                  90                  95

Leu Ala Thr Phe Gly Val Cys Asn Thr Gly Asp Arg Gln Ala Ala Leu
            100                 105                 110

Pro Ser Leu Arg Arg Leu Gly Ala Trp Leu Arg Asp Pro Gly Gly Gln
        115                 120                 125

Arg Leu Val Val Leu His Leu Glu Glu Val Thr Trp Glu Pro Thr Pro
    130                 135                 140

Ser Leu Arg Phe Gln Glu Pro Pro Pro Gly Gly Ala
145                 150                 155


<210>  12
<211>  138
<212>  PRT
<213>  artificial sequence

<220>
<223>  polypeptide

<400>  12

Leu Leu Gly Thr Glu Ala Leu Arg Ala Glu Glu Pro Ala Val Gly Thr
1               5                   10                  15

Ser Gly Leu Ile Phe Arg Glu Asp Leu Asp Trp Pro Pro Gly Ser Pro

20                  25                  30

Gln Glu Pro Leu Cys Leu Val Ala Leu Gly Gly Asp Ser Asn Gly Ser
        35                  40                  45

Ser Ser Pro Leu Arg Val Val Gly Ala Leu Ser Ala Tyr Glu Gln Ala
    50                  55                  60

Phe Leu Gly Ala Val Gln Arg Ala Arg Trp Gly Pro Arg Asp Leu Ala
65                  70                  75                  80

Thr Phe Gly Val Cys Asn Thr Gly Asp Arg Gln Ala Ala Leu Pro Ser
                85                  90                  95

Leu Arg Arg Leu Gly Ala Trp Leu Arg Asp Pro Gly Gly Gln Arg Leu
            100                 105                 110

Val Val Leu His Leu Glu Glu Val Thr Trp Glu Pro Thr Pro Ser Leu
        115                 120                 125

Arg Phe Gln Glu Pro Pro Pro Gly Gly Ala
    130                 135

<210>  13
<211>  131
<212>  PRT
<213>  artificial sequence

<220>
<223>  polypeptide

<400>  13

Arg Ala Glu Glu Pro Ala Val Gly Thr Ser Gly Leu Ile Phe Arg Glu
1               5                   10                  15

Asp Leu Asp Trp Pro Pro Gly Ser Pro Gln Glu Pro Leu Cys Leu Val
            20                  25                  30

Ala Leu Gly Gly Asp Ser Asn Gly Ser Ser Ser Pro Leu Arg Val Val
        35                  40                  45

Gly Ala Leu Ser Ala Tyr Glu Gln Ala Phe Leu Gly Ala Val Gln Arg
    50                  55                  60

Ala Arg Trp Gly Pro Arg Asp Leu Ala Thr Phe Gly Val Cys Asn Thr
65                  70                  75                  80

Gly Asp Arg Gln Ala Ala Leu Pro Ser Leu Arg Arg Leu Gly Ala Trp
                85                  90                  95

Leu Arg Asp Pro Gly Gly Gln Arg Leu Val Val Leu His Leu Glu Glu
            100                 105                 110

Val Thr Trp Glu Pro Thr Pro Ser Leu Arg Phe Gln Glu Pro Pro Pro
        115                 120                 125

Gly Gly Ala
    130

<210>  14
<211>  99
<212>  PRT
<213>  artificial sequence

<220>
<223>  polypeptide

<400>  14

Asn Pro Leu Glu Pro Ala Leu Leu Val Leu Tyr Ala Gly Pro Gly Pro
1               5                   10                  15

Glu Val Thr Val Thr Gly Ala Gly Leu Pro Gly Ala Gln Ser Leu Cys
            20                  25                  30

Pro Ser Pro Asp Thr Arg Tyr Leu Ala Leu Ala Val Asp His Pro Ala
        35                  40                  45

Arg Ala Trp Arg His Pro Gly Leu Ile Leu Thr Leu Gln Pro Arg Gly
    50                  55                  60

Asp Gly Ala Pro Leu Ser Thr Ala Gln Leu Gln Thr Leu Leu Phe Gly
65                  70                  75                  80

Ala Asp Pro Arg Cys Phe Thr Arg Met Thr Pro Ala Leu Phe Leu Leu
                85                  90                  95

Gln Arg Pro

<210>  15
<211>  573
<212>  PRT
<213>  cheval

<400>  15
   Met Arg Ala Pro Ser Leu Ser Trp Leu Ala Leu Val Leu Ser Ala Val
   1               5                   10                  15
   Gly Ala Leu Leu Arg Ala Gly Thr Pro Gly Glu Glu Val Ser Ser Thr
           20                  25                  30
   Pro Ala Leu Pro Gly Glu Pro Ala Thr Gly Thr Gly Gly Leu Ile Phe
       35                  40                  45
   His Gln Asp Trp Asp Trp Pro Pro Gly Ser Pro Gln Glu Pro Leu Gly
       50                  55                  60
   Cys Leu Val Thr Leu Asp Glu Gly Gly Asn Gln Ser Ser Ala Pro Leu
   65                  70                  75                  80
   Arg Val Ala Gly Ala Leu Ser Gly Tyr Glu Gln Ala Phe Leu Glu Ala
                   85                  90                  95
   Val Gln Arg Thr His Trp Ser Pro Arg Asp Leu Pro Thr Phe Gly Val
               100                 105                 110
   Cys Ser Pro Ala Asp Arg Gln Ala Ala Val Pro Ser Leu Gln Arg Leu
           115                 120                 125
   Gln Ala Trp Leu Gly Ala Pro Trp Gly Gln Arg Leu Val Val Leu His
       130                 135                 140
   Leu Glu Glu Val Met Trp Glu Pro Thr Pro Ser Leu Arg Phe Gln Glu
   145                 150                 155                 160
   Pro Pro Ser Gly Gly Ala Asn Pro Leu Glu Pro Ala Leu Leu Val Leu
                   165                 170                 175
   Tyr Ala Gly Pro Gly Pro Glu Val Thr Val Thr Gly Ala Gly Leu Pro
               180                 185                 190
   Gly Ala Gln Ser Leu Cys Pro Ser Pro Asp Thr Arg Tyr Leu Ala Leu
           195                 200                 205
   Ala Val Asp His Pro Ala Arg Ala Trp Arg His Pro Gly Leu Ile Leu
       210                 215                 220
   Thr Leu Gln Pro Arg Gly Asp Gly Ala Pro Leu Ser Thr Ala Gln Leu
   225                 230                 235                 240
   Gln Thr Leu Leu Phe Gly Ala Asp Pro Arg Cys Phe Thr Arg Met Thr
                   245                 250                 255
   Pro Ala Leu Phe Leu Leu Gln Arg Pro Gly Pro Ala Pro Met Pro Ala
               260                 265                 270
   His Gly Arg Leu Asp Thr Val Pro Phe Pro Pro Ala Arg Pro Ser Pro
           275                 280                 285
   Glu Pro Glu Glu Pro Arg Pro Ser Ala Asp Pro Phe Leu Glu Thr Leu
       290                 295                 300
   Thr Arg Leu Val Arg Ala Leu Arg Gly Pro Pro Thr Pro Ala Ser Pro
   305                 310                 315                 320
   Pro Arg Leu Ala Leu Asp Pro Gly Ala Leu Ala Ser Phe Pro Gln Gly
                   325                 330                 335
   Leu Val Asn Leu Ser Asp Pro Ala Ala Leu Glu Arg Leu Leu Asp Gly
               340                 345                 350
   Glu Glu Pro Leu Leu Leu Leu Leu Pro Pro Thr Ala Ala Ala Ala Gly
           355                 360                 365
   Asp Pro Ala Pro Leu Pro Asp Pro Ala Ser Ala Pro Trp Ala Ala Gly
       370                 375                 380
   Leu Ala Arg Arg Val Ala Ala Glu Leu Gln Ala Ala Ala Ala Glu Leu
   385                 390                 395                 400
   Arg Ser Leu Pro Gly Leu Pro Pro Ala Ala Glu Pro Leu Leu Ala Arg
                   405                 410                 415
   Leu Leu Ala Leu Cys Pro Gly Asp Ala Glu Asp Gln Gly Gly Pro Gly
               420                 425                 430
   Gly Pro Leu Arg Ala Leu Leu Leu Leu Lys Ala Leu Gln Gly Leu Arg
           435                 440                 445
   Ala Glu Trp Arg Gly Arg Glu Arg Ser Gly Pro Gly Arg Ala Gln Arg
       450                 455                 460
   Asn Ala Gly Ala Gly Ala Ala Asp Gly Pro Cys Ala Leu Arg Glu Leu
   465                 470                 475                 480
   Arg Val Asp Leu Arg Ala Glu Arg Ser Val Leu Ile Pro Glu Thr Tyr
                   485                 490                 495
   Gln Ala Asn Asn Cys Gln Gly Ala Cys Gly Trp Pro Gln Ser Asp Arg
               500                 505                 510
   Asn Pro Arg Tyr Gly Asn His Val Val Leu Leu Leu Lys Met Gln Ala
           515                 520                 525
   Arg Gly Ala Ala Leu Ala Arg Ala Pro Cys Cys Val Pro Thr Ala Tyr
       530                 535                 540
   Ala Gly Lys Leu Leu Ile Ser Leu Ser Glu Glu Arg Ile Ser Ala His
   545                 550                 555                 560
   His Val Pro Asn Met Val Ala Thr Glu Cys Gly Cys Arg
                   565                 570

<210>  16
<211>  551
<212>  PRT
<213>  artificial sequence

<220>
<223>  polypeptide

<400>  16

Gly Thr Pro Gly Glu Glu Val Ser Ser Thr Pro Ala Leu Pro Gly Glu
1               5                   10                  15

Pro Ala Thr Gly Thr Gly Gly Leu Ile Phe His Gln Asp Trp Asp Trp
            20                  25                  30

Pro Pro Gly Ser Pro Gln Glu Pro Leu Gly Cys Leu Val Thr Leu Asp
        35                  40                  45

Glu Gly Gly Asn Gln Ser Ser Ala Pro Leu Arg Val Ala Gly Ala Leu
    50                  55                  60

Ser Gly Tyr Glu Gln Ala Phe Leu Glu Ala Val Gln Arg Thr His Trp
65                  70                  75                  80

Ser Pro Arg Asp Leu Pro Thr Phe Gly Val Cys Ser Pro Ala Asp Arg
                85                  90                  95

Gln Ala Ala Val Pro Ser Leu Gln Arg Leu Gln Ala Trp Leu Gly Ala
            100                 105                 110

Pro Trp Gly Gln Arg Leu Val Val Leu His Leu Glu Glu Val Met Trp
        115                 120                 125

Glu Pro Thr Pro Ser Leu Arg Phe Gln Glu Pro Pro Ser Gly Gly Ala
    130                 135                 140

Asn Pro Leu Glu Pro Ala Leu Leu Val Leu Tyr Ala Gly Pro Gly Pro
145                 150                 155                 160

Glu Val Thr Val Thr Gly Ala Gly Leu Pro Gly Ala Gln Ser Leu Cys
                165                 170                 175

Pro Ser Pro Asp Thr Arg Tyr Leu Ala Leu Ala Val Asp His Pro Ala
            180                 185                 190

Arg Ala Trp Arg His Pro Gly Leu Ile Leu Thr Leu Gln Pro Arg Gly
        195                 200                 205

Asp Gly Ala Pro Leu Ser Thr Ala Gln Leu Gln Thr Leu Leu Phe Gly
    210                 215                 220

Ala Asp Pro Arg Cys Phe Thr Arg Met Thr Pro Ala Leu Phe Leu Leu
225                 230                 235                 240

Gln Arg Pro Gly Pro Ala Pro Met Pro Ala His Gly Arg Leu Asp Thr
                245                 250                 255

Val Pro Phe Pro Pro Ala Arg Pro Ser Pro Glu Pro Glu Glu Pro Arg
            260                 265                 270

Pro Ser Ala Asp Pro Phe Leu Glu Thr Leu Thr Arg Leu Val Arg Ala
        275                 280                 285

Leu Arg Gly Pro Pro Thr Pro Ala Ser Pro Pro Arg Leu Ala Leu Asp
    290                 295                 300

Pro Gly Ala Leu Ala Ser Phe Pro Gln Gly Leu Val Asn Leu Ser Asp
305                 310                 315                 320

Pro Ala Ala Leu Glu Arg Leu Leu Asp Gly Glu Glu Pro Leu Leu Leu
                325                 330                 335

Leu Leu Pro Pro Thr Ala Ala Ala Ala Gly Asp Pro Ala Pro Leu Pro
            340                 345                 350

Asp Pro Ala Ser Ala Pro Trp Ala Ala Gly Leu Ala Arg Arg Val Ala
        355                 360                 365

Ala Glu Leu Gln Ala Ala Ala Ala Glu Leu Arg Ser Leu Pro Gly Leu
    370                 375                 380

Pro Pro Ala Ala Glu Pro Leu Leu Ala Arg Leu Leu Ala Leu Cys Pro
385                 390                 395                 400

Gly Asp Ala Glu Asp Gln Gly Gly Pro Gly Gly Pro Leu Arg Ala Leu
                405                 410                 415

Leu Leu Leu Lys Ala Leu Gln Gly Leu Arg Ala Glu Trp Arg Gly Arg
            420                 425                 430

Glu Arg Ser Gly Pro Gly Arg Ala Gln Arg Asn Ala Gly Ala Gly Ala
        435                 440                 445

Ala Asp Gly Pro Cys Ala Leu Arg Glu Leu Arg Val Asp Leu Arg Ala
    450                 455                 460

Glu Arg Ser Val Leu Ile Pro Glu Thr Tyr Gln Ala Asn Asn Cys Gln
465                 470                 475                 480

Gly Ala Cys Gly Trp Pro Gln Ser Asp Arg Asn Pro Arg Tyr Gly Asn
                485                 490                 495

His Val Val Leu Leu Leu Lys Met Gln Ala Arg Gly Ala Ala Leu Ala
            500                 505                 510

Arg Ala Pro Cys Cys Val Pro Thr Ala Tyr Ala Gly Lys Leu Leu Ile
        515                 520                 525

Ser Leu Ser Glu Glu Arg Ile Ser Ala His His Val Pro Asn Met Val
    530                 535                 540

Ala Thr Glu Cys Gly Cys Arg
545                 550

<210>  17
<211>  464
<212>  PRT
<213>  artificial sequence

<220>
<223>  polypeptide

<400>  17
   Met Arg Ala Pro Ser Leu Ser Trp Leu Ala Leu Val Leu Ser Ala Val
   1               5                   10                  15
   Gly Ala Leu Leu Arg Ala Gly Thr Pro Gly Glu Glu Val Ser Ser Thr
               20                  25                  30
   Pro Ala Leu Pro Gly Glu Pro Ala Thr Gly Thr Gly Gly Leu Ile Phe
           35                  40                  45
   His Gln Asp Trp Asp Trp Pro Pro Gly Ser Pro Gln Glu Pro Leu Gly
       50                  55                  60
   Cys Leu Val Thr Leu Asp Glu Gly Gly Asn Gln Ser Ser Ala Pro Leu
   65                  70                  75                  80
   Arg Val Ala Gly Ala Leu Ser Gly Tyr Glu Gln Ala Phe Leu Glu Ala
                   85                  90                  95
   Val Gln Arg Thr His Trp Ser Pro Arg Asp Leu Pro Thr Phe Gly Val
               100                 105                 110
   Cys Ser Pro Ala Asp Arg Gln Ala Ala Val Pro Ser Leu Gln Arg Leu
           115                 120                 125
   Gln Ala Trp Leu Gly Ala Pro Trp Gly Gln Arg Leu Val Val Leu His
       130                 135                 140
   Leu Glu Glu Val Met Trp Glu Pro Thr Pro Ser Leu Arg Phe Gln Glu
   145                 150                 155                 160
   Pro Pro Ser Gly Gly Ala Asn Pro Leu Glu Pro Ala Leu Leu Val Leu
                   165                 170                 175
   Tyr Ala Gly Pro Gly Pro Glu Val Thr Val Thr Gly Ala Gly Leu Pro
               180                 185                 190
   Gly Ala Gln Ser Leu Cys Pro Ser Pro Asp Thr Arg Tyr Leu Ala Leu
           195                 200                 205
   Ala Val Asp His Pro Ala Arg Ala Trp Arg His Pro Gly Leu Ile Leu
       210                 215                 220
   Thr Leu Gln Pro Arg Gly Asp Gly Ala Pro Leu Ser Thr Ala Gln Leu
   225                 230                 235                 240
   Gln Thr Leu Leu Phe Gly Ala Asp Pro Arg Cys Phe Thr Arg Met Thr
                   245                 250                 255
   Pro Ala Leu Phe Leu Leu Gln Arg Pro Gly Pro Ala Pro Met Pro Ala
               260                 265                 270
   His Gly Arg Leu Asp Thr Val Pro Phe Pro Pro Ala Arg Pro Ser Pro
           275                 280                 285
   Glu Pro Glu Glu Pro Arg Pro Ser Ala Asp Pro Phe Leu Glu Thr Leu
       290                 295                 300
   Thr Arg Leu Val Arg Ala Leu Arg Gly Pro Pro Thr Pro Ala Ser Pro
   305                 310                 315                 320
   Pro Arg Leu Ala Leu Asp Pro Gly Ala Leu Ala Ser Phe Pro Gln Gly
   325 330 335
   Leu Val Asn Leu Ser Asp Pro Ala Ala Leu Glu Arg Leu Leu Asp Gly
   340 345 350
   Glu Glu Pro Leu Leu Leu Leu Leu Pro Pro Thr Ala Ala Ala Ala Gly
   355 360 365
   Asp Pro Ala Pro Leu Pro Asp Pro Ala Ser Ala Pro Trp Ala Ala Gly
   370 375 380
   Leu Ala Arg Arg Val Ala Ala Glu Leu Gln Ala Ala Ala Ala Glu Leu
   385 390 395 400
   Arg Ser Leu Pro Gly Leu Pro Pro Ala Ala Glu Pro Leu Leu Ala Arg
   405 410 415
   Leu Leu Ala Leu Cys Pro Gly Asp Ala Glu Asp Gln Gly Gly Pro Gly
   420 425 430
   Gly Pro Leu Arg Ala Leu Leu Leu Leu Lys Ala Leu Gln Gly Leu Arg
   435 440 445
   Ala Glu Trp Arg Gly Arg Glu Arg Ser Gly Pro Gly Arg Ala Gln Arg
   450 455 460

<210>  18
<211>  442
<212>  PRT
<213>  artificial sequence

<220>
<223>  polypeptide

<400>  18

Gly Thr Pro Gly Glu Glu Val Ser Ser Thr Pro Ala Leu Pro Gly Glu
1               5                   10                  15

Pro Ala Thr Gly Thr Gly Gly Leu Ile Phe His Gln Asp Trp Asp Trp
            20                  25                  30

Pro Pro Gly Ser Pro Gln Glu Pro Leu Gly Cys Leu Val Thr Leu Asp
        35                  40                  45

Glu Gly Gly Asn Gln Ser Ser Ala Pro Leu Arg Val Ala Gly Ala Leu
    50                  55                  60

Ser Gly Tyr Glu Gln Ala Phe Leu Glu Ala Val Gln Arg Thr His Trp
65                  70                  75                  80

Ser Pro Arg Asp Leu Pro Thr Phe Gly Val Cys Ser Pro Ala Asp Arg
                85                  90                  95

Gln Ala Ala Val Pro Ser Leu Gln Arg Leu Gln Ala Trp Leu Gly Ala
            100                 105                 110

Pro Trp Gly Gln Arg Leu Val Val Leu His Leu Glu Glu Val Met Trp
        115                 120                 125

Glu Pro Thr Pro Ser Leu Arg Phe Gln Glu Pro Pro Ser Gly Gly Ala
    130                 135                 140

Asn Pro Leu Glu Pro Ala Leu Leu Val Leu Tyr Ala Gly Pro Gly Pro
145                 150                 155                 160

Glu Val Thr Val Thr Gly Ala Gly Leu Pro Gly Ala Gln Ser Leu Cys
                165                 170                 175

Pro Ser Pro Asp Thr Arg Tyr Leu Ala Leu Ala Val Asp His Pro Ala
            180                 185                 190

Arg Ala Trp Arg His Pro Gly Leu Ile Leu Thr Leu Gln Pro Arg Gly
        195                 200                 205

Asp Gly Ala Pro Leu Ser Thr Ala Gln Leu Gln Thr Leu Leu Phe Gly
    210                 215                 220

Ala Asp Pro Arg Cys Phe Thr Arg Met Thr Pro Ala Leu Phe Leu Leu
225                 230                 235                 240

Gln Arg Pro Gly Pro Ala Pro Met Pro Ala His Gly Arg Leu Asp Thr
                245                 250                 255

Val Pro Phe Pro Pro Ala Arg Pro Ser Pro Glu Pro Glu Glu Pro Arg
            260                 265                 270

Pro Ser Ala Asp Pro Phe Leu Glu Thr Leu Thr Arg Leu Val Arg Ala
        275                 280                 285

Leu Arg Gly Pro Pro Thr Pro Ala Ser Pro Pro Arg Leu Ala Leu Asp
    290                 295                 300

Pro Gly Ala Leu Ala Ser Phe Pro Gln Gly Leu Val Asn Leu Ser Asp
305                 310                 315                 320

Pro Ala Ala Leu Glu Arg Leu Leu Asp Gly Glu Glu Pro Leu Leu Leu
                325                 330                 335

Leu Leu Pro Pro Thr Ala Ala Ala Ala Gly Asp Pro Ala Pro Leu Pro
            340                 345                 350

Asp Pro Ala Ser Ala Pro Trp Ala Ala Gly Leu Ala Arg Arg Val Ala
        355                 360                 365

Ala Glu Leu Gln Ala Ala Ala Ala Glu Leu Arg Ser Leu Pro Gly Leu
    370                 375                 380

Pro Pro Ala Ala Glu Pro Leu Leu Ala Arg Leu Leu Ala Leu Cys Pro
385                 390                 395                 400

Gly Asp Ala Glu Asp Gln Gly Gly Pro Gly Gly Pro Leu Arg Ala Leu
                405                 410                 415

Leu Leu Leu Lys Ala Leu Gln Gly Leu Arg Ala Glu Trp Arg Gly Arg
            420                 425                 430

Glu Arg Ser Gly Pro Gly Arg Ala Gln Arg
        435                 440

<210> 19
<211> 265
<212> PRT
<213> artificial sequence

<220>
<223> polypeptide

<400> 19

Met Arg Ala Pro Ser Leu Ser Trp Leu Ala Leu Val Leu Ser Ala Val
1               5                   10                  15

Gly Ala Leu Leu Arg Ala Gly Thr Pro Gly Glu Glu Val Ser Ser Thr
            20                  25                  30

Pro Ala Leu Pro Gly Glu Pro Ala Thr Gly Thr Gly Gly Leu Ile Phe
        35                  40                  45

His Gln Asp Trp Asp Trp Pro Pro Gly Ser Pro Gln Glu Pro Leu Gly
    50                  55                  60

Cys Leu Val Thr Leu Asp Glu Gly Gly Asn Gln Ser Ser Ala Pro Leu
65                  70                  75                  80

Arg Val Ala Gly Ala Leu Ser Gly Tyr Glu Gln Ala Phe Leu Glu Ala
                85                  90                  95

Val Gln Arg Thr His Trp Ser Pro Arg Asp Leu Pro Thr Phe Gly Val
            100                 105                 110

Cys Ser Pro Ala Asp Arg Gln Ala Ala Val Pro Ser Leu Gln Arg Leu
        115                 120                 125

Gln Ala Trp Leu Gly Ala Pro Trp Gly Gln Arg Leu Val Val Leu His
    130                 135                 140

Leu Glu Glu Val Met Trp Glu Pro Thr Pro Ser Leu Arg Phe Gln Glu
145                 150                 155                 160

Pro Pro Ser Gly Gly Ala Asn Pro Leu Glu Pro Ala Leu Leu Val Leu
                165                 170                 175

Tyr Ala Gly Pro Gly Pro Glu Val Thr Val Thr Gly Ala Gly Leu Pro
            180                 185                 190

Gly Ala Gln Ser Leu Cys Pro Ser Pro Asp Thr Arg Tyr Leu Ala Leu
        195                 200                 205

Ala Val Asp His Pro Ala Arg Ala Trp Arg His Pro Gly Leu Ile Leu
    210                 215                 220

Thr Leu Gln Pro Arg Gly Asp Gly Ala Pro Leu Ser Thr Ala Gln Leu
225                 230                 235                 240

Gln Thr Leu Leu Phe Gly Ala Asp Pro Arg Cys Phe Thr Arg Met Thr
                245                 250                 255

Pro Ala Leu Phe Leu Leu Gln Arg Pro
            260                 265

<210>  20
<211>  243
<212>  PRT
<213>  artificial sequence

<220>
<223>  polypeptide

<400>  20

Gly Thr Pro Gly Glu Glu Val Ser Ser Thr Pro Ala Leu Pro Gly Glu
1               5                   10                  15

Pro Ala Thr Gly Thr Gly Gly Leu Ile Phe His Gln Asp Trp Asp Trp
            20                  25                  30

Pro Pro Gly Ser Pro Gln Glu Pro Leu Gly Cys Leu Val Thr Leu Asp
        35                  40                  45

Glu Gly Gly Asn Gln Ser Ser Ala Pro Leu Arg Val Ala Gly Ala Leu
    50                  55                  60

Ser Gly Tyr Glu Gln Ala Phe Leu Glu Ala Val Gln Arg Thr His Trp
65                  70                  75                  80

Ser Pro Arg Asp Leu Pro Thr Phe Gly Val Cys Ser Pro Ala Asp Arg
                85                  90                  95

Gln Ala Ala Val Pro Ser Leu Gln Arg Leu Gln Ala Trp Leu Gly Ala
            100                 105                 110

Pro Trp Gly Gln Arg Leu Val Val Leu His Leu Glu Glu Val Met Trp
        115                 120                 125

Glu Pro Thr Pro Ser Leu Arg Phe Gln Glu Pro Pro Ser Gly Gly Ala
    130                 135                 140

Asn Pro Leu Glu Pro Ala Leu Leu Val Leu Tyr Ala Gly Pro Gly Pro
145                 150                 155                 160

Glu Val Thr Val Thr Gly Ala Gly Leu Pro Gly Ala Gln Ser Leu Cys
                165                 170                 175

Pro Ser Pro Asp Thr Arg Tyr Leu Ala Leu Ala Val Asp His Pro Ala
            180                 185                 190

Arg Ala Trp Arg His Pro Gly Leu Ile Leu Thr Leu Gln Pro Arg Gly
        195                 200                 205

Asp Gly Ala Pro Leu Ser Thr Ala Gln Leu Gln Thr Leu Leu Phe Gly
    210                 215                 220

Ala Asp Pro Arg Cys Phe Thr Arg Met Thr Pro Ala Leu Phe Leu Leu
225                 230                 235                 240

Gln Arg Pro

<210>  21
<211>  166
<212>  PRT

<213>  artificial sequence

<220>
<223>  polypeptide

<400>  21

Met Arg Ala Pro Ser Leu Ser Trp Leu Ala Leu Val Leu Ser Ala Val
1               5                   10                  15

Gly Ala Leu Leu Arg Ala Gly Thr Pro Gly Glu Glu Val Ser Ser Thr
            20                  25                  30

Pro Ala Leu Pro Gly Glu Pro Ala Thr Gly Thr Gly Gly Leu Ile Phe
        35                  40                  45

His Gln Asp Trp Asp Trp Pro Pro Gly Ser Pro Gln Glu Pro Leu Gly
    50                  55                  60

Cys Leu Val Thr Leu Asp Glu Gly Gly Asn Gln Ser Ser Ala Pro Leu
65                  70                  75                  80

Arg Val Ala Gly Ala Leu Ser Gly Tyr Glu Gln Ala Phe Leu Glu Ala
                85                  90                  95

Val Gln Arg Thr His Trp Ser Pro Arg Asp Leu Pro Thr Phe Gly Val
            100                 105                 110

Cys Ser Pro Ala Asp Arg Gln Ala Ala Val Pro Ser Leu Gln Arg Leu
        115                 120                 125

Gln Ala Trp Leu Gly Ala Pro Trp Gly Gln Arg Leu Val Val Leu His
    130                 135                 140

Leu Glu Glu Val Met Trp Glu Pro Thr Pro Ser Leu Arg Phe Gln Glu
145                 150                 155                 160

Pro Pro Ser Gly Gly Ala
                165


<210>  22
<211>  144
<212>  PRT
<213>  artificial sequence

<220>
<223>  polypeptide

<400>  22

Gly Thr Pro Gly Glu Glu Val Ser Ser Thr Pro Ala Leu Pro Gly Glu
1               5                   10                  15

Pro Ala Thr Gly Thr Gly Gly Leu Ile Phe His Gln Asp Trp Asp Trp
            20                  25                  30

Pro Pro Gly Ser Pro Gln Glu Pro Leu Gly Cys Leu Val Thr Leu Asp
        35                  40                  45

Glu Gly Gly Asn Gln Ser Ser Ala Pro Leu Arg Val Ala Gly Ala Leu
    50                  55                  60

Ser Gly Tyr Glu Gln Ala Phe Leu Glu Ala Val Gln Arg Thr His Trp
65                  70                  75                  80

Ser Pro Arg Asp Leu Pro Thr Phe Gly Val Cys Ser Pro Ala Asp Arg
                85                  90                  95

Gln Ala Ala Val Pro Ser Leu Gln Arg Leu Gln Ala Trp Leu Gly Ala
            100                 105                 110

Pro Trp Gly Gln Arg Leu Val Val Leu His Leu Glu Glu Val Met Trp
        115                 120                 125

Glu Pro Thr Pro Ser Leu Arg Phe Gln Glu Pro Pro Ser Gly Gly Ala
    130                 135                 140

<210> 23
<211> 99
<212> PRT
<213> artificial sequence

<220>
<223> polypeptide

<400> 23

Ser Pro Leu Glu Leu Ala Leu Leu Val Leu Tyr Pro Gly Pro Gly Pro
1               5                   10                  15

Glu Val Ala Val Thr Gly Ala Gly Leu Pro Gly Thr Gln Asn Leu Cys
            20                  25                  30

Arg Ser Arg Asn Thr Arg Tyr Leu Val Leu Ala Leu Asp His Pro Val
        35                  40                  45

Gly Ala Trp His Ser Pro Arg Val Thr Leu Thr Val His Ala Arg Gly
    50                  55                  60

Asp Gly Ala Pro Leu Ser Thr Pro Gln Leu Gln Glu Leu Leu Phe Gly
65                  70                  75                  80

Pro Asp Ala Arg Cys Phe Thr Arg Met Thr Pro Ala Leu Leu Val Leu
                85                  90                  95

Arg Leu Pro

<210>  24
<211>  572
<212>  PRT
<213>  chien

<400>  24

Met Gly Ala Leu Ala Leu Trp Pro Leu Ala Leu Ala Leu Ser Gly Met
1               5                   10                  15

Gly Pro Leu Leu Gly Ala Glu Ala Pro Gly Gly Glu Val Ser Gly Thr
            20                  25                  30

Pro Ala Ser Pro Gly Glu Pro Ala Thr Gly Thr Gly Gly Leu Leu Phe
        35                  40                  45

Gln Pro Asp Trp Asp Trp Pro Pro Ser Ala Pro Gln Asp Pro Leu Cys
    50                  55                  60

Leu Val Thr Leu Asp Lys Gly Gly Asn Gly Ser Ser Pro Pro Leu Arg
65                  70                  75                  80

Val Ala Gly Ala Leu Arg Gly Tyr Glu His Thr Phe Leu Glu Ala Val
                85                  90                  95

Arg Arg Ala Arg Trp Gly Pro His Asp Leu Ala Thr Phe Gly Ala Cys
            100                 105                 110

Ala Ala Ser Asp Gly Arg Thr Thr Gln Leu Ser Leu Arg Gln Leu Gln
        115                 120                 125

Ala Trp Leu Gly Ala Pro Gly Gly Arg Arg Leu Val Val Leu His Leu
    130                 135                 140

Glu Glu Val Thr Trp Glu Pro Ala Leu Ser Leu Lys Phe Gln Glu Pro
145                 150                 155                 160

Pro Pro Gly Gly Ala Ser Pro Leu Glu Leu Ala Leu Leu Val Leu Tyr
                165                 170                 175

Pro Gly Pro Gly Pro Glu Val Ala Val Thr Gly Ala Gly Leu Pro Gly
            180                 185                 190

Thr Gln Asn Leu Cys Arg Ser Arg Asn Thr Arg Tyr Leu Val Leu Ala
        195                 200                 205

Leu Asp His Pro Val Gly Ala Trp His Ser Pro Arg Val Thr Leu Thr
    210                 215                 220

Val His Ala Arg Gly Asp Gly Ala Pro Leu Ser Thr Pro Gln Leu Gln
225                 230                 235                 240

Glu Leu Leu Phe Gly Pro Asp Ala Arg Cys Phe Thr Arg Met Thr Pro
                245                 250                 255

Ala Leu Leu Val Leu Arg Leu Pro Gly Pro Thr Ala Val Pro Ala Arg
            260                 265                 270

Gly Leu Leu Asp Leu Val Pro Phe Pro Pro Pro Arg Pro Ser Arg Glu
        275                 280                 285

Pro Ala Glu Pro Pro Pro Ser Ala Asp Pro Phe Leu Glu Thr Leu Thr
    290                 295                 300

Arg Leu Val Arg Ala Leu Arg Gly Pro Pro Thr Pro Ala Ser Pro Pro
305                 310                 315                 320

Arg Leu Ala Leu Asp Pro Gly Ala Leu Ala Gly Phe Pro Gln Gly Leu
                325                 330                 335

Leu Asn Leu Ser Asp Pro Ala Thr Gln Glu Arg Leu Leu Gly Gly Glu
            340                 345                 350

Glu Pro Leu Leu Leu Leu Leu Pro Pro Pro Thr Ala Ala Ala Gly Pro
        355                 360                 365

Pro Ala Pro Pro Pro Arg Pro Ala Ser Ala Pro Trp Ala Ala Gly Leu
    370                 375                 380

Ala Leu Arg Val Ala Ala Glu Leu Arg Ala Ala Ala Ala Glu Leu Arg
385                 390                 395                 400

Gly Leu Pro Gly Leu Pro Pro Ala Ala Ala Pro Leu Leu Glu Arg Leu
                405                 410                 415

Leu Ala Leu Cys Pro Gly Gly Ser Gly Gly Ser Gly Gly Ser Gly Asp
            420                 425                 430

Pro Leu Arg Ala Leu Leu Leu Leu Lys Ala Leu Gln Gly Leu Arg Ala
        435                 440                 445

Glu Trp Arg Gly Arg Glu Arg Gly Gly Pro Pro Arg Ala Gln Arg Ser
    450                 455                 460

Ala Gly Ala Gly Ala Ala Asp Gly Pro Cys Ala Leu Arg Glu Leu Ser
465                 470                 475                 480

Val Asp Leu Arg Ala Glu Arg Ser Val Leu Ile Pro Glu Thr Tyr Gln
                485                 490                 495

Ala Asn Asn Cys Gln Gly Ala Cys Gly Trp Pro Gln Ser Asp Arg Asn
            500                 505                 510

Pro Arg Tyr Gly Asn His Val Val Leu Leu Leu Lys Met Gln Ala Arg
        515                 520                 525

Gly Ala Ala Leu Ala Arg Pro Pro Cys Cys Val Pro Thr Ala Tyr Gly
    530                 535                 540

Gly Lys Leu Leu Ile Ser Leu Ser Glu Glu Arg Ile Ser Ala His His
545                 550                 555                 560

Val Pro Asn Met Val Ala Thr Glu Cys Gly Cys Arg
                565                 570

<210>  25
<211>  551
<212>  PRT
<213>  artificial sequence

<220>
<223>  polypeptide

<400>  25

Ala Glu Ala Pro Gly Gly Glu Val Ser Gly Thr Pro Ala Ser Pro Gly
1               5                   10                  15

Glu Pro Ala Thr Gly Thr Gly Gly Leu Leu Phe Gln Pro Asp Trp Asp
            20                  25                  30

Trp Pro Pro Ser Ala Pro Gln Asp Pro Leu Cys Leu Val Thr Leu Asp
        35                  40                  45

Lys Gly Gly Asn Gly Ser Ser Pro Pro Leu Arg Val Ala Gly Ala Leu
    50                  55                  60

Arg Gly Tyr Glu His Thr Phe Leu Glu Ala Val Arg Arg Ala Arg Trp
65                  70                  75                  80

Gly Pro His Asp Leu Ala Thr Phe Gly Ala Cys Ala Ala Ser Asp Gly
                    85                  90                  95

Arg Thr Thr Gln Leu Ser Leu Arg Gln Leu Gln Ala Trp Leu Gly Ala
            100                 105                 110

Pro Gly Gly Arg Arg Leu Val Val Leu His Leu Glu Glu Val Thr Trp
        115                 120                 125

Glu Pro Ala Leu Ser Leu Lys Phe Gln Glu Pro Pro Pro Gly Gly Ala
    130                 135                 140

Ser Pro Leu Glu Leu Ala Leu Leu Val Leu Tyr Pro Gly Pro Gly Pro
145                 150                 155                 160

Glu Val Ala Val Thr Gly Ala Gly Leu Pro Gly Thr Gln Asn Leu Cys
                165                 170                 175

Arg Ser Arg Asn Thr Arg Tyr Leu Val Leu Ala Leu Asp His Pro Val
            180                 185                 190

Gly Ala Trp His Ser Pro Arg Val Thr Leu Thr Val His Ala Arg Gly
        195                 200                 205

Asp Gly Ala Pro Leu Ser Thr Pro Gln Leu Gln Glu Leu Leu Phe Gly
    210                 215                 220

Pro Asp Ala Arg Cys Phe Thr Arg Met Thr Pro Ala Leu Leu Val Leu
225                 230                 235                 240

Arg Leu Pro Gly Pro Thr Ala Val Pro Ala Arg Gly Leu Leu Asp Leu
                245                 250                 255

Val Pro Phe Pro Pro Pro Arg Pro Ser Arg Glu Pro Ala Glu Pro Pro
            260                 265                 270

Pro Ser Ala Asp Pro Phe Leu Glu Thr Leu Thr Arg Leu Val Arg Ala
        275                 280                 285

Leu Arg Gly Pro Pro Thr Pro Ala Ser Pro Pro Arg Leu Ala Leu Asp
    290                 295                 300

Pro Gly Ala Leu Ala Gly Phe Pro Gln Gly Leu Leu Asn Leu Ser Asp
305                 310                 315                 320

Pro Ala Thr Gln Glu Arg Leu Leu Gly Gly Glu Glu Pro Leu Leu Leu
                325                 330                 335

Leu Leu Pro Pro Pro Thr Ala Ala Ala Gly Pro Pro Ala Pro Pro Pro
            340                 345                 350

Arg Pro Ala Ser Ala Pro Trp Ala Ala Gly Leu Ala Leu Arg Val Ala
        355                 360                 365

Ala Glu Leu Arg Ala Ala Ala Ala Glu Leu Arg Gly Leu Pro Gly Leu
    370                 375                 380

Pro Pro Ala Ala Ala Pro Leu Leu Glu Arg Leu Leu Ala Leu Cys Pro
385                 390                 395                 400

Gly Gly Ser Gly Gly Ser Gly Gly Ser Gly Asp Pro Leu Arg Ala Leu
                405                 410                 415

Leu Leu Leu Lys Ala Leu Gln Gly Leu Arg Ala Glu Trp Arg Gly Arg
            420                 425                 430

Glu Arg Gly Gly Pro Pro Arg Ala Gln Arg Ser Ala Gly Ala Gly Ala
        435                 440                 445

Ala Asp Gly Pro Cys Ala Leu Arg Glu Leu Ser Val Asp Leu Arg Ala
    450                 455                 460

Glu Arg Ser Val Leu Ile Pro Glu Thr Tyr Gln Ala Asn Asn Cys Gln
465                 470                 475                 480

Gly Ala Cys Gly Trp Pro Gln Ser Asp Arg Asn Pro Arg Tyr Gly Asn
                485                 490                 495

His Val Val Leu Leu Leu Lys Met Gln Ala Arg Gly Ala Ala Leu Ala
            500                 505                 510

Arg Pro Pro Cys Cys Val Pro Thr Ala Tyr Gly Gly Lys Leu Leu Ile
        515                 520                 525

Ser Leu Ser Glu Glu Arg Ile Ser Ala His His Val Pro Asn Met Val
    530                 535                 540

Ala Thr Glu Cys Gly Cys Arg
545                 550

<210> 26
<211> 463
<212> PRT
<213> artificial sequence

<220>
<223>  polypeptide

<400>  26

Met Gly Ala Leu Ala Leu Trp Pro Leu Ala Leu Ala Leu Ser Gly Met
1               5                   10                  15

Gly Pro Leu Leu Gly Ala Glu Ala Pro Gly Gly Glu Val Ser Gly Thr
            20                  25                  30

Pro Ala Ser Pro Gly Glu Pro Ala Thr Gly Thr Gly Gly Leu Leu Phe
        35                  40                  45

Gln Pro Asp Trp Asp Trp Pro Pro Ser Ala Pro Gln Asp Pro Leu Cys
    50                  55                  60

Leu Val Thr Leu Asp Lys Gly Gly Asn Gly Ser Ser Pro Pro Leu Arg
65                  70                  75                  80

Val Ala Gly Ala Leu Arg Gly Tyr Glu His Thr Phe Leu Glu Ala Val
                85                  90                  95

Arg Arg Ala Arg Trp Gly Pro His Asp Leu Ala Thr Phe Gly Ala Cys
            100                 105                 110

Ala Ala Ser Asp Gly Arg Thr Thr Gln Leu Ser Leu Arg Gln Leu Gln
        115                 120                 125

Ala Trp Leu Gly Ala Pro Gly Gly Arg Arg Leu Val Val Leu His Leu
    130                 135                 140

Glu Glu Val Thr Trp Glu Pro Ala Leu Ser Leu Lys Phe Gln Glu Pro
145                 150                 155                 160

Pro Pro Gly Gly Ala Ser Pro Leu Glu Leu Ala Leu Leu Val Leu Tyr
                165                 170                 175

Pro Gly Pro Gly Pro Glu Val Ala Val Thr Gly Ala Gly Leu Pro Gly
            180                 185                 190

Thr Gln Asn Leu Cys Arg Ser Arg Asn Thr Arg Tyr Leu Val Leu Ala
        195                 200                 205

Leu Asp His Pro Val Gly Ala Trp His Ser Pro Arg Val Thr Leu Thr
    210                 215                 220

Val His Ala Arg Gly Asp Gly Ala Pro Leu Ser Thr Pro Gln Leu Gln
225                 230                 235                 240

Glu Leu Leu Phe Gly Pro Asp Ala Arg Cys Phe Thr Arg Met Thr Pro
                245                 250                 255

Ala Leu Leu Val Leu Arg Leu Pro Gly Pro Thr Ala Val Pro Ala Arg
            260                 265                 270

Gly Leu Leu Asp Leu Val Pro Phe Pro Pro Pro Arg Pro Ser Arg Glu
        275                 280                 285

Pro Ala Glu Pro Pro Pro Ser Ala Asp Pro Phe Leu Glu Thr Leu Thr
    290                 295                 300

Arg Leu Val Arg Ala Leu Arg Gly Pro Pro Thr Pro Ala Ser Pro Pro
305                 310                 315                 320

Arg Leu Ala Leu Asp Pro Gly Ala Leu Ala Gly Phe Pro Gln Gly Leu
                325                 330                 335

Leu Asn Leu Ser Asp Pro Ala Thr Gln Glu Arg Leu Leu Gly Gly Glu
            340                 345                 350

Glu Pro Leu Leu Leu Leu Leu Pro Pro Pro Thr Ala Ala Ala Gly Pro
        355                 360                 365

Pro Ala Pro Pro Pro Arg Pro Ala Ser Ala Pro Trp Ala Ala Gly Leu
    370                 375                 380

Ala Leu Arg Val Ala Ala Glu Leu Arg Ala Ala Ala Ala Glu Leu Arg
385                 390                 395                 400

Gly Leu Pro Gly Leu Pro Pro Ala Ala Ala Pro Leu Leu Glu Arg Leu
                405                 410                 415

Leu Ala Leu Cys Pro Gly Gly Ser Gly Gly Ser Gly Gly Ser Gly Asp
            420                 425                 430

Pro Leu Arg Ala Leu Leu Leu Leu Lys Ala Leu Gln Gly Leu Arg Ala
        435                 440                 445

Glu Trp Arg Gly Arg Glu Arg Gly Gly Pro Pro Arg Ala Gln Arg
    450                 455                 460

<210>  27
<211>  442
<212>  PRT
<213>  artificial sequence

<220>
<223>  polypeptide

<400>  27

Ala Glu Ala Pro Gly Gly Glu Val Ser Gly Thr Pro Ala Ser Pro Gly
1               5                   10                  15

Glu Pro Ala Thr Gly Thr Gly Gly Leu Leu Phe Gln Pro Asp Trp Asp
            20                  25                  30

Trp Pro Pro Ser Ala Pro Gln Asp Pro Leu Cys Leu Val Thr Leu Asp
        35                  40                  45

Lys Gly Gly Asn Gly Ser Ser Pro Pro Leu Arg Val Ala Gly Ala Leu
    50                  55                  60

Arg Gly Tyr Glu His Thr Phe Leu Glu Ala Val Arg Arg Ala Arg Trp
65                  70                  75                  80

Gly Pro His Asp Leu Ala Thr Phe Gly Ala Cys Ala Ala Ser Asp Gly
                85                  90                  95

Arg Thr Thr Gln Leu Ser Leu Arg Gln Leu Gln Ala Trp Leu Gly Ala
            100                 105                 110

Pro Gly Gly Arg Arg Leu Val Val Leu His Leu Glu Glu Val Thr Trp
        115                 120                 125

Glu Pro Ala Leu Ser Leu Lys Phe Gln Glu Pro Pro Pro Gly Gly Ala
    130                 135                 140

Ser Pro Leu Glu Leu Ala Leu Leu Val Leu Tyr Pro Gly Pro Gly Pro
145                 150                 155                 160

Glu Val Ala Val Thr Gly Ala Gly Leu Pro Gly Thr Gln Asn Leu Cys
                165                 170                 175

Arg Ser Arg Asn Thr Arg Tyr Leu Val Leu Ala Leu Asp His Pro Val
            180                 185                 190

Gly Ala Trp His Ser Pro Arg Val Thr Leu Thr Val His Ala Arg Gly
        195                 200                 205

Asp Gly Ala Pro Leu Ser Thr Pro Gln Leu Gln Glu Leu Leu Phe Gly
    210                 215                 220

Pro Asp Ala Arg Cys Phe Thr Arg Met Thr Pro Ala Leu Leu Val Leu
225                 230                 235                 240

Arg Leu Pro Gly Pro Thr Ala Val Pro Ala Arg Gly Leu Leu Asp Leu
                245                 250                 255

Val Pro Phe Pro Pro Pro Arg Pro Ser Arg Glu Pro Ala Glu Pro Pro
            260                 265                 270

Pro Ser Ala Asp Pro Phe Leu Glu Thr Leu Thr Arg Leu Val Arg Ala
        275                 280                 285

Leu Arg Gly Pro Pro Thr Pro Ala Ser Pro Pro Arg Leu Ala Leu Asp
    290                 295                 300

Pro Gly Ala Leu Ala Gly Phe Pro Gln Gly Leu Leu Asn Leu Ser Asp
305                 310                 315                 320

Pro Ala Thr Gln Glu Arg Leu Leu Gly Gly Glu Glu Pro Leu Leu Leu
                325                 330                 335

Leu Leu Pro Pro Pro Thr Ala Ala Ala Gly Pro Pro Ala Pro Pro Pro
            340                 345                 350

Arg Pro Ala Ser Ala Pro Trp Ala Ala Gly Leu Ala Leu Arg Val Ala
        355                 360                 365

Ala Glu Leu Arg Ala Ala Ala Ala Glu Leu Arg Gly Leu Pro Gly Leu
    370                 375                 380

Pro Pro Ala Ala Ala Pro Leu Leu Glu Arg Leu Leu Ala Leu Cys Pro
385                 390                 395                 400

Gly Gly Ser Gly Gly Ser Gly Gly Ser Gly Asp Pro Leu Arg Ala Leu
                405                 410                 415

Leu Leu Leu Lys Ala Leu Gln Gly Leu Arg Ala Glu Trp Arg Gly Arg
            420                 425                 430

Glu Arg Gly Gly Pro Pro Arg Ala Gln Arg
        435                 440

<210>  28
<211>  264
<212>  PRT
<213>  artificial sequence

<220>
<223>  polypeptide

<400>  28

Met Gly Ala Leu Ala Leu Trp Pro Leu Ala Leu Ala Leu Ser Gly Met
1               5                   10                  15

Gly Pro Leu Leu Gly Ala Glu Ala Pro Gly Gly Glu Val Ser Gly Thr
            20                  25                  30

Pro Ala Ser Pro Gly Glu Pro Ala Thr Gly Thr Gly Gly Leu Leu Phe
        35                  40                  45

Gln Pro Asp Trp Asp Trp Pro Pro Ser Ala Pro Gln Asp Pro Leu Cys
    50                  55                  60

Leu Val Thr Leu Asp Lys Gly Gly Asn Gly Ser Ser Pro Pro Leu Arg
65                  70                  75                  80

Val Ala Gly Ala Leu Arg Gly Tyr Glu His Thr Phe Leu Glu Ala Val
                85                  90                  95

Arg Arg Ala Arg Trp Gly Pro His Asp Leu Ala Thr Phe Gly Ala Cys
            100                 105                 110

Ala Ala Ser Asp Gly Arg Thr Thr Gln Leu Ser Leu Arg Gln Leu Gln
        115                 120                 125

Ala Trp Leu Gly Ala Pro Gly Gly Arg Arg Leu Val Val Leu His Leu
    130                 135                 140

Glu Glu Val Thr Trp Glu Pro Ala Leu Ser Leu Lys Phe Gln Glu Pro
145                 150                 155                 160

Pro Pro Gly Gly Ala Ser Pro Leu Glu Leu Ala Leu Leu Val Leu Tyr
                165                 170                 175

Pro Gly Pro Gly Pro Glu Val Ala Val Thr Gly Ala Gly Leu Pro Gly
            180                 185                 190

Thr Gln Asn Leu Cys Arg Ser Arg Asn Thr Arg Tyr Leu Val Leu Ala
        195                 200                 205

Leu Asp His Pro Val Gly Ala Trp His Ser Pro Arg Val Thr Leu Thr
    210                 215                 220

Val His Ala Arg Gly Asp Gly Ala Pro Leu Ser Thr Pro Gln Leu Gln
225                 230                 235                 240

Glu Leu Leu Phe Gly Pro Asp Ala Arg Cys Phe Thr Arg Met Thr Pro
                245                 250                 255

Ala Leu Leu Val Leu Arg Leu Pro
                260

<210>  29
<211>  243
<212>  PRT
<213>  artificial sequence

<220>
<223>  polypeptide

<400>  29

Ala Glu Ala Pro Gly Gly Glu Val Ser Gly Thr Pro Ala Ser Pro Gly
1               5                   10                  15

Glu Pro Ala Thr Gly Thr Gly Gly Leu Leu Phe Gln Pro Asp Trp Asp
            20                  25                  30

Trp Pro Pro Ser Ala Pro Gln Asp Pro Leu Cys Leu Val Thr Leu Asp
        35                  40                  45

Lys Gly Gly Asn Gly Ser Ser Pro Pro Leu Arg Val Ala Gly Ala Leu
    50                  55                  60

Arg Gly Tyr Glu His Thr Phe Leu Glu Ala Val Arg Arg Ala Arg Trp
65                  70                  75                  80

Gly Pro His Asp Leu Ala Thr Phe Gly Ala Cys Ala Ala Ser Asp Gly
                85                  90                  95

Arg Thr Thr Gln Leu Ser Leu Arg Gln Leu Gln Ala Trp Leu Gly Ala
            100                 105                 110

Pro Gly Gly Arg Arg Leu Val Val Leu His Leu Glu Glu Val Thr Trp
        115                 120                 125

Glu Pro Ala Leu Ser Leu Lys Phe Gln Glu Pro Pro Pro Gly Gly Ala
    130                 135                 140

Ser Pro Leu Glu Leu Ala Leu Leu Val Leu Tyr Pro Gly Pro Gly Pro
145                 150                 155                 160

Glu Val Ala Val Thr Gly Ala Gly Leu Pro Gly Thr Gln Asn Leu Cys
                165                 170                 175

Arg Ser Arg Asn Thr Arg Tyr Leu Val Leu Ala Leu Asp His Pro Val
            180                 185                 190

Gly Ala Trp His Ser Pro Arg Val Thr Leu Thr Val His Ala Arg Gly
        195                 200                 205

Asp Gly Ala Pro Leu Ser Thr Pro Gln Leu Gln Glu Leu Leu Phe Gly
    210                 215                 220

Pro Asp Ala Arg Cys Phe Thr Arg Met Thr Pro Ala Leu Leu Val Leu
225                 230                 235                 240

Arg Leu Pro

<210>  30
<211>  165
<212>  PRT
<213>  artificial sequence

<220>
<223>  polypeptide

<400>  30

Met Gly Ala Leu Ala Leu Trp Pro Leu Ala Leu Ala Leu Ser Gly Met
1               5                   10                  15

Gly Pro Leu Leu Gly Ala Glu Ala Pro Gly Gly Glu Val Ser Gly Thr
            20                  25                  30

Pro Ala Ser Pro Gly Glu Pro Ala Thr Gly Thr Gly Gly Leu Leu Phe
        35                  40                  45

Gln Pro Asp Trp Asp Trp Pro Pro Ser Ala Pro Gln Asp Pro Leu Cys
    50                  55                  60

Leu Val Thr Leu Asp Lys Gly Gly Asn Gly Ser Ser Pro Pro Leu Arg
65                  70                  75                  80

Val Ala Gly Ala Leu Arg Gly Tyr Glu His Thr Phe Leu Glu Ala Val
                85                  90                  95

Arg Arg Ala Arg Trp Gly Pro His Asp Leu Ala Thr Phe Gly Ala Cys
            100                 105                 110

Ala Ala Ser Asp Gly Arg Thr Thr Gln Leu Ser Leu Arg Gln Leu Gln
        115                 120                 125

Ala Trp Leu Gly Ala Pro Gly Gly Arg Arg Leu Val Val Leu His Leu
    130                 135                 140

Glu Glu Val Thr Trp Glu Pro Ala Leu Ser Leu Lys Phe Gln Glu Pro
145                 150                 155                 160

Pro Pro Gly Gly Ala
                165

<210>  31
<211>  144
<212>  PRT
<213>  artificial sequence

<220>
<223>  polypeptide

<400>  31

Ala Glu Ala Pro Gly Gly Glu Val Ser Gly Thr Pro Ala Ser Pro Gly
1               5                   10                  15

Glu Pro Ala Thr Gly Thr Gly Gly Leu Leu Phe Gln Pro Asp Trp Asp
            20                  25                  30

Trp Pro Pro Ser Ala Pro Gln Asp Pro Leu Cys Leu Val Thr Leu Asp
        35                  40                  45

Lys Gly Gly Asn Gly Ser Ser Pro Pro Leu Arg Val Ala Gly Ala Leu
    50                  55                  60

Arg Gly Tyr Glu His Thr Phe Leu Glu Ala Val Arg Arg Ala Arg Trp
65                  70                  75                  80

Gly Pro His Asp Leu Ala Thr Phe Gly Ala Cys Ala Ala Ser Asp Gly
                85                  90                  95

Arg Thr Thr Gln Leu Ser Leu Arg Gln Leu Gln Ala Trp Leu Gly Ala
            100                 105                 110

Pro Gly Gly Arg Arg Leu Val Val Leu His Leu Glu Glu Val Thr Trp
        115                 120                 125

Glu Pro Ala Leu Ser Leu Lys Phe Gln Glu Pro Pro Pro Gly Gly Ala
    130                 135                 140

<210>  32
<211>  100
<212>  PRT
<213>  artificial sequence

<220>
<223>  polypeptide

<400>  32

Ser Pro Pro Glu Leu Ala Leu Leu Val Val Tyr Pro Gly Pro Gly Leu
1               5                   10                  15

Glu Val Thr Val Thr Gly Ala Gly Leu Pro Gly Thr Gln Ser Leu Cys
            20                  25                  30

Leu Thr Ala Asp Ser Asp Phe Leu Ala Leu Val Val Asp His Pro Glu
        35                  40                  45

Gly Ala Trp Arg Arg Pro Gly Leu Ala Leu Thr Leu Arg Arg Arg Gly
    50                  55                  60

Asn Gly Ala Leu Leu Ser Thr Ala Gln Leu Gln Ala Leu Leu Phe Gly
65                  70                  75                  80

Ala Asp Ser Arg Cys Phe Thr Arg Lys Thr Pro Ala Leu Leu Leu Leu
                85                  90                  95

Leu Pro Ala Arg
            100

<210>  33
<211>  575
<212>  PRT
<213>  bovin

<400>  33

Met Pro Gly Pro Ser Leu Ser Leu Ala Leu Val Leu Ser Ala Met Gly
1               5                   10                  15

Ala Leu Leu Arg Pro Gly Thr Pro Arg Glu Glu Val Phe Ser Thr Ser
            20                  25                  30

Ala Leu Pro Arg Glu Gln Ala Thr Gly Ser Gly Ala Leu Ile Phe Gln
        35                  40                  45

Gln Ala Trp Asp Trp Pro Leu Ser Ser Leu Trp Leu Pro Gly Ser Pro
    50                  55                  60

Leu Asp Pro Leu Cys Leu Val Thr Leu His Gly Ser Gly Asn Gly Ser
65                  70                  75                  80

Arg Ala Pro Leu Arg Val Val Gly Val Leu Ser Ser Tyr Glu Gln Ala
                85                  90                  95

Phe Leu Glu Ala Val Arg Arg Thr His Trp Gly Leu Ser Asp Leu Thr
            100                 105                 110

Thr Phe Ala Val Cys Pro Ala Gly Asn Gly Gln Pro Val Leu Pro His
        115                 120                 125

Leu Gln Arg Leu Gln Ala Trp Leu Gly Glu Pro Gly Gly Arg Trp Leu
    130                 135                 140

Val Val Leu His Leu Glu Glu Val Thr Trp Glu Pro Thr Pro Leu Leu
145                 150                 155                 160

Arg Phe Gln Glu Pro Pro Pro Gly Gly Ala Ser Pro Pro Glu Leu Ala
                165                 170                 175

Leu Leu Val Val Tyr Pro Gly Pro Gly Leu Glu Val Thr Val Thr Gly
            180                 185                 190

Ala Gly Leu Pro Gly Thr Gln Ser Leu Cys Leu Thr Ala Asp Ser Asp
        195                 200                 205

Phe Leu Ala Leu Val Val Asp His Pro Glu Gly Ala Trp Arg Arg Pro
    210                 215                 220

Gly Leu Ala Leu Thr Leu Arg Arg Arg Gly Asn Gly Ala Leu Leu Ser
225                 230                 235                 240

Thr Ala Gln Leu Gln Ala Leu Leu Phe Gly Ala Asp Ser Arg Cys Phe
                245                 250                 255

Thr Arg Lys Thr Pro Ala Leu Leu Leu Leu Leu Pro Ala Arg Ser Ser
            260                 265                 270

Ala Pro Met Pro Ala His Gly Arg Leu Asp Leu Val Pro Phe Pro Gln
        275                 280                 285

Pro Arg Ala Ser Pro Glu Pro Glu Glu Ala Pro Pro Ser Ala Asp Pro
    290                 295                 300

Phe Leu Glu Thr Leu Thr Arg Leu Val Arg Ala Leu Ala Gly Pro Pro
305                 310                 315                 320

Ala Arg Ala Ser Pro Pro Arg Leu Ala Leu Asp Pro Gly Ala Leu Ala
                325                 330                 335

Gly Phe Pro Gln Gly Gln Val Asn Leu Ser Asp Pro Ala Ala Leu Glu
            340                 345                 350

Arg Leu Leu Asp Gly Glu Glu Pro Leu Leu Leu Leu Leu Pro Pro Thr
        355                 360                 365

Ala Ala Thr Thr Gly Val Pro Ala Thr Pro Gln Gly Pro Lys Ser Pro
    370                 375                 380

Leu Trp Ala Ala Gly Leu Ala Arg Arg Val Ala Ala Glu Leu Gln Ala
385                 390                 395                 400

Val Ala Ala Glu Leu Arg Ala Leu Pro Gly Leu Pro Pro Ala Ala Pro
                405                 410                 415

Pro Leu Leu Ala Arg Leu Leu Ala Leu Cys Pro Gly Asn Pro Asp Ser
            420                 425                 430

Pro Gly Gly Pro Leu Arg Ala Leu Leu Leu Leu Lys Ala Leu Gln Gly
        435                 440                 445

Leu Arg Ala Glu Trp Arg Gly Arg Glu Arg Ser Gly Ser Ala Arg Ala
    450                 455                 460

Gln Arg Ser Ala Gly Ala Ala Ala Ala Asp Gly Pro Cys Ala Leu Arg
465                 470                 475                 480

Glu Leu Ser Val Asp Leu Arg Ala Glu Arg Ser Val Leu Ile Pro Glu
                485                 490                 495

Thr Tyr Gln Ala Asn Asn Cys Gln Gly Ala Cys Gly Trp Pro Gln Ser
            500                 505                 510

Asp Arg Asn Pro Arg Tyr Gly Asn His Val Val Leu Leu Leu Lys Met
        515                 520                 525

Gln Ala Arg Gly Ala Thr Leu Ala Arg Pro Pro Cys Cys Val Pro Thr
    530                 535                 540

Ala Tyr Thr Gly Lys Leu Leu Ile Ser Leu Ser Glu Glu Arg Ile Ser
545                 550                 555                 560

Ala His His Val Pro Asn Met Val Ala Thr Glu Cys Gly Cys Arg
                565                 570                 575

<210>  34
<211>  558
<212>  PRT
<213>  artificial sequence

<220>
<223>  polypeptide

<400>  34

Leu Leu Arg Pro Gly Thr Pro Arg Glu Glu Val Phe Ser Thr Ser Ala
1               5                   10                  15

Leu Pro Arg Glu Gln Ala Thr Gly Ser Gly Ala Leu Ile Phe Gln Gln
            20                  25                  30

Ala Trp Asp Trp Pro Leu Ser Ser Leu Trp Leu Pro Gly Ser Pro Leu
        35                  40                  45

Asp Pro Leu Cys Leu Val Thr Leu His Gly Ser Gly Asn Gly Ser Arg
    50                  55                  60

Ala Pro Leu Arg Val Val Gly Val Leu Ser Ser Tyr Glu Gln Ala Phe
65                  70                  75                  80

Leu Glu Ala Val Arg Arg Thr His Trp Gly Leu Ser Asp Leu Thr Thr
                85                  90                  95

Phe Ala Val Cys Pro Ala Gly Asn Gly Gln Pro Val Leu Pro His Leu
            100                 105                 110

Gln Arg Leu Gln Ala Trp Leu Gly Glu Pro Gly Gly Arg Trp Leu Val
        115                 120                 125

Val Leu His Leu Glu Glu Val Thr Trp Glu Pro Thr Pro Leu Leu Arg
    130                 135                 140

Phe Gln Glu Pro Pro Pro Gly Gly Ala Ser Pro Pro Glu Leu Ala Leu
145                 150                 155                 160

Leu Val Val Tyr Pro Gly Pro Gly Leu Glu Val Thr Val Thr Gly Ala
                165                 170                 175

Gly Leu Pro Gly Thr Gln Ser Leu Cys Leu Thr Ala Asp Ser Asp Phe
            180                 185                 190

Leu Ala Leu Val Val Asp His Pro Glu Gly Ala Trp Arg Arg Pro Gly
        195                 200                 205

Leu Ala Leu Thr Leu Arg Arg Arg Gly Asn Gly Ala Leu Leu Ser Thr
    210                 215                 220

Ala Gln Leu Gln Ala Leu Leu Phe Gly Ala Asp Ser Arg Cys Phe Thr
225                 230                 235                 240

Arg Lys Thr Pro Ala Leu Leu Leu Leu Leu Pro Ala Arg Ser Ser Ala
                245                 250                 255

Pro Met Pro Ala His Gly Arg Leu Asp Leu Val Pro Phe Pro Gln Pro
        260                 265                 270

Arg Ala Ser Pro Glu Pro Glu Glu Ala Pro Pro Ser Ala Asp Pro Phe
    275                 280                 285

Leu Glu Thr Leu Thr Arg Leu Val Arg Ala Leu Ala Gly Pro Pro Ala
    290                 295                 300

Arg Ala Ser Pro Pro Arg Leu Ala Leu Asp Pro Gly Ala Leu Ala Gly
305                 310                 315                 320

Phe Pro Gln Gly Gln Val Asn Leu Ser Asp Pro Ala Ala Leu Glu Arg
                325                 330                 335

Leu Leu Asp Gly Glu Glu Pro Leu Leu Leu Leu Leu Pro Pro Thr Ala
            340                 345                 350

Ala Thr Thr Gly Val Pro Ala Thr Pro Gln Gly Pro Lys Ser Pro Leu
        355                 360                 365

Trp Ala Ala Gly Leu Ala Arg Arg Val Ala Ala Glu Leu Gln Ala Val
    370                 375                 380

Ala Ala Glu Leu Arg Ala Leu Pro Gly Leu Pro Pro Ala Ala Pro Pro
385                 390                 395                 400

Leu Leu Ala Arg Leu Leu Ala Leu Cys Pro Gly Asn Pro Asp Ser Pro
                405                 410                 415

Gly Gly Pro Leu Arg Ala Leu Leu Leu Leu Lys Ala Leu Gln Gly Leu
            420                 425                 430

Arg Ala Glu Trp Arg Gly Arg Glu Arg Ser Gly Ser Ala Arg Ala Gln
        435                 440                 445

Arg Ser Ala Gly Ala Ala Ala Ala Asp Gly Pro Cys Ala Leu Arg Glu
    450                 455                 460

Leu Ser Val Asp Leu Arg Ala Glu Arg Ser Val Leu Ile Pro Glu Thr
465                 470                 475                 480

Tyr Gln Ala Asn Asn Cys Gln Gly Ala Cys Gly Trp Pro Gln Ser Asp
                485                 490                 495

Arg Asn Pro Arg Tyr Gly Asn His Val Val Leu Leu Leu Lys Met Gln

500                 505                 510

Ala Arg Gly Ala Thr Leu Ala Arg Pro Pro Cys Cys Val Pro Thr Ala
        515                 520                 525

Tyr Thr Gly Lys Leu Leu Ile Ser Leu Ser Glu Glu Arg Ile Ser Ala
    530                 535                 540

His His Val Pro Asn Met Val Ala Thr Glu Cys Gly Cys Arg
545                 550                 555

<210>  35
<211>  551
<212>  PRT
<213>  artificial sequence

<220>
<223>  polypeptide

<400>  35

Arg Glu Glu Val Phe Ser Thr Ser Ala Leu Pro Arg Glu Gln Ala Thr
1               5                   10                  15

Gly Ser Gly Ala Leu Ile Phe Gln Gln Ala Trp Asp Trp Pro Leu Ser
            20                  25                  30

Ser Leu Trp Leu Pro Gly Ser Pro Leu Asp Pro Leu Cys Leu Val Thr
        35                  40                  45

Leu His Gly Ser Gly Asn Gly Ser Arg Ala Pro Leu Arg Val Val Gly
    50                  55                  60

Val Leu Ser Ser Tyr Glu Gln Ala Phe Leu Glu Ala Val Arg Arg Thr
65                  70                  75                  80

His Trp Gly Leu Ser Asp Leu Thr Thr Phe Ala Val Cys Pro Ala Gly
                85                  90                  95

Asn Gly Gln Pro Val Leu Pro His Leu Gln Arg Leu Gln Ala Trp Leu
            100                 105                 110

Gly Glu Pro Gly Gly Arg Trp Leu Val Val Leu His Leu Glu Glu Val
        115                 120                 125

Thr Trp Glu Pro Thr Pro Leu Leu Arg Phe Gln Glu Pro Pro Pro Gly
    130                 135                 140

Gly Ala Ser Pro Pro Glu Leu Ala Leu Leu Val Val Tyr Pro Gly Pro
145                 150                 155                 160

Gly Leu Glu Val Thr Val Thr Gly Ala Gly Leu Pro Gly Thr Gln Ser
                165                 170                 175

Leu Cys Leu Thr Ala Asp Ser Asp Phe Leu Ala Leu Val Val Asp His
            180                 185                 190

Pro Glu Gly Ala Trp Arg Arg Pro Gly Leu Ala Leu Thr Leu Arg Arg
        195                 200                 205

Arg Gly Asn Gly Ala Leu Leu Ser Thr Ala Gln Leu Gln Ala Leu Leu
    210                 215                 220

Phe Gly Ala Asp Ser Arg Cys Phe Thr Arg Lys Thr Pro Ala Leu Leu
225                 230                 235                 240

Leu Leu Leu Pro Ala Arg Ser Ser Ala Pro Met Pro Ala His Gly Arg
                245                 250                 255

Leu Asp Leu Val Pro Phe Pro Gln Pro Arg Ala Ser Pro Glu Pro Glu
            260                 265                 270

Glu Ala Pro Pro Ser Ala Asp Pro Phe Leu Glu Thr Leu Thr Arg Leu
        275                 280                 285

Val Arg Ala Leu Ala Gly Pro Pro Ala Arg Ala Ser Pro Pro Arg Leu
    290                 295                 300

Ala Leu Asp Pro Gly Ala Leu Ala Gly Phe Pro Gln Gly Gln Val Asn
305                 310                 315                 320

Leu Ser Asp Pro Ala Ala Leu Glu Arg Leu Leu Asp Gly Glu Glu Pro
                325                 330                 335

Leu Leu Leu Leu Leu Pro Pro Thr Ala Ala Thr Thr Gly Val Pro Ala
            340                 345                 350

Thr Pro Gln Gly Pro Lys Ser Pro Leu Trp Ala Ala Gly Leu Ala Arg
        355                 360                 365

Arg Val Ala Ala Glu Leu Gln Ala Val Ala Ala Glu Leu Arg Ala Leu
    370                 375                 380

Pro Gly Leu Pro Pro Ala Ala Pro Pro Leu Leu Ala Arg Leu Leu Ala
385                 390                 395                 400

Leu Cys Pro Gly Asn Pro Asp Ser Pro Gly Gly Pro Leu Arg Ala Leu

405                 410                 415

Leu Leu Leu Lys Ala Leu Gln Gly Leu Arg Ala Glu Trp Arg Gly Arg
            420                 425                 430

Glu Arg Ser Gly Ser Ala Arg Ala Gln Arg Ser Ala Gly Ala Ala Ala
        435                 440                 445

Ala Asp Gly Pro Cys Ala Leu Arg Glu Leu Ser Val Asp Leu Arg Ala
    450                 455                 460

Glu Arg Ser Val Leu Ile Pro Glu Thr Tyr Gln Ala Asn Asn Cys Gln
465                 470                 475                 480

Gly Ala Cys Gly Trp Pro Gln Ser Asp Arg Asn Pro Arg Tyr Gly Asn
                485                 490                 495

His Val Val Leu Leu Leu Lys Met Gln Ala Arg Gly Ala Thr Leu Ala
            500                 505                 510

Arg Pro Pro Cys Cys Val Pro Thr Ala Tyr Thr Gly Lys Leu Leu Ile
        515                 520                 525

Ser Leu Ser Glu Glu Arg Ile Ser Ala His His Val Pro Asn Met Val
    530                 535                 540

Ala Thr Glu Cys Gly Cys Arg
545                 550

<210>  36
<211>  466
<212>  PRT
<213>  artificial sequence

<220>
<223>  polypeptide

<400>  36

Met Pro Gly Pro Ser Leu Ser Leu Ala Leu Val Leu Ser Ala Met Gly
1               5                   10                  15

Ala Leu Leu Arg Pro Gly Thr Pro Arg Glu Glu Val Phe Ser Thr Ser
            20                  25                  30

Ala Leu Pro Arg Glu Gln Ala Thr Gly Ser Gly Ala Leu Ile Phe Gln
        35                  40                  45

Gln Ala Trp Asp Trp Pro Leu Ser Ser Leu Trp Leu Pro Gly Ser Pro
    50                  55                  60

Leu Asp Pro Leu Cys Leu Val Thr Leu His Gly Ser Gly Asn Gly Ser
65                  70                  75                  80

Arg Ala Pro Leu Arg Val Val Gly Val Leu Ser Ser Tyr Glu Gln Ala
                85                  90                  95

Phe Leu Glu Ala Val Arg Arg Thr His Trp Gly Leu Ser Asp Leu Thr
            100                 105                 110

Thr Phe Ala Val Cys Pro Ala Gly Asn Gly Gln Pro Val Leu Pro His
        115                 120                 125

Leu Gln Arg Leu Gln Ala Trp Leu Gly Glu Pro Gly Gly Arg Trp Leu
    130                 135                 140

Val Val Leu His Leu Glu Glu Val Thr Trp Glu Pro Thr Pro Leu Leu
145                 150                 155                 160

Arg Phe Gln Glu Pro Pro Pro Gly Gly Ala Ser Pro Pro Glu Leu Ala
                165                 170                 175

Leu Leu Val Val Tyr Pro Gly Pro Gly Leu Glu Val Thr Val Thr Gly
            180                 185                 190

Ala Gly Leu Pro Gly Thr Gln Ser Leu Cys Leu Thr Ala Asp Ser Asp
        195                 200                 205

Phe Leu Ala Leu Val Val Asp His Pro Glu Gly Ala Trp Arg Arg Pro
    210                 215                 220

Gly Leu Ala Leu Thr Leu Arg Arg Arg Gly Asn Gly Ala Leu Leu Ser
225                 230                 235                 240

Thr Ala Gln Leu Gln Ala Leu Leu Phe Gly Ala Asp Ser Arg Cys Phe
                245                 250                 255

Thr Arg Lys Thr Pro Ala Leu Leu Leu Leu Leu Pro Ala Arg Ser Ser
            260                 265                 270

Ala Pro Met Pro Ala His Gly Arg Leu Asp Leu Val Pro Phe Pro Gln
        275                 280                 285

Pro Arg Ala Ser Pro Glu Pro Glu Glu Ala Pro Pro Ser Ala Asp Pro
    290                 295                 300

Phe Leu Glu Thr Leu Thr Arg Leu Val Arg Ala Leu Ala Gly Pro Pro

305                 310                 315                 320

Ala Arg Ala Ser Pro Pro Arg Leu Ala Leu Asp Pro Gly Ala Leu Ala
                325                 330                 335

Gly Phe Pro Gln Gly Gln Val Asn Leu Ser Asp Pro Ala Ala Leu Glu
            340                 345                 350

Arg Leu Leu Asp Gly Glu Glu Pro Leu Leu Leu Leu Leu Pro Pro Thr
        355                 360                 365

Ala Ala Thr Thr Gly Val Pro Ala Thr Pro Gln Gly Pro Lys Ser Pro
    370                 375                 380

Leu Trp Ala Ala Gly Leu Ala Arg Arg Val Ala Ala Glu Leu Gln Ala
385                 390                 395                 400

Val Ala Ala Glu Leu Arg Ala Leu Pro Gly Leu Pro Pro Ala Ala Pro
                405                 410                 415

Pro Leu Leu Ala Arg Leu Leu Ala Leu Cys Pro Gly Asn Pro Asp Ser
            420                 425                 430

Pro Gly Gly Pro Leu Arg Ala Leu Leu Leu Leu Lys Ala Leu Gln Gly
        435                 440                 445

Leu Arg Ala Glu Trp Arg Gly Arg Glu Arg Ser Gly Ser Ala Arg Ala
    450                 455                 460

Gln Arg
465

<210>  37
<211>  449
<212>  PRT
<213>  artificial sequence

<220>
<223>  polypeptide

<400>  37

Leu Leu Arg Pro Gly Thr Pro Arg Glu Glu Val Phe Ser Thr Ser Ala
1               5                   10                  15

Leu Pro Arg Glu Gln Ala Thr Gly Ser Gly Ala Leu Ile Phe Gln Gln
            20                  25                  30

Ala Trp Asp Trp Pro Leu Ser Ser Leu Trp Leu Pro Gly Ser Pro Leu
        35                  40                  45

Asp Pro Leu Cys Leu Val Thr Leu His Gly Ser Gly Asn Gly Ser Arg
    50                  55                  60

Ala Pro Leu Arg Val Val Gly Val Leu Ser Ser Tyr Glu Gln Ala Phe
65                  70                  75                  80

Leu Glu Ala Val Arg Arg Thr His Trp Gly Leu Ser Asp Leu Thr Thr
                85                  90                  95

Phe Ala Val Cys Pro Ala Gly Asn Gly Gln Pro Val Leu Pro His Leu
            100                 105                 110

Gln Arg Leu Gln Ala Trp Leu Gly Glu Pro Gly Gly Arg Trp Leu Val
        115                 120                 125

Val Leu His Leu Glu Glu Val Thr Trp Glu Pro Thr Pro Leu Leu Arg
    130                 135                 140

Phe Gln Glu Pro Pro Pro Gly Gly Ala Ser Pro Pro Glu Leu Ala Leu
145                 150                 155                 160

Leu Val Val Tyr Pro Gly Pro Gly Leu Glu Val Thr Val Thr Gly Ala
                165                 170                 175

Gly Leu Pro Gly Thr Gln Ser Leu Cys Leu Thr Ala Asp Ser Asp Phe
            180                 185                 190

Leu Ala Leu Val Val Asp His Pro Glu Gly Ala Trp Arg Arg Pro Gly
        195                 200                 205

Leu Ala Leu Thr Leu Arg Arg Arg Gly Asn Gly Ala Leu Leu Ser Thr
    210                 215                 220

Ala Gln Leu Gln Ala Leu Leu Phe Gly Ala Asp Ser Arg Cys Phe Thr
225                 230                 235                 240

Arg Lys Thr Pro Ala Leu Leu Leu Leu Leu Pro Ala Arg Ser Ser Ala
                245                 250                 255

Pro Met Pro Ala His Gly Arg Leu Asp Leu Val Pro Phe Pro Gln Pro
            260                 265                 270

Arg Ala Ser Pro Glu Pro Glu Glu Ala Pro Pro Ser Ala Asp Pro Phe
        275                 280                 285

Leu Glu Thr Leu Thr Arg Leu Val Arg Ala Leu Ala Gly Pro Pro Ala

290                 295                 300

Arg Ala Ser Pro Pro Arg Leu Ala Leu Asp Pro Gly Ala Leu Ala Gly
305                 310                 315                 320

Phe Pro Gln Gly Gln Val Asn Leu Ser Asp Pro Ala Ala Leu Glu Arg
                325                 330                 335

Leu Leu Asp Gly Glu Glu Pro Leu Leu Leu Leu Pro Pro Thr Ala
            340                 345                 350

Ala Thr Thr Gly Val Pro Ala Thr Pro Gln Gly Pro Lys Ser Pro Leu
        355                 360                 365

Trp Ala Ala Gly Leu Ala Arg Arg Val Ala Ala Glu Leu Gln Ala Val
    370                 375                 380

Ala Ala Glu Leu Arg Ala Leu Pro Gly Leu Pro Pro Ala Ala Pro Pro
385                 390                 395                 400

Leu Leu Ala Arg Leu Leu Ala Leu Cys Pro Gly Asn Pro Asp Ser Pro
                405                 410                 415

Gly Gly Pro Leu Arg Ala Leu Leu Leu Leu Lys Ala Leu Gln Gly Leu
            420                 425                 430

Arg Ala Glu Trp Arg Gly Arg Glu Arg Ser Gly Ser Ala Arg Ala Gln
        435                 440                 445

Arg

<210>  38
<211>  442
<212>  PRT
<213>  artificial sequence

<220>
<223>  polypeptide

<400>  38

Arg Glu Glu Val Phe Ser Thr Ser Ala Leu Pro Arg Glu Gln Ala Thr
1               5                   10                  15

Gly Ser Gly Ala Leu Ile Phe Gln Gln Ala Trp Asp Trp Pro Leu Ser
            20                  25                  30

Ser Leu Trp Leu Pro Gly Ser Pro Leu Asp Pro Leu Cys Leu Val Thr
        35                  40                  45

Leu His Gly Ser Gly Asn Gly Ser Arg Ala Pro Leu Arg Val Val Gly
    50                  55                  60

Val Leu Ser Ser Tyr Glu Gln Ala Phe Leu Glu Ala Val Arg Arg Thr
65                  70                  75                  80

His Trp Gly Leu Ser Asp Leu Thr Thr Phe Ala Val Cys Pro Ala Gly
                85                  90                  95

Asn Gly Gln Pro Val Leu Pro His Leu Gln Arg Leu Gln Ala Trp Leu
            100                 105                 110

Gly Glu Pro Gly Gly Arg Trp Leu Val Val Leu His Leu Glu Glu Val
        115                 120                 125

Thr Trp Glu Pro Thr Pro Leu Leu Arg Phe Gln Glu Pro Pro Pro Gly
    130                 135                 140

Gly Ala Ser Pro Pro Glu Leu Ala Leu Leu Val Val Tyr Pro Gly Pro
145                 150                 155                 160

Gly Leu Glu Val Thr Val Thr Gly Ala Gly Leu Pro Gly Thr Gln Ser
                165                 170                 175

Leu Cys Leu Thr Ala Asp Ser Asp Phe Leu Ala Leu Val Val Asp His
            180                 185                 190

Pro Glu Gly Ala Trp Arg Arg Pro Gly Leu Ala Leu Thr Leu Arg Arg
        195                 200                 205

Arg Gly Asn Gly Ala Leu Leu Ser Thr Ala Gln Leu Gln Ala Leu Leu
    210                 215                 220

Phe Gly Ala Asp Ser Arg Cys Phe Thr Arg Lys Thr Pro Ala Leu Leu
225                 230                 235                 240

Leu Leu Leu Pro Ala Arg Ser Ser Ala Pro Met Pro Ala His Gly Arg
                245                 250                 255

Leu Asp Leu Val Pro Phe Pro Gln Pro Arg Ala Ser Pro Glu Pro Glu
            260                 265                 270

Glu Ala Pro Pro Ser Ala Asp Pro Phe Leu Glu Thr Leu Thr Arg Leu
        275                 280                 285

Val Arg Ala Leu Ala Gly Pro Pro Ala Arg Ala Ser Pro Pro Arg Leu

290                 295                 300

Ala Leu Asp Pro Gly Ala Leu Ala Gly Phe Pro Gln Gly Gln Val Asn
305                 310                 315                 320

Leu Ser Asp Pro Ala Ala Leu Glu Arg Leu Leu Asp Gly Glu Glu Pro
                325                 330                 335

Leu Leu Leu Leu Leu Pro Pro Thr Ala Ala Thr Thr Gly Val Pro Ala
            340                 345                 350

Thr Pro Gln Gly Pro Lys Ser Pro Leu Trp Ala Ala Gly Leu Ala Arg
        355                 360                 365

Arg Val Ala Ala Glu Leu Gln Ala Val Ala Ala Glu Leu Arg Ala Leu
    370                 375                 380

Pro Gly Leu Pro Pro Ala Ala Pro Pro Leu Leu Ala Arg Leu Leu Ala
385                 390                 395                 400

Leu Cys Pro Gly Asn Pro Asp Ser Pro Gly Gly Pro Leu Arg Ala Leu
                405                 410                 415

Leu Leu Leu Lys Ala Leu Gln Gly Leu Arg Ala Glu Trp Arg Gly Arg
            420                 425                 430

Glu Arg Ser Gly Ser Ala Arg Ala Gln Arg
        435                 440

<210>  39
<211>  270
<212>  PRT
<213>  artificial sequence

<220>
<223>  polypeptide

<400>  39

Met Pro Gly Pro Ser Leu Ser Leu Ala Leu Val Leu Ser Ala Met Gly
1               5                   10                  15

Ala Leu Leu Arg Pro Gly Thr Pro Arg Glu Glu Val Phe Ser Thr Ser
            20                  25                  30

Ala Leu Pro Arg Glu Gln Ala Thr Gly Ser Gly Ala Leu Ile Phe Gln
        35                  40                  45

Gln Ala Trp Asp Trp Pro Leu Ser Ser Leu Trp Leu Pro Gly Ser Pro
    50                  55                  60

Leu Asp Pro Leu Cys Leu Val Thr Leu His Gly Ser Gly Asn Gly Ser
65                  70                  75                  80

Arg Ala Pro Leu Arg Val Val Gly Val Leu Ser Ser Tyr Glu Gln Ala
                85                  90                  95

Phe Leu Glu Ala Val Arg Arg Thr His Trp Gly Leu Ser Asp Leu Thr
            100                 105                 110

Thr Phe Ala Val Cys Pro Ala Gly Asn Gly Gln Pro Val Leu Pro His
        115                 120                 125

Leu Gln Arg Leu Gln Ala Trp Leu Gly Glu Pro Gly Gly Arg Trp Leu
    130                 135                 140

Val Val Leu His Leu Glu Glu Val Thr Trp Glu Pro Thr Pro Leu Leu
145                 150                 155                 160

Arg Phe Gln Glu Pro Pro Pro Gly Gly Ala Ser Pro Pro Glu Leu Ala
                165                 170                 175

Leu Leu Val Val Tyr Pro Gly Pro Gly Leu Glu Val Thr Val Thr Gly
            180                 185                 190

Ala Gly Leu Pro Gly Thr Gln Ser Leu Cys Leu Thr Ala Asp Ser Asp
        195                 200                 205

Phe Leu Ala Leu Val Val Asp His Pro Glu Gly Ala Trp Arg Arg Pro
    210                 215                 220

Gly Leu Ala Leu Thr Leu Arg Arg Arg Gly Asn Gly Ala Leu Leu Ser
225                 230                 235                 240

Thr Ala Gln Leu Gln Ala Leu Leu Phe Gly Ala Asp Ser Arg Cys Phe
                245                 250                 255

Thr Arg Lys Thr Pro Ala Leu Leu Leu Leu Leu Pro Ala Arg
            260                 265                 270

<210>  40
<211>  253
<212>  PRT
<213>  artificial sequence

<220>
<223>  polypeptide

<400>  40

Leu Leu Arg Pro Gly Thr Pro Arg Glu Glu Val Phe Ser Thr Ser Ala
1               5                   10                  15

Leu Pro Arg Glu Gln Ala Thr Gly Ser Gly Ala Leu Ile Phe Gln Gln
            20                  25                  30

Ala Trp Asp Trp Pro Leu Ser Ser Leu Trp Leu Pro Gly Ser Pro Leu
        35                  40                  45

Asp Pro Leu Cys Leu Val Thr Leu His Gly Ser Gly Asn Gly Ser Arg
    50                  55                  60

Ala Pro Leu Arg Val Val Gly Val Leu Ser Ser Tyr Glu Gln Ala Phe
65                  70                  75                  80

Leu Glu Ala Val Arg Arg Thr His Trp Gly Leu Ser Asp Leu Thr Thr
                85                  90                  95

Phe Ala Val Cys Pro Ala Gly Asn Gly Gln Pro Val Leu Pro His Leu
            100                 105                 110

Gln Arg Leu Gln Ala Trp Leu Gly Glu Pro Gly Gly Arg Trp Leu Val
        115                 120                 125

Val Leu His Leu Glu Glu Val Thr Trp Glu Pro Thr Pro Leu Leu Arg
    130                 135                 140

Phe Gln Glu Pro Pro Pro Gly Gly Ala Ser Pro Pro Glu Leu Ala Leu
145                 150                 155                 160

Leu Val Val Tyr Pro Gly Pro Gly Leu Glu Val Thr Val Thr Gly Ala
                165                 170                 175

Gly Leu Pro Gly Thr Gln Ser Leu Cys Leu Thr Ala Asp Ser Asp Phe
            180                 185                 190

Leu Ala Leu Val Val Asp His Pro Glu Gly Ala Trp Arg Arg Pro Gly
        195                 200                 205

Leu Ala Leu Thr Leu Arg Arg Arg Gly Asn Gly Ala Leu Leu Ser Thr
    210                 215                 220

Ala Gln Leu Gln Ala Leu Leu Phe Gly Ala Asp Ser Arg Cys Phe Thr
225                 230                 235                 240

Arg Lys Thr Pro Ala Leu Leu Leu Leu Leu Pro Ala Arg
                245                 250

<210>  41
<211>  246
<212>  PRT
<213>  artificial sequence

<220>
<223>  polypeptide

<400>  41

Arg Glu Glu Val Phe Ser Thr Ser Ala Leu Pro Arg Glu Gln Ala Thr
1               5                   10                  15

Gly Ser Gly Ala Leu Ile Phe Gln Gln Ala Trp Asp Trp Pro Leu Ser
            20                  25                  30

Ser Leu Trp Leu Pro Gly Ser Pro Leu Asp Pro Leu Cys Leu Val Thr
        35                  40                  45

Leu His Gly Ser Gly Asn Gly Ser Arg Ala Pro Leu Arg Val Val Gly
    50                  55                  60

Val Leu Ser Ser Tyr Glu Gln Ala Phe Leu Glu Ala Val Arg Arg Thr
65                  70                  75                  80

His Trp Gly Leu Ser Asp Leu Thr Thr Phe Ala Val Cys Pro Ala Gly
                85                  90                  95

Asn Gly Gln Pro Val Leu Pro His Leu Gln Arg Leu Gln Ala Trp Leu
            100                 105                 110

Gly Glu Pro Gly Gly Arg Trp Leu Val Val Leu His Leu Glu Glu Val
        115                 120                 125

Thr Trp Glu Pro Thr Pro Leu Leu Arg Phe Gln Glu Pro Pro Pro Gly
    130                 135                 140

Gly Ala Ser Pro Pro Glu Leu Ala Leu Leu Val Val Tyr Pro Gly Pro
145                 150                 155                 160

Gly Leu Glu Val Thr Val Thr Gly Ala Gly Leu Pro Gly Thr Gln Ser
                165                 170                 175

Leu Cys Leu Thr Ala Asp Ser Asp Phe Leu Ala Leu Val Val Asp His
            180                 185                 190

Pro Glu Gly Ala Trp Arg Arg Pro Gly Leu Ala Leu Thr Leu Arg Arg
        195                 200                 205

Arg Gly Asn Gly Ala Leu Leu Ser Thr Ala Gln Leu Gln Ala Leu Leu
    210                 215                 220

Phe Gly Ala Asp Ser Arg Cys Phe Thr Arg Lys Thr Pro Ala Leu Leu
225                 230                 235                 240

Leu Leu Leu Pro Ala Arg
                245

<210>  42
<211>  170
<212>  PRT
<213>  artificial sequence

<220>
<223>  polypeptide

<400>  42

Met Pro Gly Pro Ser Leu Ser Leu Ala Leu Val Leu Ser Ala Met Gly
1               5                   10                  15

Ala Leu Leu Arg Pro Gly Thr Pro Arg Glu Glu Val Phe Ser Thr Ser
            20                  25                  30

Ala Leu Pro Arg Glu Gln Ala Thr Gly Ser Gly Ala Leu Ile Phe Gln
        35                  40                  45

Gln Ala Trp Asp Trp Pro Leu Ser Ser Leu Trp Leu Pro Gly Ser Pro
    50                  55                  60

Leu Asp Pro Leu Cys Leu Val Thr Leu His Gly Ser Gly Asn Gly Ser
65                  70                  75                  80

Arg Ala Pro Leu Arg Val Val Gly Val Leu Ser Ser Tyr Glu Gln Ala
                85                  90                  95

Phe Leu Glu Ala Val Arg Arg Thr His Trp Gly Leu Ser Asp Leu Thr
            100                 105                 110

Thr Phe Ala Val Cys Pro Ala Gly Asn Gly Gln Pro Val Leu Pro His
        115                 120                 125

Leu Gln Arg Leu Gln Ala Trp Leu Gly Glu Pro Gly Gly Arg Trp Leu
    130                 135                 140

Val Val Leu His Leu Glu Glu Val Thr Trp Glu Pro Thr Pro Leu Leu
145                 150                 155                 160

Arg Phe Gln Glu Pro Pro Pro Gly Gly Ala
                165                 170

<210>  43
<211>  153
<212>  PRT
<213>  artificial sequence

<220>
<223>  polypeptide

<400>  43

Leu Leu Arg Pro Gly Thr Pro Arg Glu Glu Val Phe Ser Thr Ser Ala
1               5                   10                  15

Leu Pro Arg Glu Gln Ala Thr Gly Ser Gly Ala Leu Ile Phe Gln Gln
            20                  25                  30

Ala Trp Asp Trp Pro Leu Ser Ser Leu Trp Leu Pro Gly Ser Pro Leu
        35                  40                  45

Asp Pro Leu Cys Leu Val Thr Leu His Gly Ser Gly Asn Gly Ser Arg
    50                  55                  60

Ala Pro Leu Arg Val Val Gly Val Leu Ser Ser Tyr Glu Gln Ala Phe
65                  70                  75                  80

Leu Glu Ala Val Arg Arg Thr His Trp Gly Leu Ser Asp Leu Thr Thr
                85                  90                  95

Phe Ala Val Cys Pro Ala Gly Asn Gly Gln Pro Val Leu Pro His Leu
            100                 105                 110

Gln Arg Leu Gln Ala Trp Leu Gly Glu Pro Gly Gly Arg Trp Leu Val
        115                 120                 125

Val Leu His Leu Glu Glu Val Thr Trp Glu Pro Thr Pro Leu Leu Arg
    130                 135                 140

Phe Gln Glu Pro Pro Pro Gly Gly Ala
145                 150

<210>  44
<211>  146
<212>  PRT
<213>  artificial sequence

<220>
<223>  polypeptide

<400>  44

Arg Glu Glu Val Phe Ser Thr Ser Ala Leu Pro Arg Glu Gln Ala Thr
1               5                   10                  15

Gly Ser Gly Ala Leu Ile Phe Gln Gln Ala Trp Asp Trp Pro Leu Ser
            20                  25                  30

Ser Leu Trp Leu Pro Gly Ser Pro Leu Asp Pro Leu Cys Leu Val Thr
        35                  40                  45

Leu His Gly Ser Gly Asn Gly Ser Arg Ala Pro Leu Arg Val Val Gly
    50                  55                  60

Val Leu Ser Ser Tyr Glu Gln Ala Phe Leu Glu Ala Val Arg Arg Thr
65                  70                  75                  80

His Trp Gly Leu Ser Asp Leu Thr Thr Phe Ala Val Cys Pro Ala Gly
                85                  90                  95

Asn Gly Gln Pro Val Leu Pro His Leu Gln Arg Leu Gln Ala Trp Leu
            100                 105                 110

Gly Glu Pro Gly Gly Arg Trp Leu Val Val Leu His Leu Glu Glu Val
        115                 120                 125

Thr Trp Glu Pro Thr Pro Leu Leu Arg Phe Gln Glu Pro Pro Pro Gly
    130                 135                 140

Gly Ala
145

<210>  45
<211>  12
<212>  PRT
<213>  artificial sequence

<220>
<223>  epitope

<400>  45

Val Leu Ala Val Asp Arg Pro Ala Gly Ala Trp Arg
1               5                   10

<210>  46
<211>  12
<212>  PRT
<213>  artificial sequence

<220>
<223>  epitope

<400>  46

Pro Arg Gly Glu Asp Ser Arg Leu Ser Thr Ala Arg
1               5                   10


<210>  47
<211>  12
<212>  PRT
<213>  artificial sequence

<220>
<223>  epitope

<400>  47

His Arg Cys Phe Thr Arg Met Thr Pro Ala Leu Leu
1               5                   10


<210>  48
<211>  12
<212>  PRT
<213>  artificial sequence

<220>
<223>  epitope

<400>  48

Ala Leu Ala Val Asp His Pro Ala Arg Ala Trp Arg
1               5                   10


<210>  49
<211>  12
<212>  PRT
<213>  artificial sequence

<220>
<223>  epitope

<400>  49

Pro Arg Gly Asp Gly Ala Pro Leu Ser Thr Ala Gln
1               5                   10


<210>  50
<211>  12
<212>  PRT
<213>  artificial sequence

<220>
<223>  epitope

<400>  50

Pro Arg Cys Phe Thr Arg Met Thr Pro Ala Leu Phe
1               5                   10


<210>  51

<211>  12
<212>  PRT
<213>  artificial sequence

<220>
<223>  epitope

<400>  51

Val Leu Ala Leu Asp His Pro Val Gly Ala Trp His
1               5                   10


<210>  52
<211>  12
<212>  PRT
<213>  artificial sequence

<220>
<223>  epitope

<400>  52

Ala Arg Gly Asp Gly Ala Pro Leu Ser Thr Pro Gln
1               5                   10


<210>  53
<211>  12
<212>  PRT
<213>  artificial sequence

<220>
<223>  epitope

<400>  53

Ala Arg Cys Phe Thr Arg Met Thr Pro Ala Leu Leu
1               5                   10


<210>  54
<211>  12
<212>  PRT
<213>  artificial sequence

<220>
<223>  epitope

<400>  54

Ala Leu Val Val Asp His Pro Glu Gly Ala Trp Arg
1               5                   10


<210>  55
<211>  12
<212>  PRT
<213>  artificial sequence

<220>
<223>  epitope

<400>  55

Arg Arg Gly Asn Gly Ala Leu Leu Ser Thr Ala Gln
1               5                   10


<210>  56
<211>  12
<212>  PRT
<213>  artificial sequence

<220>
<223>  epitope

<400>  56

Ser Arg Cys Phe Thr Arg Lys Thr Pro Ala Leu Leu
1               5                   10


<210>  57
<211>  196
<212>  PRT
<213>  artificial sequence

<220>
<223>  polypeptide

<400>  57

Glu Pro Ala Pro Leu Pro Ala His Gly Gln Leu Asp Thr Val Pro Phe
1               5                   10                  15


Pro Pro Pro Arg Pro Ser Ala Glu Leu Glu Glu Ser Pro Pro Ser Ala
            20                  25                  30


Asp Pro Phe Leu Glu Thr Leu Thr Arg Leu Val Arg Ala Leu Arg Val
        35                  40                  45


Pro Pro Ala Arg Ala Ser Ala Pro Arg Leu Ala Leu Asp Pro Asp Ala
    50                  55                  60


Leu Ala Gly Phe Pro Gln Gly Leu Val Asn Leu Ser Asp Pro Ala Ala
65                  70                  75                  80


Leu Glu Arg Leu Leu Asp Gly Glu Glu Pro Leu Leu Leu Leu Leu Arg
                85                  90                  95


Pro Thr Ala Ala Thr Thr Gly Asp Pro Ala Pro Leu His Asp Pro Thr
            100                 105                 110


Ser Ala Pro Trp Ala Thr Ala Leu Ala Arg Arg Val Ala Ala Glu Leu
        115                 120                 125


Gln Ala Ala Ala Ala Glu Leu Arg Ser Leu Pro Gly Leu Pro Pro Ala

130                 135                 140

Thr Ala Pro Leu Leu Ala Arg Leu Leu Ala Leu Cys Pro Gly Gly Pro
145                 150                 155                 160

Gly Gly Leu Gly Asp Pro Leu Arg Ala Leu Leu Leu Leu Lys Ala Leu
                165                 170                 175

Gln Gly Leu Arg Val Glu Trp Arg Gly Arg Asp Pro Arg Gly Pro Gly
            180                 185                 190

Arg Ala Gln Arg
        195

<210>  58
<211>  199
<212>  PRT
<213>  artificial sequence

<220>
<223>  polypeptide

<400>  58

Gly Pro Ala Pro Met Pro Ala His Gly Arg Leu Asp Thr Val Pro Phe
1               5                   10                  15

Pro Pro Ala Arg Pro Ser Pro Glu Pro Glu Glu Pro Arg Pro Ser Ala
            20                  25                  30

Asp Pro Phe Leu Glu Thr Leu Thr Arg Leu Val Arg Ala Leu Arg Gly
        35                  40                  45

Pro Pro Thr Pro Ala Ser Pro Pro Arg Leu Ala Leu Asp Pro Gly Ala
    50                  55                  60

Leu Ala Ser Phe Pro Gln Gly Leu Val Asn Leu Ser Asp Pro Ala Ala
65                  70                  75                  80

Leu Glu Arg Leu Leu Asp Gly Glu Glu Pro Leu Leu Leu Leu Leu Pro
                85                  90                  95

Pro Thr Ala Ala Ala Ala Gly Asp Pro Ala Pro Leu Pro Asp Pro Ala
            100                 105                 110

Ser Ala Pro Trp Ala Ala Gly Leu Ala Arg Arg Val Ala Ala Glu Leu
        115                 120                 125

Gln Ala Ala Ala Ala Glu Leu Arg Ser Leu Pro Gly Leu Pro Pro Ala
    130                 135                 140

Ala Glu Pro Leu Leu Ala Arg Leu Leu Ala Leu Cys Pro Gly Asp Ala
145                 150                 155                 160

Glu Asp Gln Gly Gly Pro Gly Gly Pro Leu Arg Ala Leu Leu Leu Leu
                165                 170                 175

Lys Ala Leu Gln Gly Leu Arg Ala Glu Trp Arg Gly Arg Glu Arg Ser
            180                 185                 190

Gly Pro Gly Arg Ala Gln Arg
        195

<210>  59
<211>  199
<212>  PRT
<213>  artificial sequence

<220>
<223>  polypeptide

<400>  59

Gly Pro Thr Ala Val Pro Ala Arg Gly Leu Leu Asp Leu Val Pro Phe
1               5                   10                  15

Pro Pro Pro Arg Pro Ser Arg Glu Pro Ala Glu Pro Pro Pro Ser Ala
            20                  25                  30

Asp Pro Phe Leu Glu Thr Leu Thr Arg Leu Val Arg Ala Leu Arg Gly
        35                  40                  45

Pro Pro Thr Pro Ala Ser Pro Pro Arg Leu Ala Leu Asp Pro Gly Ala
    50                  55                  60

Leu Ala Gly Phe Pro Gln Gly Leu Leu Asn Leu Ser Asp Pro Ala Thr
65                  70                  75                  80

Gln Glu Arg Leu Leu Gly Gly Glu Glu Pro Leu Leu Leu Leu Leu Pro
                85                  90                  95

Pro Pro Thr Ala Ala Ala Gly Pro Pro Ala Pro Pro Pro Arg Pro Ala
            100                 105                 110

Ser Ala Pro Trp Ala Ala Gly Leu Ala Leu Arg Val Ala Ala Glu Leu
        115                 120                 125

Arg Ala Ala Ala Ala Glu Leu Arg Gly Leu Pro Gly Leu Pro Pro Ala
    130                 135                 140

Ala Ala Pro Leu Leu Glu Arg Leu Leu Ala Leu Cys Pro Gly Gly Ser
145                 150                 155                 160

Gly Gly Ser Gly Gly Ser Gly Asp Pro Leu Arg Ala Leu Leu Leu Leu
                165                 170                 175

Lys Ala Leu Gln Gly Leu Arg Ala Glu Trp Arg Gly Arg Glu Arg Gly
            180                 185                 190

Gly Pro Pro Arg Ala Gln Arg
        195

<210>  60
<211>  196
<212>  PRT
<213>  artificial sequence

<220>
<223>  polypeptide

<400>  60

Ser Ser Ala Pro Met Pro Ala His Gly Arg Leu Asp Leu Val Pro Phe
1               5                   10                  15

Pro Gln Pro Arg Ala Ser Pro Glu Pro Glu Glu Ala Pro Pro Ser Ala
            20                  25                  30

Asp Pro Phe Leu Glu Thr Leu Thr Arg Leu Val Arg Ala Leu Ala Gly
        35                  40                  45

Pro Pro Ala Arg Ala Ser Pro Pro Arg Leu Ala Leu Asp Pro Gly Ala
    50                  55                  60

Leu Ala Gly Phe Pro Gln Gly Gln Val Asn Leu Ser Asp Pro Ala Ala
65                  70                  75                  80

Leu Glu Arg Leu Leu Asp Gly Glu Glu Pro Leu Leu Leu Leu Leu Pro
                85                  90                  95

Pro Thr Ala Ala Thr Thr Gly Val Pro Ala Thr Pro Gln Gly Pro Lys
            100                 105                 110

Ser Pro Leu Trp Ala Ala Gly Leu Ala Arg Arg Val Ala Ala Glu Leu
        115                 120                 125

Gln Ala Val Ala Ala Glu Leu Arg Ala Leu Pro Gly Leu Pro Pro Ala
    130                 135                 140

Ala Pro Pro Leu Leu Ala Arg Leu Leu Ala Leu Cys Pro Gly Asn Pro
145                 150                 155                 160

Asp Ser Pro Gly Gly Pro Leu Arg Ala Leu Leu Leu Leu Lys Ala Leu
                165                 170                 175

Gln Gly Leu Arg Ala Glu Trp Arg Gly Arg Glu Arg Ser Gly Ser Ala
            180                 185                 190

Arg Ala Gln Arg
        195

<210>  61
<211>  12
<212>  PRT
<213>  artificial sequence

<220>
<223>  epitope

<400>  61

Leu Pro Ala His Gly Gln Leu Asp Thr Val Pro Phe
1               5                   10

<210>  62
<211>  12
<212>  PRT
<213>  artificial sequence

<220>
<223>  epitope

<400>  62

Leu Ser Asp Pro Ala Ala Leu Glu Arg Leu Leu Asp
1               5                   10

<210>  63
<211>  12
<212>  PRT
<213>  artificial sequence

<220>
<223>  epitope

<400>  63

Glu Arg Leu Leu Asp Gly Glu Glu Pro Leu Leu Leu
1               5                   10

<210>  64
<211>  12
<212>  PRT
<213>  artificial sequence

<220>
<223>  epitope

<400>  64

Glu Pro Leu Leu Leu Leu Leu Arg Pro Thr Ala Ala
1               5                   10


<210>  65
<211>  12
<212>  PRT
<213>  artificial sequence

<220>
<223>  epitope

<400>  65

Gly Asp Pro Ala Pro Leu His Asp Pro Thr Ser Ala
1               5                   10


<210>  66
<211>  12
<212>  PRT
<213>  artificial sequence

<220>
<223>  epitope

<400>  66

Asp Pro Thr Ser Ala Pro Trp Ala Thr Ala Leu Ala
1               5                   10


<210>  67
<211>  12
<212>  PRT
<213>  artificial sequence

<220>
<223>  epitope

<400>  67

Leu Lys Ala Leu Gln Gly Leu Arg Val Glu Trp Arg
1               5                   10


<210>  68
<211>  12
<212>  PRT
<213>  artificial sequence

<220>
<223>  epitope

<400>  68

Arg Val Glu Trp Arg Gly Arg Asp Pro Arg Gly Pro
1               5                   10

<210>  69
<211>  12
<212>  PRT
<213>  artificial sequence

<220>
<223>  epitope

<400>  69

Met Pro Ala His Gly Arg Leu Asp Thr Val Pro Phe
1               5                   10


<210>  70
<211>  12
<212>  PRT
<213>  artificial sequence

<220>
<223>  epitope

<400>  70

Glu Pro Leu Leu Leu Leu Leu Pro Pro Thr Ala Ala
1               5                   10


<210>  71
<211>  12
<212>  PRT
<213>  artificial sequence

<220>
<223>  epitope

<400>  71

Gly Asp Pro Ala Pro Leu Pro Asp Pro Ala Ser Ala
1               5                   10


<210>  72
<211>  12
<212>  PRT
<213>  artificial sequence

<220>
<223>  epitope

<400>  72

Asp Pro Ala Ser Ala Pro Trp Ala Ala Gly Leu Ala
1               5                   10


<210>  73
<211>  12
<212>  PRT
<213>  artificial sequence

<220>
<223>  epitope

<400>  73

Leu Lys Ala Leu Gln Gly Leu Arg Ala Glu Trp Arg
1               5                   10


<210>  74
<211>  12
<212>  PRT
<213>  artificial sequence

<220>
<223>  epitope

<400>  74

Arg Ala Glu Trp Arg Gly Arg Glu Arg Ser Gly Pro
1               5                   10


<210>  75
<211>  12
<212>  PRT
<213>  artificial sequence

<220>
<223>  epitope

<400>  75

Val Pro Ala Arg Gly Leu Leu Asp Leu Val Pro Phe
1               5                   10


<210>  76
<211>  12
<212>  PRT
<213>  artificial sequence

<220>
<223>  epitope

<400>  76

Leu Ser Asp Pro Ala Thr Gln Glu Arg Leu Leu Gly
1               5                   10


<210>  77
<211>  12
<212>  PRT
<213>  artificial sequence

<220>
<223>  epitope

<400>  77

Glu Arg Leu Leu Gly Gly Glu Glu Pro Leu Leu Leu
1               5                   10

<210>  78
<211>  13
<212>  PRT
<213>  artificial sequence

<220>
<223>  epitope

<400>  78

Glu Pro Leu Leu Leu Leu Leu Pro Pro Pro Thr Ala Ala
1               5                   10


<210>  79
<211>  12
<212>  PRT
<213>  artificial sequence

<220>
<223>  epitope

<400>  79

Gly Pro Pro Ala Pro Pro Pro Arg Pro Ala Ser Ala
1               5                   10


<210>  80
<211>  12
<212>  PRT
<213>  artificial sequence

<220>
<223>  epitope

<400>  80

Arg Pro Ala Ser Ala Pro Trp Ala Ala Gly Leu Ala
1               5                   10


<210>  81
<211>  12
<212>  PRT
<213>  artificial sequence

<220>
<223>  epitope

<400>  81

Arg Ala Glu Trp Arg Gly Arg Glu Arg Gly Gly Pro
1               5                   10


<210>  82
<211>  12
<212>  PRT
<213>  artificial sequence

<220>
<223>  epitope

<400>  82

Met Pro Ala His Gly Arg Leu Asp Leu Val Pro Phe
1               5                   10


<210>  83
<211>  12
<212>  PRT
<213>  artificial sequence

<220>
<223>  epitope

<400>  83

Gly Val Pro Ala Thr Pro Gln Gly Pro Lys Ser Pro
1               5                   10


<210>  84
<211>  12
<212>  PRT
<213>  artificial sequence

<220>
<223>  epitope

<400>  84

Gly Pro Lys Ser Pro Leu Trp Ala Ala Gly Leu Ala
1               5                   10


<210>  85
<211>  12
<212>  PRT
<213>  artificial sequence

<220>
<223>  epitope

<400>  85

Arg Ala Glu Trp Arg Gly Arg Glu Arg Ser Gly Ser
1               5                   10


<210>  86
<211>  468
<212>  DNA
<213>  artificial sequence

<220>
<223>  polynucleotide

<400>  86
atgcgggacc tgcccctgac aagcctggct ctggtgctgt ctgctctggg agccctcctg      60

ggaacagaag ccctgagagc cgaagaacct gccgtgggca catccggcct gatcttcaga     120

gaggacctgg actggcctcc cggcagccct caggaacctc tctgtctggt cgctctgggc 180

ggcgacagca atggctctag cagccctctg agagtcgtgg gcgccctgtc tgcctacgag 240

caggcttttc tgggagccgt gcagagggct agatggggcc ctagagatct ggccaccttc 300

ggcgtgtgca acaccggcga tagacaggcc gctctgccca gcctgagaag gctgggagct 360

tggctgagag atcctggcgg ccagagactg gtggtgctgc acctggaaga agtgacctgg 420

gagcctaccc ctagcctgcg gtttcaggaa ccacctcctg gcggagcc 468

<210> 87
<211> 765
<212> DNA
<213> artificial sequence

<220>
<223> polynucleotide

<400> 87
atgcgggacc tgcccctgac aagcctggct ctggtgctgt ctgctctggg agccctcctg 60

ggaacagaag ccctgagagc cgaagaacct gccgtgggca catccggcct gatcttcaga 120

gaggacctgg actggcctcc cggcagccct caggaacctc tctgtctggt cgctctgggc 180

ggcgacagca atggctctag cagccctctg agagtcgtgg gcgccctgtc tgcctacgag 240

caggcttttc tgggagccgt gcagagggct agatggggcc ctagagatct ggccaccttc 300

ggcgtgtgca acaccggcga tagacaggcc gctctgccca gcctgagaag gctgggagct 360

tggctgagag atcctggcgg ccagagactg gtggtgctgc acctggaaga agtgacctgg 420

gagcctaccc ctagcctgcg gtttcaggaa ccacctcctg gcggagccgg acctcctgaa 480

ctggctctgc tggtcctgta tcctggccct ggccccgaag tgaccgtgac aagagctgga 540

ctgcctggcg cccagtccct gtgccctagc agagacacca gatacctggt gctggccgtg 600

gacagacctg ccggcgcttg gagaggaagt ggcctggcac tgaccctgca gcccagaggc 660

gaggatagca gactgagcac cgccagactg caggccctgc tgtttggcga cgaccaccgg 720

tgcttcacca gaatgacccc tgccctgctg ctgctcccca gatct 765

<210> 88
<211> 1353
<212> DNA
<213> artificial sequence

<220>
<223> polynucleotide

<400> 88
atgcgggacc tgcccctgac aagcctggct ctggtgctgt ctgctctggg agccctcctg 60

ggaacagaag ccctgagagc cgaagaacct gccgtgggca catccggcct gatcttcaga 120

gaggacctgg actggcctcc cggcagccct caggaacctc tctgtctggt cgctctgggc 180

ggcgacagca atggctctag cagccctctg agagtcgtgg gcgccctgtc tgcctacgag 240

caggcttttc tgggagccgt gcagagggct agatggggcc ctagagatct ggccaccttc 300

ggcgtgtgca acaccggcga tagacaggcc gctctgccca gcctgagaag gctgggagct 360

tggctgagag atcctggcgg ccagagactg gtggtgctgc acctggaaga agtgacctgg 420

gagcctaccc ctagcctgcg gtttcaggaa ccacctcctg gcggagccgg acctcctgaa 480

ctggctctgc tggtcctgta tcctggccct ggccccgaag tgaccgtgac aagagctgga 540

ctgcctggcg cccagtccct gtgccctagc agagacacca gatacctggt gctggccgtg 600

gacagacctg ccggcgcttg gagaggaagt ggcctggcac tgaccctgca gcccagaggc 660

gaggatagca gactgagcac cgccagactg caggccctgc tgtttggcga cgaccaccgg 720

tgcttcacca gaatgacccc tgccctgctg ctgctcccca gatctgaacc tgctcctctg 780

cctgcccacg gacagctgga taccgtgcct ttcccaccac ctagacccag cgccgagctg 840

gaagagtctc ctcctagcgc cgaccccttc ctggaaaccc tgactagact cgtgcgggcc 900

ctgagggtgc caccagctag agcatctgcc cctagactgg cactggatcc cgatgccctg 960

gccggctttc ctcagggact cgtgaacctg tctgacccag ccgctctgga aagactgctg 1020

gacggcgaag aaccactcct cctgctgctc agacctaccg ccgccacaac aggcgatcct 1080

gcccctctgc atgatcccac atctgctcca tgggccaccg ccctggctag aagagtggct 1140

gctgaactgc aggctgccgc cgctgagctg agatctctgc caggactgcc tcctgccaca 1200

gccccactgc tggctagact gctcgctctg tgtccaggcg gacctggcgg actgggagat 1260

ccactgagag cactgctgct cctcaaggcc ctccagggcc tcagggtcga atggcgggga 1320

agagatccta gaggcccagg cagagcccag aga 1353

<210> 89
<211> 1679
<212> DNA
<213> artificial sequence

<220>
<223> polynucleotide

<400> 89
atgcgggacc tgcccctgac aagcctggct ctggtgctgt ctgctctggg agccctcctg 60

ggaacagaag ccctgagagc cgaagaacct gccgtgggca catccggcct gatcttcaga 120

gaggacctgg actggcctcc cggcagccct caggaacctc tctgtctggt cgctctgggc 180

ggcgacagca atggctctag cagccctctg agagtcgtgg gcgccctgtc tgcctacgag 240

caggcttttc tgggagccgt gcagagggct agatggggcc ctagagatct ggccaccttc 300

ggcgtgtgca acaccggcga tagacaggcc gctctgccca gcctgagaag gctgggagct 360

tggctgagag atcctggcgg ccagagactg gtggtgctgc acctggaaga agtgacctgg 420

gagcctaccc ctagcctgcg gtttcaggaa ccacctcctg gcggagccgg acctcctgaa 480

ctggctctgc tggtcctgta tcctggccct ggccccgaag tgaccgtgac aagagctgga 540

ctgcctggcg cccagtccct gtgccctagc agagacacca gatacctggt gctggccgtg 600

gacagacctg ccggcgcttg gagaggaagt ggcctggcac tgaccctgca gcccagaggc 660

gaggatagca gactgagcac cgccagactg caggccctgc tgtttggcga cgaccaccgg 720

tgcttcacca gaatgacccc tgccctgctg ctgctcccca gatctgaacc tgctcctctg 780

cctgcccacg gacagctgga taccgtgcct ttcccaccac ctagacccag cgccgagctg 840

gaagagtctc ctcctagcgc cgaccccttc ctggaaaccc tgactagact cgtgcgggcc 900

ctgagggtgc caccagctag agcatctgcc cctagactgg cactggatcc cgatgccctg 960

gccggctttc ctcagggact cgtgaacctg tctgacccag ccgctctgga aagactgctg 1020

gacggcgaag aaccactcct cctgctgctc agacctaccg ccgccacaac aggcgatcct 1080

gcccctctgc atgatcccac atctgctcca tgggccaccg ccctggctag aagagtggct 1140

gctgaactgc aggctgccgc cgctgagctg agatctctgc caggactgcc tcctgccaca 1200

gccccactgc tggctagact gctcgctctg tgtccaggcg gacctggcgg actgggagat 1260

ccactgagag cactgctgct cctcaaggcc ctccagggcc tcagggtcga atggcgggga 1320

agagatccta gaggcccagg cagagcccag agaagcgctg gcgctacagc tgccgatgga 1380

ccttgcgctc tgcgggaact gagcgtggac ctgagggccg agagaagcgt cctgatcccc 1440

gagacatacc aggccaacaa ctgccagggc gtgtgcggct ggcctcagag cgacagaaac 1500

cccagatacg gcaatcacgt ggtgctgctg ctgaagatgc aagtccgagg cgccgctctg 1560

gcaagacctc cttgttgtgt gcctaccgcc tacgccggca agctgctgat ctccctgagc 1620

gaggaacgga tagcgcccac cacgtgccca acatggtggc cacagagtgc ggctgccgg 1679

<210> 90
<211> 12
<212> PRT
<213> artificial sequence

<220>
<223> peptide

<400> 90

Met Arg Asp Leu Pro Leu Thr Ser Leu Ala Leu Val
1               5                   10

<210> 91
<211> 12
<212> PRT

<213>  artificial sequence

<220>
<223>  peptide

<400>  91

Ser Leu Ala Leu Val Leu Ser Ala Leu Gly Ala Leu
1               5                   10


<210>  92
<211>  12
<212>  PRT
<213>  artificial sequence

<220>
<223>  peptide

<400>  92

Ala Leu Gly Ala Leu Leu Gly Thr Glu Ala Leu Arg
1               5                   10


<210>  93
<211>  12
<212>  PRT
<213>  artificial sequence

<220>
<223>  peptide

<400>  93

Thr Glu Ala Leu Arg Ala Glu Glu Pro Ala Val Gly
1               5                   10


<210>  94
<211>  12
<212>  PRT
<213>  artificial sequence

<220>
<223>  peptide

<400>  94

Glu Pro Ala Val Gly Thr Ser Gly Leu Ile Phe Arg
1               5                   10


<210>  95
<211>  12
<212>  PRT
<213>  artificial sequence

<220>
<223>  peptide

<400>  95

Gly Leu Ile Phe Arg Glu Asp Leu Asp Trp Pro Pro
1               5                   10


<210>  96
<211>  12
<212>  PRT
<213>  artificial sequence

<220>
<223>  peptide

<400>  96

Leu Asp Trp Pro Pro Gly Ser Pro Gln Glu Pro Leu
1               5                   10


<210>  97
<211>  12
<212>  PRT
<213>  artificial sequence

<220>
<223>  peptide

<400>  97

Pro Gln Glu Pro Leu Cys Leu Val Ala Leu Gly Gly
1               5                   10


<210>  98
<211>  12
<212>  PRT
<213>  artificial sequence

<220>
<223>  peptide

<400>  98

Val Ala Leu Gly Gly Asp Ser Asn Gly Ser Ser Ser
1               5                   10


<210>  99
<211>  12
<212>  PRT
<213>  artificial sequence

<220>
<223>  peptide

<400>  99

Asn Gly Ser Ser Ser Pro Leu Arg Val Val Gly Ala
1               5                   10


<210>  100
<211>  12
<212>  PRT

<213>  artificial sequence

<220>
<223>  peptide

<400>  100

Arg Val Val Gly Ala Leu Ser Ala Tyr Glu Gln Ala
1               5                   10


<210>  101
<211>  12
<212>  PRT
<213>  artificial sequence

<220>
<223>  peptide

<400>  101

Ala Tyr Glu Gln Ala Phe Leu Gly Ala Val Gln Arg
1               5                   10


<210>  102
<211>  12
<212>  PRT
<213>  artificial sequence

<220>
<223>  peptide

<400>  102

Gly Ala Val Gln Arg Ala Arg Trp Gly Pro Arg Asp
1               5                   10


<210>  103
<211>  12
<212>  PRT
<213>  artificial sequence

<220>
<223>  peptide

<400>  103

Trp Gly Pro Arg Asp Leu Ala Thr Phe Gly Val Cys
1               5                   10


<210>  104
<211>  12
<212>  PRT
<213>  artificial sequence

<220>
<223>  peptide

<400>  104

Thr Phe Gly Val Cys Asn Thr Gly Asp Arg Gln Ala
1               5                   10

<210>  105
<211>  12
<212>  PRT
<213>  artificial sequence

<220>
<223>  peptide

<400>  105

Gly Asp Arg Gln Ala Ala Leu Pro Ser Leu Arg Arg
1               5                   10

<210>  106
<211>  12
<212>  PRT
<213>  artificial sequence

<220>
<223>  peptide

<400>  106

Pro Ser Leu Arg Arg Leu Gly Ala Trp Leu Arg Asp
1               5                   10

<210>  107
<211>  12
<212>  PRT
<213>  artificial sequence

<220>
<223>  peptide

<400>  107

Ala Trp Leu Arg Asp Pro Gly Gly Gln Arg Leu Val
1               5                   10

<210>  108
<211>  12
<212>  PRT
<213>  artificial sequence

<220>
<223>  peptide

<400>  108

Gly Gln Arg Leu Val Val Leu His Leu Glu Glu Val
1               5                   10

<210>  109
<211>  12
<212>  PRT

<213>  artificial sequence

<220>
<223>  peptide

<400>  109

His Leu Glu Glu Val Thr Trp Glu Pro Thr Pro Ser
1               5                   10


<210>  110
<211>  12
<212>  PRT
<213>  artificial sequence

<220>
<223>  peptide

<400>  110

Glu Pro Thr Pro Ser Leu Arg Phe Gln Glu Pro Pro
1               5                   10


<210>  111
<211>  12
<212>  PRT
<213>  artificial sequence

<220>
<223>  peptide

<400>  111

Phe Gln Glu Pro Pro Pro Gly Gly Ala Gly Pro Pro
1               5                   10


<210>  112
<211>  12
<212>  PRT
<213>  artificial sequence

<220>
<223>  peptide

<400>  112

Gly Ala Gly Pro Pro Glu Leu Ala Leu Leu Val Leu
1               5                   10


<210>  113
<211>  12
<212>  PRT
<213>  artificial sequence

<220>
<223>  peptide

<400>  113

Ala Leu Leu Val Leu Tyr Pro Gly Pro Gly Pro Glu
1               5                   10

<210>  114
<211>  12
<212>  PRT
<213>  artificial sequence

<220>
<223>  peptide

<400>  114

Gly Pro Gly Pro Glu Val Thr Val Thr Arg Ala Gly
1               5                   10

<210>  115
<211>  12
<212>  PRT
<213>  artificial sequence

<220>
<223>  peptide

<400>  115

Val Thr Arg Ala Gly Leu Pro Gly Ala Gln Ser Leu
1               5                   10

<210>  116
<211>  12
<212>  PRT
<213>  artificial sequence

<220>
<223>  peptide

<400>  116

Gly Ala Gln Ser Leu Cys Pro Ser Arg Asp Thr Arg
1               5                   10

<210>  117
<211>  12
<212>  PRT
<213>  artificial sequence

<220>
<223>  peptide

<400>  117

Ser Arg Asp Thr Arg Tyr Leu Val Leu Ala Val Asp
1               5                   10

<210>  118
<211>  12
<212>  PRT

<213>  artificial sequence

<220>
<223>  peptide

<400>  118

Ala Gly Ala Trp Arg Gly Ser Gly Leu Ala Leu Thr
1               5                   10


<210>  119
<211>  12
<212>  PRT
<213>  artificial sequence

<220>
<223>  peptide

<400>  119

Gly Leu Ala Leu Thr Leu Gln Pro Arg Gly Glu Asp
1               5                   10


<210>  120
<211>  12
<212>  PRT
<213>  artificial sequence

<220>
<223>  peptide

<400>  120

Leu Ser Thr Ala Arg Leu Gln Ala Leu Leu Phe Gly
1               5                   10


<210>  121
<211>  12
<212>  PRT
<213>  artificial sequence

<220>
<223>  peptide

<400>  121

Ala Leu Leu Phe Gly Asp Asp His Arg Cys Phe Thr
1               5                   10


<210>  122
<211>  12
<212>  PRT
<213>  artificial sequence

<220>
<223>  peptide

<400>  122

Thr Pro Ala Leu Leu Leu Leu Pro Arg Ser Glu Pro
1               5                   10


<210>  123
<211>  12
<212>  PRT
<213>  artificial sequence

<220>
<223>  peptide

<400>  123

Pro Arg Ser Glu Pro Ala Pro Leu Pro Ala His Gly
1               5                   10


<210>  124
<211>  12
<212>  PRT
<213>  artificial sequence

<220>
<223>  peptide

<400>  124

Asp Thr Val Pro Phe Pro Pro Pro Arg Pro Ser Ala
1               5                   10


<210>  125
<211>  12
<212>  PRT
<213>  artificial sequence

<220>
<223>  peptide

<400>  125

Pro Arg Pro Ser Ala Glu Leu Glu Glu Ser Pro Pro
1               5                   10


<210>  126
<211>  12
<212>  PRT
<213>  artificial sequence

<220>
<223>  peptide

<400>  126

Glu Glu Ser Pro Pro Ser Ala Asp Pro Phe Leu Glu
1               5                   10


<210>  127
<211>  12
<212>  PRT

<213>  artificial sequence

<220>
<223>  peptide

<400>  127

Asp Pro Phe Leu Glu Thr Leu Thr Arg Leu Val Arg
1               5                   10


<210>  128
<211>  12
<212>  PRT
<213>  artificial sequence

<220>
<223>  peptide

<400>  128

Thr Arg Leu Val Arg Ala Leu Arg Val Pro Pro Ala
1               5                   10


<210>  129
<211>  12
<212>  PRT
<213>  artificial sequence

<220>
<223>  peptide

<400>  129

Arg Val Pro Pro Ala Arg Ala Ser Ala Pro Arg Leu
1               5                   10


<210>  130
<211>  12
<212>  PRT
<213>  artificial sequence

<220>
<223>  peptide

<400>  130

Ser Ala Pro Arg Leu Ala Leu Asp Pro Asp Ala Leu
1               5                   10


<210>  131
<211>  12
<212>  PRT
<213>  artificial sequence

<220>
<223>  peptide

<400>  131

Asp Pro Asp Ala Leu Ala Gly Phe Pro Gln Gly Leu
1               5                   10

<210>  132
<211>  12
<212>  PRT
<213>  artificial sequence

<220>
<223>  peptide

<400>  132

Phe Pro Gln Gly Leu Val Asn Leu Ser Asp Pro Ala
1               5                   10

<210>  133
<211>  12
<212>  PRT
<213>  artificial sequence

<220>
<223>  peptide

<400>  133

Arg Pro Thr Ala Ala Thr Thr Gly Asp Pro Ala Pro
1               5                   10

<210>  134
<211>  12
<212>  PRT
<213>  artificial sequence

<220>
<223>  peptide

<400>  134

Ala Thr Ala Leu Ala Arg Arg Val Ala Ala Glu Leu
1               5                   10

<210>  135
<211>  12
<212>  PRT
<213>  artificial sequence

<220>
<223>  peptide

<400>  135

Val Ala Ala Glu Leu Gln Ala Ala Ala Ala Glu Leu
1               5                   10

<210>  136
<211>  12
<212>  PRT

<213>  artificial sequence

<220>
<223>  peptide

<400>  136

Ala Ala Ala Glu Leu Arg Ser Leu Pro Gly Leu Pro
1               5                   10


<210>  137
<211>  12
<212>  PRT
<213>  artificial sequence

<220>
<223>  peptide

<400>  137

Leu Pro Gly Leu Pro Pro Ala Thr Ala Pro Leu Leu
1               5                   10


<210>  138
<211>  12
<212>  PRT
<213>  artificial sequence

<220>
<223>  peptide

<400>  138

Thr Ala Pro Leu Leu Ala Arg Leu Leu Ala Leu Cys
1               5                   10


<210>  139
<211>  12
<212>  PRT
<213>  artificial sequence

<220>
<223>  peptide

<400>  139

Leu Leu Ala Leu Cys Pro Gly Gly Pro Gly Gly Leu
1               5                   10


<210>  140
<211>  12
<212>  PRT
<213>  artificial sequence

<220>
<223>  peptide

<400>  140

Gly Pro Gly Gly Leu Gly Asp Pro Leu Arg Ala Leu
1               5                   10


<210>  141
<211>  12
<212>  PRT
<213>  artificial sequence

<220>
<223>  peptide

<400>  141

Pro Leu Arg Ala Leu Leu Leu Leu Lys Ala Leu Gln
1               5                   10


<210>  142
<211>  12
<212>  PRT
<213>  artificial sequence

<220>
<223>  peptide

<400>  142

Asp Pro Arg Gly Pro Gly Arg Ala Gln Arg Ser Ala
1               5                   10


<210>  143
<211>  12
<212>  PRT
<213>  artificial sequence

<220>
<223>  peptide

<400>  143

Ala Gln Arg Ser Ala Gly Ala Thr Ala Ala Asp Gly
1               5                   10


<210>  144
<211>  12
<212>  PRT
<213>  artificial sequence

<220>
<223>  peptide

<400>  144

Thr Ala Ala Asp Gly Pro Cys Ala Leu Arg Glu Leu
1               5                   10


<210>  145
<211>  12
<212>  PRT

<213>  artificial sequence

<220>
<223>  peptide

<400>  145

Ala Leu Arg Glu Leu Ser Val Asp Leu Arg Ala Glu
1               5                   10


<210>  146
<211>  12
<212>  PRT
<213>  artificial sequence

<220>
<223>  peptide

<400>  146

Asp Leu Arg Ala Glu Arg Ser Val Leu Ile Pro Glu
1               5                   10


<210>  147
<211>  12
<212>  PRT
<213>  artificial sequence

<220>
<223>  peptide

<400>  147

Val Leu Ile Pro Glu Thr Tyr Gln Ala Asn Asn Cys
1               5                   10


<210>  148
<211>  12
<212>  PRT
<213>  artificial sequence

<220>
<223>  peptide

<400>  148

Gln Ala Asn Asn Cys Gln Gly Val Cys Gly Trp Pro
1               5                   10


<210>  149
<211>  12
<212>  PRT
<213>  artificial sequence

<220>
<223>  peptide

<400>  149

Val Cys Gly Trp Pro Gln Ser Asp Arg Asn Pro Arg
1               5                   10

<210>  150
<211>  12
<212>  PRT
<213>  artificial sequence

<220>
<223>  peptide

<400>  150

Asp Arg Asn Pro Arg Tyr Gly Asn His Val Val Leu
1               5                   10

<210>  151
<211>  12
<212>  PRT
<213>  artificial sequence

<220>
<223>  peptide

<400>  151

Asn His Val Val Leu Leu Leu Lys Met Gln Val Arg
1               5                   10

<210>  152
<211>  12
<212>  PRT
<213>  artificial sequence

<220>
<223>  peptide

<400>  152

Lys Met Gln Val Arg Gly Ala Ala Leu Ala Arg Pro
1               5                   10

<210>  153
<211>  12
<212>  PRT
<213>  artificial sequence

<220>
<223>  peptide

<400>  153

Ala Leu Ala Arg Pro Pro Cys Cys Val Pro Thr Ala
1               5                   10

<210>  154
<211>  12
<212>  PRT

<213>  artificial sequence

<220>
<223>  peptide

<400>  154

Cys Val Pro Thr Ala Tyr Ala Gly Lys Leu Leu Ile
1               5                   10


<210>  155
<211>  12
<212>  PRT
<213>  artificial sequence

<220>
<223>  peptide

<400>  155

Gly Lys Leu Leu Ile Ser Leu Ser Glu Glu Arg Ile
1               5                   10


<210>  156
<211>  12
<212>  PRT
<213>  artificial sequence

<220>
<223>  peptide

<400>  156

Ser Glu Glu Arg Ile Ser Ala His His Val Pro Asn
1               5                   10


<210>  157
<211>  12
<212>  PRT
<213>  artificial sequence

<220>
<223>  peptide

<400>  157

His His Val Pro Asn Met Val Ala Thr Glu Cys Gly
1               5                   10


<210>  158
<211>  12
<212>  PRT
<213>  artificial sequence

<220>
<223>  peptide

<400>  158

Val Pro Asn Met Val Ala Thr Glu Cys Gly Cys Arg
1               5                   10